# EUROPEAN PATENT SPECIFICATION

(11) **EP 4 376 998 B1**
(45) Date of publication and mention of the grant of the patent: **04.12.2024**
(21) Application number: 23744342.9
(22) Date of filing: 28.06.2023
(51) Int. Cl.: B01J 19/00

(54) **METHODS AND COMPOSITIONS FOR REFINING FEATURE BOUNDARIES IN MOLECULAR ARRAYS**
VERFAHREN UND ZUSAMMENSETZUNGEN ZUR VERFEINERUNG VON MERKMALSGRENZEN IN MOLEKÜLARRAYS
PROCÉDÉS ET COMPOSITIONS POUR AFFINER DES LIMITES DE CARACTÉRISTIQUES DANS DES MATRICES MOLÉCULAIRES

(30) Priority: 29.06.2022 US 202263356922 P
(43) Date of publication of application: 05.06.2024
(60) Divisional application: 24208662.7
(73) Proprietor: 10X Genomics, Inc., Pleasanton, CA 94588-3260 (US)
(72) Inventor: RAMACHANDRAN IYER, Eswar Prasad, Pleasanton, California 94588-3260 (US)
(74) Representative: J A Kemp LLP
(86) International application number: PCT/US2023/069221
(87) International publication number: WO 2024/006797

(56) References cited:
- WO-A1-2006/044956
- WO-A1-2020/159794
- WO-A2-00/33084
- DE-A1- 19 932 488

## Description

### FIELD

The present disclosure relates in some aspects to molecular arrays and methods for manufacturing and refining molecular arrays on a substrate, which can include removing non-specific oligonucleotides on array feature boundaries.

### BACKGROUND

Arrays of nucleic acids are an important tool in the biotechnology industry and related fields. There are two main ways of producing nucleic acid arrays in which the immobilized nucleic acids are covalently attached to a substrate surface, e.g., via *in situ* synthesis in which a nucleic acid polymer is grown on the surface of the substrate in a step-wise, nucleotide-by-nucleotide fashion, or via deposition of a full, pre-synthesized nucleic acid/polypeptide, cDNA fragment, etc., onto the surface of the array.

At present, there remains a need to generate molecular arrays for analyzing at high resolution the spatial expression patterns of large numbers of genes, proteins, or other biologically active molecules simultaneously. Provided are methods, uses and articles of manufacture that meet these and other needs.

WO 2006/044956 discloses methods for assembly of high fidelity synthetic polynucleotides.

### SUMMARY

Nucleic acid arrays in which a plurality of distinct or different nucleic acids are patterned on a solid support surface find use in a variety of applications, including gene expression analysis, drug screening, nucleic acid sequencing, mutation analysis, and the like. A feature of many arrays that have been developed is that each of the distinct nucleic acids of the array is stably attached to a discrete location on the array surface, such that its position remains constant and known throughout the use of the array. Stable attachment is achieved in a number of different ways, such as covalent bonding of a nucleic acid polymer to the support surface and non-covalent interaction of the nucleic acid polymer with the surface.

The invention provides a method for providing an array comprising first and second regions on a substrate, comprising: (a) attaching first oligonucleotide molecules to first nucleic acid molecules immobilized in the first region via hybridization and/or ligation to generate first extended nucleic acid molecules; (b) attaching second oligonucleotide molecules to second nucleic acid molecules immobilized in the second region via hybridization and/or ligation to generate second extended nucleic acid molecules; and (c) irradiating the substrate through a mask comprising an opening corresponding to a boundary region between the first and second regions, thereby removing (e.g., cleaving), blocking (e.g., covering with an inert material), or inactivating (e.g., capping the 3' or 5' terminus) nucleic acid molecules in the boundary region. In some embodiments, the boundary region comprises part of the first region and/or part of the second region.

In any of the embodiments herein, the first nucleic acid molecules can be directly or indirectly attached to the substrate, e.g., via a linker or spacer. In any of the embodiments herein, the second nucleic acid molecules can be directly or indirectly attached to the substrate, e.g., via a linker or spacer.

In any of the embodiments herein, the first and second regions can be of about 2 µm to about 20 µm in average diameter, optionally wherein the first and second regions are of about 5 µm. In any of the embodiments herein, the opening in the mask can correspond to the boundary region. In any of the embodiments herein, the opening and/or the boundary region can be between about 1 µm and about 20 µm in length, optionally wherein the length is of about 5 µm, and between about 0.1 µm and about 2 µm in width, optionally wherein the width is of about 0.5 µm. In any of the embodiments herein, the removed or inactivated nucleic acid molecules in the boundary region can comprise (i) a first oligonucleotide molecule attached to a second nucleic acid molecule and/or (ii) a second oligonucleotide molecule attached to a first nucleic acid molecule.

In any of the embodiments herein, the substrate can be irradiated through the mask with irradiation sufficient to cleave nucleic acid molecules in the boundary region off the substrate, optionally wherein the substrate is irradiated through the mask with UV light. In some embodiments, the method further comprises removing the cleaved nucleic acid molecules and cleavage products thereof. In any of the embodiments herein, a positive photoresist can be applied to the substrate and upon irradiation through the mask, the positive photoresist can be degraded to expose nucleic acid molecules in the boundary region which are cleaved by the irradiation.

In any of the embodiments herein, a negative photoresist can be applied to the substrate and upon irradiation through the mask, the negative photoresist can be strengthened to render nucleic acid molecules in the boundary region inactive or inaccessible for ligation, optionally wherein the negative photoresist is strengthened by polymerization or cross-linking.

In any of the embodiments herein, a positive photoresist can be applied to the substrate and upon irradiation through the mask, the positive photoresist can be degraded to expose nucleic acid molecules in the boundary region, while nucleic acid molecules in the masked region(s) remain covered by the positive photoresist. In some embodiments, the method further comprises removing or inactivating the exposed nucleic acid molecules. In some embodiments, the exposed nucleic acid molecules are digested by a nuclease, optionally wherein the nuclease is an endonuclease, a 3'→5' exonuclease, and/or a 5'→3' exonuclease. In some embodiments, the exposed nucleic acid molecules are modified at the 3' or 5' end, rendering the exposed nucleic acid molecules unavailable for ligation. In some embodiments, the exposed nucleic acid molecules comprise modified 3' ends selected from the group consisting of dideoxy C-3' (3'-ddC), 3' inverted dT, 3' C3 spacer, 3'Amino, and 3' phosphorylation.

In any of the embodiments herein, the attaching step in (a) and/or (b) can comprise photolithography using a photoresist, a 3' or 5' photo-cleavable protective group, and/or a photo-cleavable polymer that blocks hybridization and/or ligation.

Using a method disclosed herein, a large diversity of barcodes can be created in molecules on the substrate via sequential rounds of light exposure, hybridization, ligation, and further interceded and/or followed by one or more rounds of photo-controllably cleaning and/or refining array feature boundaries, to improve spatial array resolution and sensitivity. In any of the embodiments herein, a method disclosed herein can photo-controllably remove, block, and/or inactivate nucleic acid molecules at feature boundaries. For instance, a positive photoresist covering a feature boundary region can be removed (e.g., by light exposure through a photomask and application of a developer) to allow degradation (e.g., by UV irradiation or chemical cleavage) or blocking/inactivation of nucleic acid molecules (e.g., using blocking groups at 3' or 5' that block hybridization or ligation) in the boundary region, whereas nucleic acid molecules outside the boundary region are not removed, blocked, and/or inactivated. Alternatively, a negative photoresist covering a feature boundary region can be exposed to light and developed such that the hardened negative photoresist can block/inactivate nucleic acid molecules in the feature boundary region, whereas nucleic acid molecules outside the boundary region are not blocked and/or inactivated since the negative photoresist outside the boundary region are not exposed to light and can be removed during developing.

### BRIEF DESCRIPTION OF THE DRAWINGS

The drawings illustrate certain features and advantages of this disclosure. These embodiments are not intended to limit the scope of the appended claims in any manner.

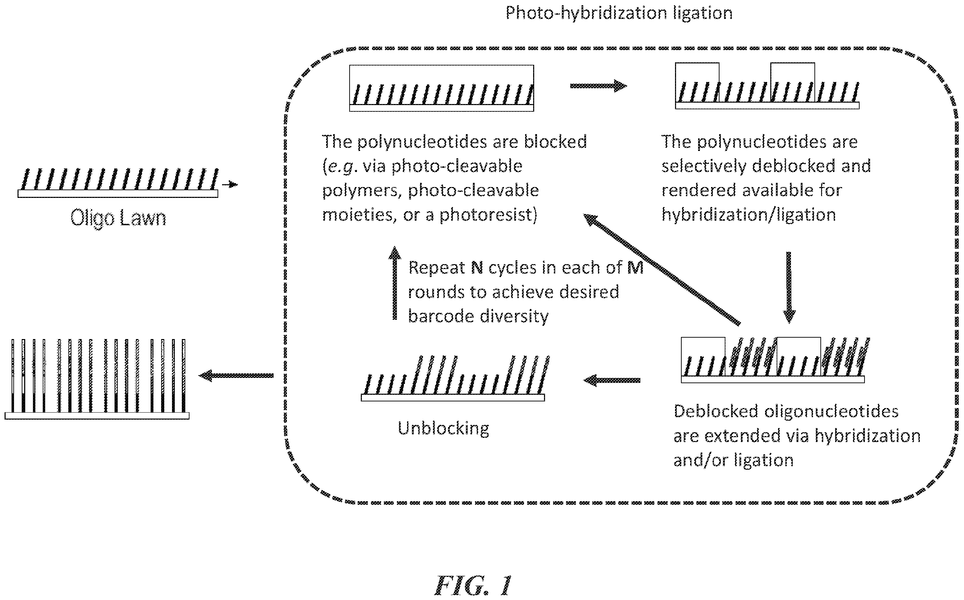

**FIG. 1** shows a schematic illustrating an exemplary method for *in situ* array generation. Oligonucleotides on a substrate can be extended by photolithography-guided oligonucleotide hybridization and ligation (photo-hybridization ligation) to generate nucleic acid arrays in sequential oligonucleotide hybridization and ligation cycles to generate barcode diversity in the nucleic acid arrays.

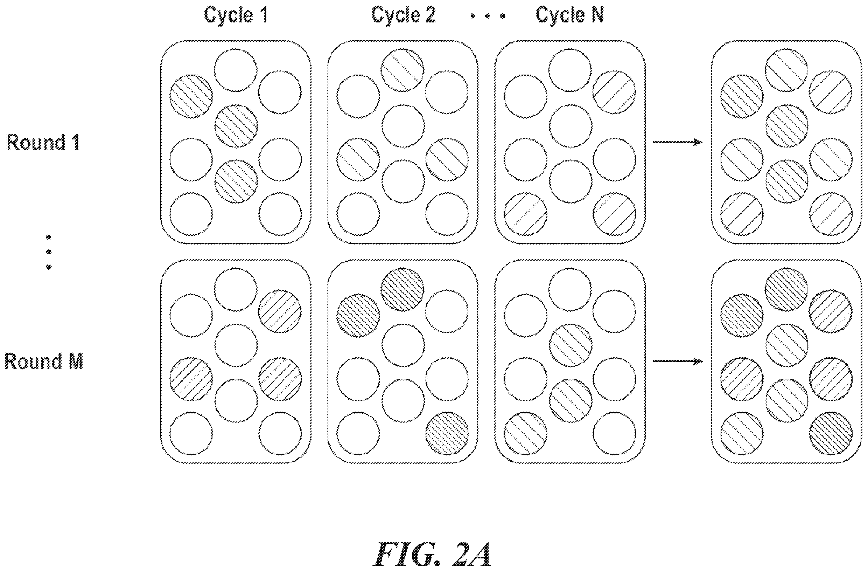

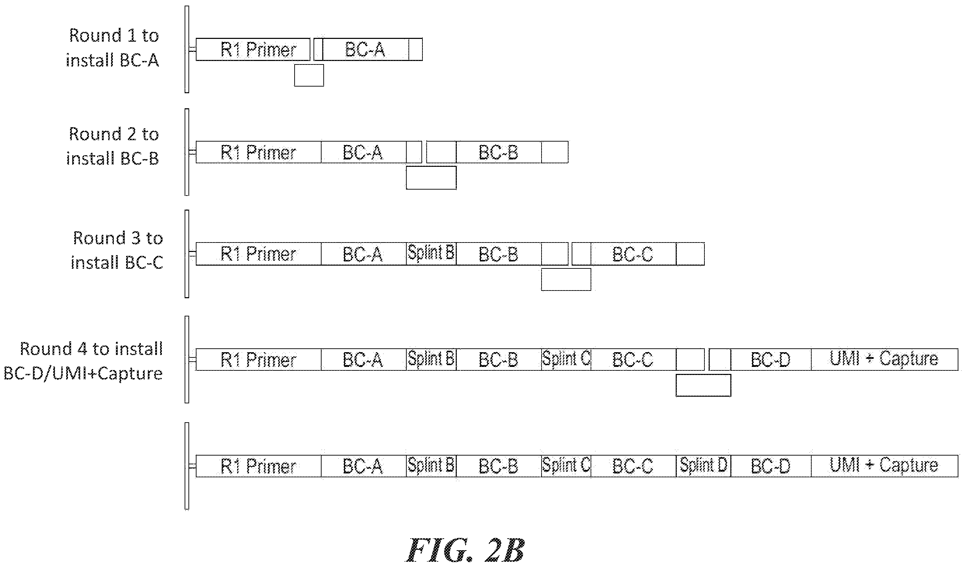

**FIGS. 2A-2B** show oligonucleotides comprising barcodes or parts thereof can be installed in ***M*** sequential rounds each comprising ***N*** cycles of photo-hybridization ligation. **FIG. 2A** shows a schematic illustrating an exemplary method comprising ***M*** rounds, each of which comprise ***N*** cycles of light exposure of a region on the substrate and oligonucleotide attachment to exposed oligonucleotide molecules on the substrate. **FIG. 2B** shows an exemplary barcoded molecule generated using a method described herein.

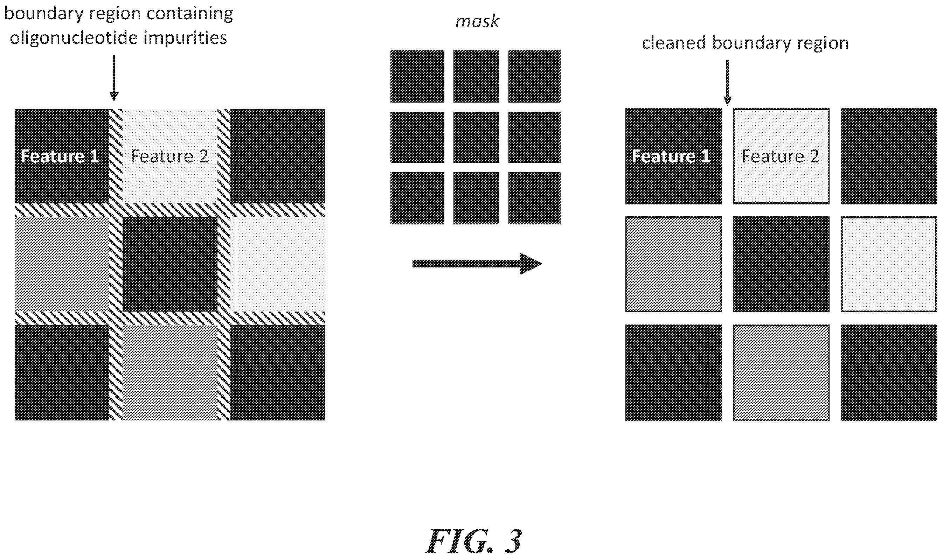

**FIG. 3** shows an exemplary method for refinement of a molecular array by removing, blocking, and/or inactivating nucleic acid molecules on feature boundaries.

### DETAILED DESCRIPTION

The technical information set out below may in some respects go beyond the disclosure of the invention, which is defined by the appended claims. The additional technical information is provided to place the invention in a broader technical context and to illustrate possible related technical developments. Such additional technical information which does not fall within the scope of the appended claims is not part of the invention. Accordingly, any aspects, embodiments and examples of the present disclosure which do not fall under the scope of the appended claims do not form part of the invention and are merely provided for illustrative purposes. If a definition set forth herein is contrary to or otherwise inconsistent with a definition set forth in the patents, applications, published applications and other publications that are herein referenced, the definition set forth herein prevails over the definition that is in the reference.

The section headings used herein are for organizational purposes only and are not to be construed as limiting the subject matter described.

### I. OVERVIEW

Oligonucleotide arrays for spatial transcriptomics may be made by mechanical spotting, bead arrays, and/or *in situ* base-by-base synthesis of the oligonucleotides. In some cases, mechanical spotting is ideal for larger spot sizes (*e.g.*, 30 microns in diameter or greater), since fully elaborated oligos (*e.g.*, with a desired combination and diversity of barcodes) can be spotted in a known position with high purity and fidelity. However, methods to decrease spot sizes or features at or below 10 microns (e.g., single cell scale resolution) in diameter with sufficient throughput are lacking. In some aspects, bead arrays offer a way to increase feature density. For example, barcodes are generated by first attaching an oligonucleotide to all beads and then performing multiple rounds of split-pool ligations to generate barcodes combinatorially. However, in some aspects, bead arrays can result in random barcoded bead arrays that must be decoded prior to use and each array ultimately has a unique pattern. Additionally, even monodisperse beads at the 1-10 micron scale may have some variability that results in a range of feature sizes with the potential for variable oligo density.

Methods for *in situ* generated arrays have utilized photo-cleavable protecting groups to synthesize barcode oligos one nucleotide at a time. The feature size can be precisely controlled using photomasks and the generated array is known and uniform across all arrays with no decoding needed. However, the oligo fidelity decreases with increasing oligo length with a ~99% per step (i.e., per nucleotide) efficiency using base-by-base *in situ* oligonucleotide synthesis. A method to overcome fidelity issues is to directly ligate or hybridize entire sequences instead of bases onto mask-mediated photo-de-protected regions and combinatorially build barcodes on an array. Nevertheless, this approach still requires a large number of masks and long ligation cycles to fully construct all barcodes, thus reducing fidelity of alignment over the cycles.

In particular, *in situ* synthesized oligonucleotide arrays may have oligonucleotide impurities at feature boundaries due to overlap of adjacent features. The overlap in features can result from alignment error or stray light during photolithography, e.g., as shown in **FIG. 3****,** left panel The overlapping regions can be "cleaned" by either covering or blocking the non-specific oligonucleotides or releasing the non-specific oligonucleotides. For instance, during or after any cycle of the photolithographic workflow shown in **FIG. 1****,** a step can be introduced to remove, block, or inactivate the non-specific oligonucleotides or non-extended oligonucleotides on the boundaries. Oligonucleotide release and removal can be performed using cleavage strategies such as UV cleavage. In some embodiments, the oligonucleotides in a boundary region can be rendered inactive and/or inaccessible for hybridization and/or ligation. In some embodiments, the 3' or 5' terminus of an oligonucleotide in a boundary region can be rendered inactive and/or inaccessible for hybridization and/or ligation. Oligonucleotides can be rendered inactive and/or inaccessible by covering them with a biochemically inert layer, such as photoresist, that prevents and/or blocks access to the oligonucleotides. An example of a mask, such as a photomask, designed to protect synthesized features while allowing selective targeting of boundary regions is shown in **FIG. 3****,** where solid black areas of the mask block light and white areas allow light to pass to irradiate oligonucleotides on the feature boundaries.

### II. MOLECULAR ARRAYS

In some aspects, the methods provided herein comprises attaching oligonucleotides (e.g. a barcode) to a substrate. Oligonucleotides may be attached to the substrate according to the methods set forth in U.S. Patent Nos. 6,737,236, 7,259,258, 7,375,234, 7,427,678, 5,610,287, 5,807,522, 5,837,860, and 5,472,881; U.S. Patent Application Publication Nos. 2008/0280773 and 2011/0059865; Shalon et al. (1996) Genome Research, 639-645; Rogers et al. (1999) Analytical Biochemistry 266, 23-30; Stimpson et al. (1995) Proc. Natl. Acad. Sci. USA 92, 6379-6383; Beattie et al. (1995) Clin. Chem. 45, 700-706; Lamture et al. (1994) Nucleic Acids Research 22, 2121-2125; Beier et al. (1999) Nucleic Acids Research 27, 1970-1977; Joos et al. (1997) Analytical Biochemistry 247, 96-101; Nikiforov et al. (1995) Analytical Biochemistry 227, 201-209; Timofeev et al. (1996) Nucleic Acids Research 24, 3142-3148; Chrisey et al. (1996) Nucleic Acids Research 24, 3031-3039; Guo et al. (1994) Nucleic Acids Research 22, 5456-5465; Running and Urdea (1990) BioTechniques 8, 276-279; Fahy et al. (1993) Nucleic Acids Research 21, 1819-1826; Zhang *et al.* (1991) 19, 3929-3933; and Rogers et al. (1997) Gene Therapy 4, 1387-1392.

Arrays can be prepared by a variety of methods. In some embodiments, arrays are prepared through the synthesis (e.g., *in situ* synthesis) of oligonucleotides on the array, or by jet printing or lithography. For example, light-directed synthesis of high-density DNA oligonucleotides can be achieved by photolithography or solid-phase DNA synthesis. To implement photolithographic synthesis, synthetic linkers modified with photochemical protecting groups can be attached to a substrate and the photochemical protecting groups can be modified using a photolithographic mask (applied to specific areas of the substrate) and light, thereby producing an array having localized photo-deprotection. Such methods are described e.g., in Miller et al., "Basic concepts of microarrays and potential applications in clinical microbiology." Clinical microbiology reviews 22.4 (2009): 611-633; US201314111482A; US9593365B2; US2019203275; and WO2018091676.

In any of the embodiments herein, oligonucleotide molecules on the substrate can be immobilized in a plurality of features (e.g. specific regions, such as the first or second region on a substrate, as described herein). In any of the embodiments herein, the 3' terminal nucleotides of the immobilized oligonucleotide molecules can be distal to the substrate or array surface. In any of the embodiments herein, the 5' terminal nucleotides of the immobilized oligonucleotide molecules can be more proximal to the substrate or array surface than the 3' terminal nucleotides. In any of the embodiments herein, one or more nucleotides at or near the 5' terminus of each immobilized oligonucleotide can be directly or indirectly attached to the substrate or array surface, thereby immobilizing the oligonucleotides. In any of the embodiments herein, the 3' terminus of each immobilized oligonucleotide can project away from the substrate or array surface. In any of the embodiments herein, the 5' terminal nucleotides of the immobilized oligonucleotide molecules can be distal to the substrate or array surface. In any of the embodiments herein, the 3' terminal nucleotides of the immobilized oligonucleotide molecules can be more proximal to the substrate or array surface than the 5' terminal nucleotides. In any of the embodiments herein, one or more nucleotides at or near the 3' terminus of each immobilized oligonucleotide can be directly or indirectly attached to the substrate or array surface, thereby immobilizing the oligonucleotides. In any of the embodiments herein, the 5' terminus of each immobilized oligonucleotide can project away from the substrate or array surface.

In some embodiments, a method provided herein comprises a step of providing the substrate. A wide variety of different substrates can be used . In general, a substrate can be any suitable support material. The substrate may comprise materials of one or more of the IUPAC Groups 4, 6, 11, 12, 13, 14, and 15 elements, plastic material, silicon dioxide, glass, fused silica, mica, ceramic, or metals deposited on the aforementioned substrates. Exemplary substrates include, but are not limited to, glass, modified and/or functionalized glass, hydrogels, films, membranes, plastics (including e.g., acrylics, polystyrene, copolymers of styrene and other materials, polypropylene, polyethylene, polybutylene, polyurethanes, Teflon^{™}, cyclic olefins, polyimides etc.), nylon, ceramics, resins, Zeonor, silica or silica-based materials including silicon and modified silicon, carbon, quartz, metals, inorganic glasses, optical fiber bundles, and polymers, such as polystyrene, cyclic olefin copolymers (COCs), cyclic olefin polymers (COPs), polypropylene, polyethylene and polycarbonate. In some embodiments, the substrate is a glass substrate.

A substrate can be of any desired shape. For example, a substrate can be typically a thin (e.g., sub-centimeter), flat shape (e.g., square, rectangle or a circle). In some embodiments, a substrate structure has rounded corners (e.g., for increased safety or robustness). In some embodiments, a substrate structure has one or more cut-off corners (e.g., for use with a slide clamp or cross-table). In some embodiments, where a substrate structure is flat, the substrate structure can be any appropriate type of support having a flat surface (e.g., a chip, wafer, e.g., a silicon-based wafer, die, or a slide such as a microscope slide).

In some embodiments, a substrate comprising an array of molecules is provided, e.g., in the form of a lawn of polymers (e.g., oligonucleotides), or polymers arranged on the substrate in a pattern. Examples of polymers on an array may include, but are not limited to, nucleic acids, peptides, phospholipids, polysaccharides, heteromacromolecules in which one moiety is covalently bound to any of the above, polyurethanes, polyesters, polycarbonates, polyureas, polyamides, polyethyleneimines, polyarylene sulfides, polysiloxanes, polyimides, and polyacetates. The molecules occupying different features of an array typically differ from one another, although some redundancy in which the same polymer occupies multiple features can be useful as a control. For example, in a nucleic acid array, the nucleic acid molecules within the same feature are typically the same or share one or more sequences, whereas nucleic acid molecules occupying different features are mostly different from one another.

In addition to those above, a wide variety of other features can be used to form the arrays described herein. For example, in some embodiments, features that are formed from polymers and/or biopolymers that are jet printed, screen printed, or electrostatically deposited on a substrate can be used to form arrays.

In some examples, the molecules on the array may be nucleic acids, such as oligonucleotides. The oligonucleotide can be single-stranded or double-stranded. Nucleic acid molecules on an array may be DNA or RNA. The DNA may be single-stranded or double-stranded. The DNA may include, but are not limited to, mitochondrial DNA, cell-free DNA, complementary DNA (cDNA), genomic DNA, plasmid DNA, cosmid DNA, bacterial artificial chromosome (BAC), or yeast artificial chromosome (YAC). The RNA may include, but are not limited to, mRNAs, tRNAs, snRNAs, rRNAs, retroviruses, small non-coding RNAs, microRNAs, polysomal RNAs, pre-mRNAs, intronic RNA, viral RNA, cell free RNA and fragments thereof. The non-coding RNA, or ncRNA can include snoRNAs, microRNAs, siRNAs, piRNAs and long nc RNAs.

The oligonucleotide, such as a first oligonucleotide, a second oligonucleotide, a third oligonucleotide, etc., is at least about 4 nucleotides in length, such as at least any of about 5, 6, 7, 8, 9, 10, 11, 12, 13, 14, 15, 16, 17, 18, 19, 20, 25, 30, 35, 40, 45, 50, 55, 60, 65, 70, 75, 70, or more, nucleotides in length. In some embodiments, the oligonucleotide is at least 4 nucleotides in length. In some embodiments, the oligonucleotide is less than about 70 nucleotides in length, such as less than any of about 65, 60, 55, 50, 45, 40, 35, 30, 25, 20, 19, 18, 17, 16, 15, 14, 13, 12, 11, 10, 9, 8, 7, 8, 6, 5, 4, or fewer, nucleotides in length. In some embodiments, the oligonucleotide is between about 4 and about 70 nucleotides in length, such as between about 4 and about 10 nucleotides in length, between about 5 and about 20 nucleotides in length, between about 10 and about 50 nucleotides in length, and between about 30 and about 70 nucleotides in length.

In some embodiments, the molecules on an array comprise oligonucleotide barcodes. The barcode sequences are optional. In some embodiments, a first oligonucleotide comprises a first barcode sequence. In some embodiments, a second oligonucleotide comprises a second barcode sequence. In some embodiments, a third oligonucleotide comprises a third barcode sequence. In some embodiments, the first oligonucleotide comprises a first barcode sequence but the second oligonucleotide does not comprise a barcode sequence. In some embodiments, the first oligonucleotide comprises a first barcode sequence and the second oligonucleotide comprises a second barcode sequence. In some embodiments, the first oligonucleotide comprises a first barcode sequence but the third oligonucleotide does not comprise a barcode sequence. In some embodiments, the first oligonucleotide comprises a first barcode sequence and the third oligonucleotide comprises a third barcode sequence. In some embodiments, the second oligonucleotide comprises a second barcode sequence but the third oligonucleotide does not comprise a barcode sequence. In some embodiments, the second oligonucleotide comprises a second barcode sequence and the third oligonucleotide comprises a third barcode sequence.

In some embodiments, each of the first, second, and third oligonucleotides comprise a barcode sequence (e.g., a first, second, and third barcode sequence, respectively). In some embodiments, the barcode sequence on the first oligonucleotide (e.g., a first barcode sequence) is different from the barcode sequence on the second oligonucleotide (e.g., a second barcode sequence). In some embodiments, the barcode sequence on the first oligonucleotide is different from the barcode sequence on the third oligonucleotide (e.g., a third barcode sequence). In some embodiments, the barcode sequence on the second oligonucleotide is different from the barcode sequence on the third oligonucleotide. In some embodiments, each of the first, second, and third oligonucleotide barcode sequences are different.

A barcode sequence can be of varied length. In some embodiments, the barcode sequence is about 3, about 4, about 5, about 6, about 7, about 8, about 9, about 10, about 11, about 12, about 13, about 14, about 15, about 16, about 17, about 18, about 19, about 20, about 21, about 22, about 23, about 24, about 25, about 30, about 35, about 40, about 45, about 50, about 55, about 60, about 65, or about 70 nucleotides in length. In some embodiments, the barcode sequence is between about 4 and about 25 nucleotides in length. In some embodiments, the barcode sequences is between about 10 and about 50 nucleotides in length. The nucleotides can be completely contiguous, e.g., in a single contiguous stretch of adjacent nucleotides, or they can be separated into two or more separate subsequences that are separated by 1 or more nucleotides. In some embodiments, the barcode sequence can be about 4, about 5, about 6, about 7, about 8, about 9, about 10, about 11, about 12, about 13, about 14, about 15, about 16, about 17, about 18, about 19, about 20, about 21, about 22, about 23, about 24, about 25 nucleotides or longer. In some embodiments, the barcode sequence can be at least about 4, about 5, about 6, about 7, about 8, about 9, about 10, about 11, about 12, about 13, about 14, about 15, about 16, about 17, about 18, about 19, about 20, about 21, about 22, about 23, about 24, about 25 nucleotides or longer. In some embodiments, the barcode sequence can be at most about 4, about 5, about 6, about 7, about 8, about 9, about 10, about 11, about 12, about 13, about 14, about 15, about 16, about 17, about 18, about 19, about 20, about 21, about 22, about 23, about 24, about 25 nucleotides or shorter.

The oligonucleotide can include one or more (e.g., two or more, three or more, four or more, five or more) Unique Molecular Identifiers (UMIs). A unique molecular identifier is a contiguous nucleic acid segment or two or more non-contiguous nucleic acid segments that function as a label or identifier for a particular analyte, or for a capture probe that binds a particular analyte (e.g., via the capture domain). A UMI can be unique. A UMI can include one or more specific polynucleotides sequences, one or more random nucleic acid and/or amino acid sequences, and/or one or more synthetic nucleic acid and/or amino acid sequences. In some embodiments, the UMI is a nucleic acid sequence that does not substantially hybridize to analyte nucleic acid molecules in a biological sample. In some embodiments, the UMI has less than 90% sequence identity (e.g., less than 80%, 70%, 60%, 50%, or less than 40% sequence identity) to the nucleic acid sequences across a substantial part (e.g., 80% or more) of the nucleic acid molecules in the biological sample.

The UMI can include from about 6 to about 20 or more nucleotides within the sequence of capture probes, e.g., barcoded oligonucleotides in an array generated using a method disclosed herein. In some embodiments, the length of a UMI sequence can be about 6, 7, 8, 9, 10, 11, 12, 13, 14, 15, 16, 17, 18, 19, 20 nucleotides or longer. In some embodiments, the length of a UMI sequence can be at least about 6, 7, 8, 9, 10, 11, 12, 13, 14, 15, 16, 17, 18, 19, 20 nucleotides or longer. In some embodiments, the length of a UMI sequence is at most about 6, 7, 8, 9, 10, 11, 12, 13, 14, 15, 16, 17, 18, 19, 20 nucleotides or shorter. These nucleotides can be contiguous, e.g., in a single stretch of adjacent nucleotides, or they can be separated into two or more separate subsequences that are separated by 1 or more nucleotides. Separated UMI subsequences can be from about 4 to about 16 nucleotides in length. In some embodiments, the UMI subsequence can be about 4, 5, 6, 7, 8, 9, 10, 11, 12, 13, 14, 15, 16 nucleotides or longer. In some embodiments, the UMI subsequence can be at least about 4, 5, 6, 7, 8, 9, 10, 11, 12, 13, 14, 15, 16 nucleotides or longer. In some embodiments, the UMI subsequence can be at most about 4, 5, 6, 7, 8, 9, 10, 11, 12, 13, 14, 15, 16 nucleotides or shorter.

In some embodiments, a UMI is attached to other parts of a nucleic acid in a reversible or irreversible manner. In some embodiments, a UMI is added to, for example, a fragment of a DNA or RNA sample before, during, and/or after sequencing of the analyte. In some embodiments, a UMI allows for identification and/or quantification of individual sequencing-reads. In some embodiments, a UMI is a used as a fluorescent barcode for which fluorescently labeled oligonucleotide probes hybridize to the UMI.

In some embodiments, a method provided herein further comprises a step of providing the substrate. A wide variety of different substrates can be used for the foregoing purposes. In general, a substrate can be any suitable support material. The substrate may comprise materials of one or more of the IUPAC Groups 4, 6, 11, 12, 13, 14, and 15 elements, plastic material, silicon dioxide, glass, fused silica, mica, ceramic, or metals deposited on the aforementioned substrates. Exemplary substrates include, but are not limited to, glass, modified and/or functionalized glass, hydrogels, films, membranes, plastics (including e.g., acrylics, polystyrene, copolymers of styrene and other materials, polypropylene, polyethylene, polybutylene, polyurethanes, Teflon^{™}, cyclic olefins, polyimides etc.), nylon, ceramics, resins, Zeonor, silica or silica-based materials including silicon and modified silicon, carbon, metals, inorganic glasses, optical fiber bundles, and polymers, such as polystyrene, cyclic olefin copolymers (COCs), cyclic olefin polymers (COPs), polypropylene, polyethylene and polycarbonate.

A substrate can be of any desired shape. For example, a substrate can be typically a thin, flat shape (e.g., a square or a rectangle). In some embodiments, a substrate structure has rounded corners (e.g., for increased safety or robustness). In some embodiments, a substrate structure has one or more cut-off corners (e.g., for use with a slide clamp or cross-table). In some embodiments, where a substrate structure is flat, the substrate structure can be any appropriate type of support having a flat surface (e.g., a chip or a slide such as a microscope slide).

### III. GENERATION OF MOLECULAR ARRAYS

### A. Light-Controlled Surface Patterning

Provided herein in some embodiments are methods and uses of photo-hybridization-ligation combinatorial barcode generation using any suitable light-controlled surface patterning (e.g., photoresist, polymers, caged oligonucleotides, etc.) and photolithography. For example, a method disclosed herein may comprise photocontrollable ligation, wherein local irradiation causes degradation of photoresist and oligonucleotides to be exposed for ligation. In some aspects, a method disclosed herein provides one or more advantages as compared to available arraying methods. For example, a large diversity of barcodes can be created via sequential rounds of UV exposure, hybridization, ligation, removal and reapplication using light-controlled surface patterning; a protection/deprotection step is not necessarily required for ligating oligonucleotides to the substrate; the feature size can be controlled using photomasks; and the generated array at any discrete location can be known across all arrays with no decoding needed.

The methods of the present disclosure comprise irradiating a substrate to render oligonucleotide molecules in one or more regions on the substrate available for oligonucleotide attachment, whereas oligonucleotide molecules in one or more other regions on the substrate are not available for oligonucleotide attachment. In some embodiments, the irradiation is selective. For example, one or more photomasks can be used such that only one or more specific regions of the array are exposed to stimuli (*e.g.*, exposure to light such as UV, and/or exposure to heat induced by laser). In some embodiments, the method comprises irradiating oligonucleotide molecules in one or more regions with a first light exposure while oligonucleotide molecules in one or more other regions are not irradiated with the first light exposure. For instance, the substrate is exposed to the first light exposure when the oligonucleotide molecules in the one or more other regions are photomasked while the oligonucleotide molecules in the one or more regions are not photomasked. Alternatively, a focused light exposure such as one provided using a laser may be used to irradiate the oligonucleotide molecules in the one or more regions but not the oligonucleotide molecules in the one or more other regions, even when the oligonucleotide molecules in the one or more other regions are not masked from the light. For example, the distance (e.g. pitch) between features may be selected to prevent the laser from stimulating oligonucleotides of an adjacent feature.

In some embodiments, during and after the irradiation, the oligonucleotide molecules in the one or more other regions are protected from hybridization (e.g., hybridization to a splint and/or an oligonucleotide molecule). In some embodiments, during and after the irradiation, the oligonucleotide molecules in the one or more other regions are protected from ligation (e.g., hybridization to a splint and/or an oligonucleotide molecule). In some embodiments, during and after the irradiation, the oligonucleotide molecules in the one or more other regions are protected from hybridization and ligation. Various strategies for the protection of the oligonucleotides from hybridization and/or ligation are contemplated herein. Any suitable strategy may be used.

The irradiating may be multiplexed. In some embodiments, the method comprises irradiating the substrate in multiple cycles. For instance, each cycle of irradiating may comprise irradiating one or more sub-regions that are different from the one or more sub-regions irradiated in another cycle. In some embodiments, the method further comprises translating a photomask from a first position to a second position relative to the substrate, each position for a cycle of irradiating the substrate.

In some aspects, following the irradiation, the method comprises attaching an oligonucleotide of at least four nucleotides in length to an oligonucleotide molecule in a sub-region to generate an immobilized nucleic acid on the substrate. In some embodiments, a Round 1 oligonucleotide comprises a first barcode sequence. In some embodiments, a Round 2 oligonucleotide comprises a second barcode sequence. In some embodiments, a Round 3 oligonucleotide comprises a third barcode sequence. Thus, in some embodiments, the immobilized nucleic acid generated after Rounds 1-3 comprises the first, second, and third barcode sequences.

In some embodiments, the first, second, and/or third oligonucleotide comprises a sequence that hybridizes to a splint which in turn hybridizes to an oligonucleotide molecule immobilized on the substrate. In some embodiments, the first oligonucleotide comprises a sequence that hybridizes to a splint which in turn hybridizes to an oligonucleotide molecule (e.g., a primer or a partial primer) immobilized on the substrate. In some embodiments, the second oligonucleotide comprises a sequence that hybridizes to a splint which in turn hybridizes to the first oligonucleotide. In some embodiments, the second oligonucleotide further comprises a sequence that hybridizes to a splint which in turn hybridizes to the third oligonucleotide. In some embodiments, the third oligonucleotide comprises a sequence that hybridizes to a splint which in turn hybridizes to the second oligonucleotide. In some embodiments, the third oligonucleotide further comprises a sequence that hybridizes to a splint which in turn hybridizes to the fourth oligonucleotide. In some embodiments, the fourth oligonucleotide comprises a sequence that hybridizes to a splint which in turn hybridizes to the third oligonucleotide.

In some embodiments, the first, second, and/or third oligonucleotide is at least about 4 nucleotides in length, such as at least any of about 5, 6, 7, 8, 9, 10, 11, 12, 13, 14, 15, 16, 17, 18, 19, 20, 25, 30, 35, 40, 45, 50, 55, 60, 65, 70, 75, 70, or more, nucleotides in length. In some embodiments, the first, second, and/or third oligonucleotide is at least 4 nucleotides in length. In some embodiments, the first, second, and/or third oligonucleotide is less than about 70 nucleotides in length, such as less than any of about 65, 60, 55, 50, 45, 40, 35, 30, 25, 20, 19, 18, 17, 16, 15, 14, 13, 12, 11, 10, 9, 8, 7, 8, 6, 5, 4, or fewer, nucleotides in length. In some embodiments, the first, second, and/or third oligonucleotide is between about 4 and about 70 nucleotides in length, such as between about 4 and about 10 nucleotides in length, between about 5 and about 20 nucleotides in length, between about 10 and about 50 nucleotides in length, and between about 30 and about 70 nucleotides in length.

As described herein, the immobilized nucleic acid generated using a method provided herein can provide a higher resolution and/or higher barcode accuracy compared to certain methods of light mediated base-by-base *in situ* synthesis.

**FIG. 1** provides a non-limiting example of a method for generation of an *in situ* oligonucleotide array using hybridization/ligation. Oligonucleotides on an array can be blocked (e.g., bound by photo-cleavable polymers, comprising photo-cleavable moieties, or covered by a photoresist). For example, a photoresist coating can be applied to a substrate comprising nucleic acid molecules, such as an oligo lawn. The substrate is subjected to patterned UV exposure (e.g., through a photomask) to selectively deblock oligonucleotides at pre-determined positions on the substrate. The patterned UV exposure may be achieved by irradiating the substrate with UV light through a photomask. After deblocking, oligonucleotides comprising a first part of a barcode are applied to the substrate, and hybridized to splints that hybridize to the deblocked oligonucleotide molecules (e.g., on an oligo lawn). The oligonucleotides may be ligated to the deblocked oligonucleotide molecules at the pre-determined positions. The deblocking-hybridization-ligation steps can be repeated for *N* cycles in each round, each cycle for one or more different pre-determined positions on the substrate. A round may be repeated ***M* times** to ligate ***M*** parts of the barcode onto the substrate, generating a nucleotide array with ***N^{M}*** sequence diversity.

**FIGS. 2A-2B** further provide schematic demonstration of exemplary methods for synthesis of barcoded oligonucleotides. In **FIG. 2A****,** features are shown in circles. During cycle 1 of round 1, a first part of a barcode is attached to oligonucleotide molecules in a first set of features. Cycle 2 of round 1 attaches a first part of a barcode to a different set of features, wherein the sequence of the oligonucleotide attached to the different sets of features may be different. Upon completion of ***N*** cycles in round 1, all features are ligated with the first part of a barcode. Similarly, upon completion of ***N*** cycles in round ***M*,** all features are ligated with the ***M***^{th} part of a barcode, and an array with ***N^{M}*** barcode diversity is generated. Oligonucleotide probes for capturing analytes or proxies thereof may be generated using a method disclosed herein, for example, using two, three, four, or more rounds of hybridization and ligation, e.g. as shown in **FIG. 2B****.** Different components of immobilized oligonucleotides can be installed in sequential rounds. For instance, a first oligonucleotide comprising a first barcode sequence is installed which further comprises a sequence that hybridizes to a first splint, which in turn hybridizes to an immobilized oligonucleotide on a substrate). The first oligonucleotide is then ligated to the immobilized oligonucleotide using the first splint as template, extending the immobilized oligonucleotide on the substrate. A second oligonucleotide comprising a second barcode sequence is installed which comprises a sequence that hybridizes to a second splint which in turn hybridizes to the extended oligonucleotide molecule. The second oligonucleotide is then ligated to the extended oligonucleotide using the second splint as template, further extending the immobilized oligonucleotide on the substrate, thereby generating a further extended oligonucleotide molecule. A third oligonucleotide comprising a third barcode sequence is installed which comprises a sequence that hybridizes to a third splint which in turn hybridizes to the further extended oligonucleotide molecule. The third oligonucleotide is then ligated to the further extended oligonucleotide using the third splint as template, thereby generating an even further extended immobilized oligonucleotide. Optionally, a fourth oligonucleotide comprising a fourth barcode sequence, UMI, and a capture sequence, is installed by photo-hybridization ligation, and comprises a sequence that hybridizes to a fourth splint which in turn hybridizes to the further extended oligonucleotide molecule. The fourth oligonucleotide is then ligated to the even further extended oligonucleotide using the fourth splint as template, further extending the immobilized oligonucleotide.

### a. Photoresist

In some embodiments, a method disclosed herein comprises (a) irradiating a substrate comprising an unmasked first region and a masked second region, whereby a photoresist in the first region is degraded to render oligonucleotide molecules in the first region available for hybridization and/or ligation, whereas oligonucleotide molecules in the second region are protected by a photoresist in the second region from hybridization and/or ligation; and (b) attaching an oligonucleotide comprising a barcode sequence to oligonucleotide molecules in the first region via hybridization and/or ligation, wherein oligonucleotide molecules in the second region do not receive the barcode sequence, thereby providing on the substrate an array comprising different oligonucleotide molecules in the first and second regions.

In some embodiments, oligonucleotide molecules on the substrate comprise one or more common sequences. In some embodiments, oligonucleotide molecules on the substrate comprise functional groups. In some embodiments, the functional groups are not protected by a photo-sensitive moiety prior to the irradiating step. In some embodiments, the functional groups are 3' hydroxyl groups of nucleotides. In some embodiments, the method further comprises forming a pattern of oligonucleotide molecules on the substrate prior to applying the photoresist to the substrate. In some embodiments, forming the pattern of oligonucleotide molecules comprises: irradiating a substrate comprising a plurality of functional groups and a photoresist through a patterned mask, whereby the photoresist in a first region of the substrate is degraded, rendering functional groups in the first region available for reacting with functional groups in functionalized oligonucleotide molecules, whereas functional groups in a second region of the substrate are protected by the photoresist from reacting with functional groups in the oligonucleotide molecules; and contacting the substrate with the functionalized oligonucleotide molecules, wherein the functionalized oligonucleotide molecules are coupled to functional groups in the first region but not to functional groups in the second region, thereby forming a pattern of oligonucleotide molecules on the substrate. In some embodiments, the functional groups in the functionalized oligonucleotide molecules are amino groups. In some embodiments, the method further comprises rendering the reaction between functional groups of the substrate and the functionalized oligonucleotide molecules irreversible. In some embodiments, the irradiating and contacting steps are repeated in one or more cycles. In some embodiments, the photoresist is not removed prior to, during, or between the one or more cycles. In some embodiments, the substrate is irradiated through a patterned mask. In some embodiments, the method comprises removing the patterned mask after the irradiating step, wherein the same patterned mask is re-used in a subsequent cycle of the irradiating and contacting steps. In some embodiments, the photoresist in the first region of the substrate is dissolved by a developer and removed. In some embodiments, the barcode sequence is between about 4 and about 25 nucleotides in length. In some embodiments, the oligonucleotide comprising the barcode sequence is between about 10 and about 50 nucleotides in length. In some embodiments, the oligonucleotide comprising the barcode sequence is hybridized to an oligonucleotide molecule in the first region. In some embodiments, the oligonucleotide comprising the barcode sequence is ligated to an oligonucleotide molecule in the first region. In some embodiments, the oligonucleotide comprising the barcode sequence is hybridized to a splint which is in turn hybridized to an oligonucleotide molecule in the first region. In some embodiments, the method further comprises ligating the oligonucleotide comprising the barcode sequence to the oligonucleotide molecule to generate a barcoded oligonucleotide molecule in the first region. In some embodiments, the method further comprises blocking the 3' or 5' termini of barcoded oligonucleotide molecules and/or unligated oligonucleotide molecules in the first region from ligation. In some embodiments, the photoresist is not removed prior to, during, or between any of the cycles. In some embodiments, the feature is no more than 10 microns in diameter.

In some embodiments, a method disclosed herein comprises (a) irradiating a substrate comprising an unmasked first region and a masked second region, whereby a photoresist in the first region is degraded to render oligonucleotide molecules in the first region available for hybridization and/or ligation, whereas oligonucleotide molecules in the second region are protected by the photoresist in the second region from hybridization and/or ligation; and (b) contacting oligonucleotide molecules in the first region with a first splint and a first oligonucleotide comprising a first barcode sequence, wherein the first splint hybridizes to the first oligonucleotide and the oligonucleotide molecules in the first region, wherein the first oligonucleotide is ligated to the oligonucleotide molecules in the first region, and the first oligonucleotide is not ligated to oligonucleotide molecules in the second region, thereby providing on the substrate an array comprising different oligonucleotide molecules in the first and second regions. In some embodiments, the photoresist is a first photoresist, and the first oligonucleotide is ligated to the oligonucleotide molecules in the first region to generate first extended oligonucleotide molecules, and the method further comprises: (c) applying a second photoresist to the substrate, optionally wherein the second photoresist is applied after the first photoresist is removed from the substrate; (d) irradiating the substrate while the first region is masked and the second region is unmasked, whereby the first or second photoresist in the second region is degraded to render oligonucleotide molecules in the second region available for hybridization and/or ligation, whereas the first extended oligonucleotide molecules in the first region are protected by the second photoresist in the first region from hybridization and/or ligation; and (e) contacting oligonucleotide molecules in the second region with a second splint and a second oligonucleotide comprising a second barcode sequence, wherein the second splint hybridizes to the second oligonucleotide and the oligonucleotide molecules in the second region, wherein the second oligonucleotide is ligated to the oligonucleotide molecules in the second region to generate second extended oligonucleotide molecules, and the second oligonucleotide is not ligated to the first extended oligonucleotide molecules in the first region.

In some embodiments, the oligonucleotide molecules are protected from hybridization by a photoresist covering the oligonucleotide molecules. In some embodiments, the oligonucleotide molecules are protected from ligation by a photoresist covering the oligonucleotide molecules. In some embodiments, the oligonucleotide molecules are protected from hybridization and ligation by a photoresist covering the oligonucleotide molecules. In some embodiments, the photoresist in irradiated regions is removed. In some embodiments, the photoresist in masked or non-irradiated regions is not removed.

In some aspects, a photoresist is a light-sensitive material used in processes (such as photolithography and photoengraving) to form a pattern on a surface. A photoresist may comprise a polymer, a sensitizer, and/or a solvent. The photoresist composition used herein is not limited to any specific proportions of the various components. Photoresists can be classified as positive or negative. In positive photoresists, the photochemical reaction that occurs during light exposure weakens the polymer, making it more soluble to developer, so a positive pattern is achieved. In the case of negative photoresists, exposure to light causes polymerization of the photoresist, and therefore the negative photoresist remains on the surface of the substrate where it is exposed, and the developer solution removes only the unexposed areas. In some embodiments, the photoresist used herein is a negative photoresist. In some embodiments, the photoresist used herein is a positive photoresist. In some embodiments, the photoresist is removable with UV light.

The photoresist may experience changes in pH upon irradiation. In some embodiments, the photoresist in one or more regions comprises a photoacid generator (PAG). In some embodiments, the photoresist in one or more other regions comprises a PAG. In some embodiments, the photoresist in the one or more regions and the one or more other regions comprises a PAG. In some embodiments, the photoresist in the one or more regions and the one or more other regions comprises the same PAG. In some embodiments, the photoresist in the one or more regions and the one or more other regions comprises different PAG. In some embodiments, the PAG or PAGs irreversibly release protons upon absorption of light. PAGs may be used as components of photocurable polymer formulations and chemically amplified photoresists. Examples of PAGs include triphenylsulfonium triflate, diphenylsulfonium triflate, diphenyliodonium nitrate, N-Hydroxynaphthalimide triflate, triarylsulfonium hexafluorophosphate salts, N-hydroxy-5-norbornene-2,3-dicarboximide perfluoro-1-butanesulfonate, bis(4-tert-butylphenyl)iodonium perfluoro-1-butanesulfonate, etc.

In some embodiments, the photoresist further comprises an acid scavenger. In some embodiments, the photoresist in the one or more regions and the one or more other regions comprises the same acid scavenger. In some embodiments, the photoresist in the one or more regions and the one or more other regions comprises different acid scavengers. In some embodiments, an acid scavenger acts to neutralize, adsorb and/or buffer acids, and may comprise a base or alkaline compound. In some embodiments, acid scavengers act to reduce the amount or concentration of protons or protonated water. In some embodiments, an acid scavenger acts to neutralize, diminish, or buffer acid produced by a PAG. In some embodiments, an acid scavenger exhibits little or no stratification over time or following exposure to heat. In some embodiments, acid scavengers may be further subdivided into "organic bases" and "polymeric bases." A polymeric base is an acid scavenger (e.g., basic unit) attached to a longer polymeric unit. A polymer is typically composed of a number of coupled or linked monomers. The monomers can be the same (to form a homopolymer) or different (to form a copolymer). In a polymeric base, at least some of the monomers act as acid scavengers. An organic base is a base which is joined to or part of a non-polymeric unit. Non-limiting examples of organic bases include, without limitation, amine compounds (e.g., primary, secondary and tertiary amines). Generally any type of acid scavenger, defined here as a traditional Lewis Base, an electron pair donor, can be used in accordance with the present disclosure.

In some embodiments, the photoresist further comprises a base quencher. Base quenchers may be used in photoresist formulations to improve performance by quenching reactions of photoacids that diffuse into unexposed regions. Base quenchers may comprise aliphatic amines, aromatic amines, carboxylates, hydroxides, or combinations thereof. Examples of base quenchers include but are not limited to, trioctylamine, 1,8-diazabicyclo[5.4.0]undec-7-ene (DBU), 1-piperidineethanol (1PE), tetrabutylammonium hydroxide (TBAH), dimethylamino pyridine, 7-diethylamino-4-methyl coumarin (Coumarin 1), tertiary amines, sterically hindered diamine and guanidine bases such as 1,8-bis(dimethylamino)naphthalene (PROTON SPONGE), berberine, or polymeric amines such as in the PLURONIC or TETRONIC series commercially available from BASF. In some embodiments, the photoresist in the one or more regions and the one or more other regions comprises the same base quencher. In some embodiments, the photoresist in the one or more regions and the one or more other regions comprises different base quenchers.

In some embodiments, the photoresist further comprises a photosensitizer. A photosensitizer is a molecule that produces a chemical change in another molecule in a photochemical process. Photosensitizers are commonly used in polymer chemistry in reactions such as photopolymerization, photocrosslinking, and photodegradation. Photosensitizers generally act by absorbing ultraviolet or visible region of electromagnetic radiation and transferring it to adjacent molecules. In some embodiments, photosensitizer shifts the photo sensitivity to a longer wavelength of electromagnetic radiation. The sensitizer, also called a photosensitizer, is capable of activating the PAG at, for example, a longer wavelength of light in accordance with an aspect of the present disclosure. Preferably, the concentration of the sensitizer is greater than that of the PAG, such as 1.1 times to 5 times greater, for example, 1.1 times to 3 times greater the concentration of PAG. Exemplary sensitizers suitable for use in the disclosure include but are not limited to, isopropylthioxanthone (ITX) and 10H-phenoxazine (PhX). In some embodiments, the photoresist in the one or more regions and the one or more other regions comprises the same photosensitizer. In some embodiments, the photoresist in the one or more regions and the one or more other regions comprises different photosensitizers.

In some embodiments, the photoresist further comprises a matrix. The matrix generally refers to polymeric materials that may provide sufficient adhesion to the substrate when the photoresist formulation is applied to the top surface of the substrate, and may form a substantially uniform film when dissolved in a solvent and spread on top of a substrate. Examples of a matrix may include, but are not limited to, polyester, polyimide, polyethylene naphthalate (PEN), polyvinyl chloride (PVC), polymethylmethacrylate (PMMA), polyglycidalmethacrylate (PGMA), and polycarbonate, or a combination thereof. The matrix may be chosen based on the wavelength of the radiation used for the generation of acid when using the photoresist formulation, the adhesion properties of the matrix to the top surface of the substrate, the compatibility of the matrix to other components of the formulation, and the ease of removable or degradation (if needed) after use. In some embodiments, the photoresist in the one or more regions and the one or more other regions comprises the same matrix. In some embodiments, the photoresist in the one or more regions and the one or more other regions comprises different matrices.

In some embodiments, the photoresist further comprises a surfactant. Surfactants may be used to improve coating uniformity, and may include ionic, non-ionic, monomeric, oligomeric, and polymeric species, or combinations thereof. Examples of possible surfactants include fluorine-containing surfactants such as the FLUORAD series available from 3M Company in St. Paul, Minn., and siloxane-containing surfactants such as the SILWET series available from Union Carbide Corporation in Danbury, Conn. In some embodiments, the photoresist in the one or more regions and the one or more other regions comprises the same surfactant. In some embodiments, the photoresist in the one or more regions and the one or more other regions comprises different surfactants.

In some embodiments, the photoresist further comprises a casting solvent. A casting solvent may be used so that the photoresist may be applied evenly on the substrate surface to provide a defect-free coating. Examples of suitable casting solvents may include ethers, glycol ethers, aromatic hydrocarbons, ketones, esters, ethyl lactate, γ-butyrolactone, cyclohexanone, ethoxyethylpropionate (EEP), a combination of EEP and gamma-butyrolactone (GBL), and propylene glycol methyl ether acetate (PGMEA). In some embodiments, the photoresist in the one or more regions and the one or more other regions comprises the same casting solvent. In some embodiments, the photoresist in the one or more regions and the one or more other regions comprises different casting solvents.

Methods of applying photoresist to the substrate include, but are not limited to, dipping, spreading, spraying, or any combination thereof. In some embodiments, the photoresist is applied via spin coating, thereby forming a photoresist layer on the substrate.

In some embodiments, the photoresist is in direct contact with the oligonucleotides on the substrate. In some embodiments, the oligonucleotide molecules on the substrate are embedded in the photoresist. In some embodiments, the photoresist is not in direct contact with the oligonucleotides. In some embodiments, oligonucleotide molecules on the substrate are embedded in an underlayer that is underneath the photoresist. For example, oligonucleotide molecules on the substrate may be embedded in a soluble polymer underlayer (e.g., a soluble polyimide underlayer (XU-218)), and the photoresist forms a photoresist layer on top of the underlayer.

In some embodiments, the photoresist may be removed and re-applied one or more times. For example, the photoresist may be stripped from the substrate and/or the oligonucleotides ligated to the substrate. Removal of photoresist can be accomplished with various degrees of effectiveness. In some embodiments, the photoresist is completely removed from the substrate and/or the oligonucleotides ligated to the substrate before re-application. Methods of removing photoresist may include, but are not limited to, using organic solvent mixtures, using liquid chemicals, exposure to a plasma environment, or other dry techniques such as UV/O₃ exposure. In some embodiments, the photoresist is stripped using organic solvent.

In some embodiments, one or more photomasks may be used to selectively remove photoresist on the substrate. The mask is designed in such a way that the exposure sites can be selected, and thus specify the coordinates on the array where each oligonucleotide can be attached. The process can be repeated, a new mask is applied activating different sets of sites and coupling different barcodes, allowing oligonucleotide molecules to be constructed at each site. This process can be used to synthesize hundreds of thousands or millions of different oligonucleotides. In some embodiments, the substrate is irradiated through a patterned mask. The mask may be an opaque plate or film with transparent areas that allow light to shine through in a pre-defined pattern. After the irradiation step, the mask may be removed, translated to a different region on the substrate, or rotated. In some embodiments, a different photomasking pattern may be used in each barcoding round. In some embodiments, the same photomasking pattern may be used in each barcoding round. Using a series of photomasks, photoresist in desired regions of the substrate may be iteratively irradiated and subsequently removed.

The material of the photomask used herein may comprise silica with chrome in the opaque part. For example, the photomask may be transparent fused silica blanks covered with a pattern defined with a chrome metal absorbing film. The photomask may be used at various irradiation wavelengths, which include but are not limited to, 365 nm, 248 nm, and 193 nm. In some embodiments, the irradiation step herein can be performed for a duration of between about 1 minute and about 10 minutes, for example, for about 2 minutes, about 4 minutes, about 6 minutes, or about 8 minutes. In some embodiments, the irradiation can be performed at a total light dose of between about one and about ten mW/mm², for example, at about 2 mW/mm², about 4 mW/mm², about 6 mW/mm², or about 8 mW/mm². In some embodiments, the irradiation can be performed at a total light dose of between about one and about ten mW/mm² and for a duration of between about 1 minute and about 10 minutes.

### b. Polymers

In some embodiments, a method disclosed herein comprises irradiating a first polynucleotide immobilized on a substrate with a first light exposure while a second polynucleotide immobilized on the substrate is not irradiated with the first light exposure, wherein the first polynucleotide is bound to a first photo-cleavable polymer that inhibits or blocks hybridization and/or ligation to the first polynucleotide, and the second polynucleotide is bound to a second photo-cleavable polymer that inhibits or blocks hybridization and/or ligation to the second polynucleotide, thereby cleaving the first photo-cleavable polymer such that the inhibition or blocking of hybridization and/or ligation to the first polynucleotide is reduced or eliminated, whereas hybridization and/or ligation to the second polynucleotide remains inhibited or blocked by the second photo-cleavable polymer, wherein a first barcode is attached to the first polynucleotide via hybridization and/or ligation, thereby providing on the substrate an array comprising the first and second polynucleotides, wherein the first polynucleotide is barcoded with the first barcode and the second polynucleotide is not barcoded with the first barcode.

In some embodiments, the method further comprises irradiating the second polynucleotide with a second light exposure, thereby cleaving the second photo-cleavable polymer such that the inhibition or blocking of hybridization and/or ligation to the second polynucleotide is reduced or eliminated. In some embodiments, the second polynucleotide is irradiated with the second light exposure while the first polynucleotide is not irradiated with the second light exposure. In some embodiments, the method further comprises attaching a second barcode to the second polynucleotide via hybridization and/or ligation, thereby providing on the substrate an array comprising the first polynucleotide barcoded with the first barcode and the second polynucleotide barcoded with the second barcode. In some embodiments, hybridization and/or ligation to the first polynucleotide barcoded with the first barcode is inhibited or blocked. In some embodiments, the first barcode comprises a first photo-cleavable moiety that inhibits or blocks hybridization and/or ligation, thereby inhibiting or blocking hybridization and/or ligation to the first polynucleotide barcoded with the first barcode. In some embodiment, the first photo-cleavable moiety comprises a photo-caged nucleobase, a photo-cleavable linker, a photo-cleavable hairpin and/or a photo-caged 3'-hydroxyl group. In some embodiments, the first barcode is a DNA oligonucleotide. In some embodiments, the first barcode is between about 5 and about 20 nucleotides in length. In some embodiments, the substrate comprises a plurality of differentially barcoded polynucleotides immobilized thereon. In some embodiments, the irradiating comprises using a photomask to selectively irradiate the first polynucleotide or the second polynucleotide. In some embodiments, the attachment of the first barcode and/or the second barcode comprises ligating one end of the first/second barcode to one end of the first/second polynucleotide, respectively. In some embodiments, the attachment of the first barcode comprises hybridizing one end of the first barcode and one end of the first polynucleotide to a first splint. In some embodiments, the method further comprises ligating the first barcode to the first polynucleotide hybridized to the first splint. In some embodiments, the first barcode is directly ligated to the first polynucleotide, without gap filling. In some embodiments, ligating the first barcode to the first polynucleotide is preceded by gap filling. In some embodiments, the first splint is a DNA oligonucleotide at least 4 nucleotides in length. In some embodiments, the first photo-cleavable polymer and/or the second photo-cleavable polymer are UV degradable. In some embodiments, the first photo-cleavable polymer and/or the second photo-cleavable polymer comprise a polyethylenimine (PEI).

In some embodiments, a method disclosed herein comprises: (a1) irradiating polynucleotide P1 immobilized on a substrate with light while polynucleotide P2 immobilized on the substrate is photomasked, wherein polynucleotides P1 and P2 are bound to a photo-cleavable polymer that inhibits or blocks hybridization and/or ligation to P1 and P2, respectively, thereby cleaving the photo-cleavable polymer to allow hybridization and/or ligation to P1, whereas hybridization and/or ligation to P2 remain inhibited or blocked by the photo-cleavable polymer; and (b1) attaching barcode 1A to P1 via hybridization and/or ligation to form a barcoded polynucleotide 1A-P1, thereby providing on the substrate an array comprising polynucleotides 1A-P1 and P2. In some embodiments, the method further comprises: (c1) irradiating P2 with light, thereby cleaving the photo-cleavable polymer to allow hybridization and/or ligation to P2; and (d1) attaching barcode 1B to P2 via hybridization and/or ligation to form a barcoded polynucleotide 1B-P2, thereby providing on the substrate an array comprising barcoded polynucleotides 1A-P1 and 1B-P2. In some embodiments, polynucleotides of different nucleic acid sequences are immobilized on the substrate in a pattern comprising rows and columns prior to the irradiation.

In some embodiments, the oligonucleotide molecules are protected from hybridization by a polymer binding to the oligonucleotide molecules. In some embodiments, the oligonucleotide molecules are protected from ligation by a polymer binding to the oligonucleotide molecules. In some embodiments, the oligonucleotide molecules are protected from hybridization and ligation by a polymer binding to the oligonucleotide molecules. In some embodiments, the polymer is a photo-cleavable polymer. In some embodiments, the polymer (e.g., photo-cleavable polymer) binds to the oligonucleotide molecules in a non-sequence specific manner. In some embodiments, the photo-cleavable polymers in irradiated regions are cleaved and the photo-cleavable polymers in masked or non-irradiated regions are not cleaved.

In some embodiments, a photo-cleavable polymer disclosed herein is not part of an oligonucleotide. In some embodiments, a photo-cleavable polymer disclosed herein is not covalently bonded to an oligonucleotide. In some embodiments, a photo-cleavable polymer disclosed herein is noncovalently bound to an oligonucleotide. In some embodiments, the oligonucleotide is prevented from hybridization to a nucleic acid such as a splint. In some embodiments, a photo-cleavable polymer disclosed herein inhibits or blocks ligation to either end of the oligonucleotide, while hybridization of a nucleic acid to the oligonucleotide may or may not be inhibited or blocked. For example, the photo-cleavable polymer bound to an oligonucleotide may inhibit or block the 3' or 5' end of the oligonucleotide from chemical or enzymatic ligation, e.g., even when a splint may hybridize to the oligonucleotide in order to bring a ligation partner in proximity to the 3' or 5' end of the oligonucleotide. In some embodiments, the photo-cleavable polymer may cap the 3' or 5' end of the oligonucleotide.

In some embodiments, the photo-cleavable polymer is UV degradable. In some embodiments, the photo-cleavable polymer comprises a UV-degradable group (e.g., a UV-degradable functional moiety). In some embodiments, the UV-degradable group is within the backbone or at each subunit of the photo-cleavable polymer. In some embodiments, the UV-degradable group comprises a nitrobenzyl group, e.g., within a PEG (polyethylene glycol), a PDMS (polydimethylsiloxane), or a polyethylenimine (PEI), for example, in the polymer backbone or at each subunit. Complete cleavage of the nitrobenzyl group(s) is not required for nucleic acid release. In some embodiments, cleavage of a portion of the UV-degradable groups is sufficient to render the oligonucleotides available for hybridization and/or ligation.

In some embodiments, the photo-cleavable polymer is synthetic, semi-synthetic, or natural. In some embodiments, the photo-cleavable polymer comprises a material selected from the group consisting of a PEG (polyethylene glycol), a PDMS (polydimethylsiloxane), a polyethylenimine (PEI), a polyacrylate, a lipid, a nanoparticle, a DNA, an RNA, a synthetic oligodeoxynucleotide (ODN), a xeno nucleic acid (XNA), a peptide nucleic acid (PNA), a locked nucleic acid (LNA), a 1,5-anhydrohexitol nucleic acid (HNA), a cyclohexene nucleic acid (CeNA), a threose nucleic acid (TNA), a glycol nucleic acid (GNA), a fluoro arabino nucleic acid (FANA), and a polypeptide. In some embodiments, the polyacrylate and/or the lipid is cationic, optionally wherein the cationic lipid is Lipofectamine. In some embodiments, the photo-cleavable polymer comprises the formulaa of (dNTP)₆-PC-(dNTP)₆-PC-(dNTP)₆-PC-(dNTP)₆, wherein PC is a photo-cleavable moiety.

### c. Oligonucleotides Comprising Photo-cleavable Moieties

In some embodiments, a method disclosed herein comprises irradiating a first polynucleotide immobilized on a substrate with a first light exposure while a second polynucleotide immobilized on the substrate is not irradiated with the first light exposure, wherein the first polynucleotide comprises a first photo-cleavable moiety that inhibits or blocks hybridization and/or ligation to the first polynucleotide, and the second polynucleotide comprises a second photo-cleavable moiety that inhibits or blocks hybridization and/or ligation to the second polynucleotide, thereby cleaving the first photo-cleavable moiety such that the inhibition or blocking of hybridization and/or ligation to the first polynucleotide is reduced or eliminated, whereas hybridization and/or ligation to the second polynucleotide remain inhibited or blocked by the second photo-cleavable moiety, wherein a first barcode is attached to the first polynucleotide via hybridization and/or ligation, thereby providing on the substrate an array comprising the first and second polynucleotides, wherein the first polynucleotide is barcoded with the first barcode and the second polynucleotide is not barcoded with the first barcode.

In some embodiments, the method further comprises irradiating the second polynucleotide with a second light exposure, thereby cleaving the second photo-cleavable moiety such that the inhibition or blocking of hybridization and/or ligation to the second polynucleotide is reduced or eliminated. In some embodiments, the second polynucleotide is irradiated with the second light exposure while the first polynucleotide is not irradiated with the second light exposure. In some embodiments, the method further comprises attaching a second barcode to the second polynucleotide via hybridization and/or ligation, thereby providing on the substrate an array comprising the first polynucleotide barcoded with the first barcode and the second polynucleotide barcoded with the second barcode. In some embodiments, hybridization and/or ligation to the first polynucleotide barcoded with the first barcode is inhibited or blocked, and/or hybridization and/or ligation to the second polynucleotide barcoded with the second barcode is inhibited or blocked. In some embodiments, the first barcode comprises a third photo-cleavable moiety that inhibits or blocks hybridization and/or ligation, thereby inhibiting or blocking hybridization and/or ligation to the first polynucleotide barcoded with the first barcode. In some embodiments, the first photo-cleavable moiety and/or the second photo-cleavable moiety comprise a photo-caged nucleobase. In some embodiments, the first photo-cleavable moiety and/or the second photo-cleavable moiety comprise a photo-cleavable hairpin. In some embodiments, the first photo-cleavable moiety and/or the second photo-cleavable moiety comprise a photo-caged 3'-hydroxyl group. In some embodiments, the substrate comprises a plurality of differentially barcoded polynucleotides immobilized thereon. In some embodiments, irradiating the sample comprises using a photomask to selectively irradiate the first polynucleotide or the second polynucleotide. In some embodiments, the attachment of the first barcode-comprises ligating one end of the first barcode to one end of the first polynucleotide. In some embodiments, the attachment of the first barcode comprises hybridizing one end of the first barcode and one end of the first polynucleotide to a first splint. In some embodiments, the method further comprises ligating the first barcode to the first polynucleotide hybridized to the first splint. In some embodiments, ligating the first barcode to the first polynucleotide, respectively, is preceded by gap filling.

In some embodiments, a method disclosed herein comprises (a1) irradiating polynucleotide P1 immobilized on a substrate with light while polynucleotide P2 immobilized on the substrate is photomasked, wherein polynucleotides P1 and P2 comprise a photo-cleavable moiety that inhibits or blocks hybridization and/or ligation to P1 and P2, respectively, thereby cleaving the photo-cleavable moiety to allow hybridization and/or ligation to P1, whereas hybridization and/or ligation to P2 remain inhibited or blocked by the photo-cleavable moiety; and (b1) attaching barcode 1A to P1 via hybridization and/or ligation to form a barcoded polynucleotide 1A-P1, wherein barcode 1A comprises the photo-cleavable moiety which inhibits or blocks hybridization and/or ligation to 1A-P1, thereby providing on the substrate an array comprising polynucleotides 1A-P1 and P2 each comprising the photo-cleavable moiety that inhibits or blocks hybridization and/or ligation. In some embodiments, the method further comprises: (c1) irradiating P2 with light while 1A-P1 is photomasked, thereby cleaving the photo-cleavable moiety to allow hybridization and/or ligation to P2, whereas hybridization and/or ligation to 1A-P1 remain inhibited or blocked by the photo-cleavable moiety; and (d1) attaching barcode 1B to P2 via hybridization and/or ligation to form a barcoded polynucleotide 1B-P2, wherein barcode 1B comprises the photo-cleavable moiety which inhibits or blocks hybridization and/or ligation to 1B-P2, thereby providing on the substrate an array comprising barcoded polynucleotides 1A-P1 and 1B-P2 each comprising the photo-cleavable moiety that inhibits or blocks hybridization and/or ligation. In some embodiments, the method further comprises: (a2) irradiating one of 1A-P1 and 1B-P2 with light while the other is photomasked, thereby cleaving the photo-cleavable moiety to allow hybridization and/or ligation to the irradiated polynucleotide, whereas hybridization and/or ligation to the photomasked polynucleotide remains inhibited or blocked by the photo-cleavable moiety; and (b2) attaching barcode 2A to the irradiated polynucleotide via hybridization and/or ligation to form a 2A-barcoded polynucleotide, wherein barcode 2A comprises the photo-cleavable moiety which inhibits or blocks hybridization and/or ligation, thereby providing on the substrate an array comprising barcoded polynucleotides each comprising the photo-cleavable moiety that inhibits or blocks hybridization and/or ligation. In some embodiments, the method further comprises (c2) irradiating the photomasked polynucleotide in step a2 with light while the 2A-barcoded polynucleotide is photomasked, thereby cleaving the photo-cleavable moiety to allow hybridization and/or ligation, whereas hybridization and/or ligation to the 2A-barcoded polynucleotide remain inhibited or blocked by the photo-cleavable moiety; and (d2) attaching barcode 2B to the irradiated polynucleotide in step c2 via hybridization and/or ligation to form a 2B-barcoded polynucleotide, wherein barcode 2B comprises the photo-cleavable moiety which inhibits or blocks hybridization and/or ligation, thereby providing on the substrate an array comprising barcoded polynucleotides each comprising the photo-cleavable moiety that inhibits or blocks hybridization and/or ligation. In some embodiments, steps a1-d1 form round 1 and steps a2-d2 form round 2, the method further comprising steps a*i*-d*i* in round *i*, wherein barcodes *i*A and *i*B are attached to provide barcoded polynucleotides on the substrate, and wherein *i* is an integer greater than 2.

In some embodiments, the photo-cleavable moiety comprises a photo-caged nucleobase. In some embodiments, the photo-caged nucleobase is a photo-caged deoxythymidine (dT). In some embodiments, the photo-cleavable moiety comprises the following structure: 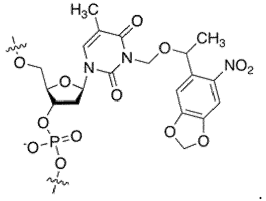 In some embodiments, the photo-cleavable moiety comprises a photo-cleavable hairpin. In some embodiments, the photo-cleavable moiety comprises the following structure: 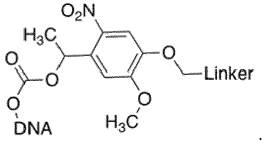 In some embodiments, the photo-cleavable moiety comprises a photo-caged 3'-hydroxyl group. In some embodiments, the photo-cleavable moiety comprises the following structure: 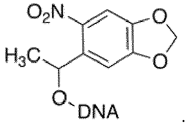 In some embodiments, polynucleotides of different nucleic acid sequences are immobilized on the substrate in a pattern comprising rows and columns prior to the irradiation.

In some embodiments, the oligonucleotide molecules are protected from hybridization by a protective group of each oligonucleotide molecule. In some embodiments, the oligonucleotide molecules are protected from ligation by a protective group of each oligonucleotide molecule. In some embodiments, the oligonucleotide molecules are protected from hybridization and ligation by a protective group of each oligonucleotide molecule. In some embodiments, the protective group is a photo-cleavable protective group. In some embodiments, the photo-cleavable protective groups in irradiated regions are cleaved and the photo-cleavable protective groups in masked or non-irradiated regions are not cleaved. Specifically, in some embodiments, the oligonucleotide molecules are protected from hybridization using photo-caged oligonucleotides. In some embodiments, the oligonucleotide molecules are protected from ligation using photo-caged oligonucleotides. In some embodiments, the oligonucleotide molecules are protected from hybridization and ligation using photo-caged oligonucleotides. In some embodiments, the photo-caged oligonucleotides comprises one or more photo-cleavable moieties, such as a photo-cleavable protective group. For example, hybridization can be blocked using a synthetic nucleotide with a photo-cleavable protecting group on a nucleobase and/or a photo-cleavable hairpin that dissociates upon cleavage. In other examples, ligation can be controlled using a photo-cleavable moiety, such as a photo-caged 3'-hydroxyl group.

In some embodiments, a photo-cleavable moiety disclosed herein is part of an oligonucleotide (e.g., a first oligonucleotide or a second oligonucleotide), such as oligonucleotide molecules in one or more other regions on the substrate, and inhibits or blocks hybridization to the oligonucleotide (e.g., the hybridization of a splint and/or a third oligonucleotide to the first and/or second oligonucleotide), but does not inhibit or block hybridization to the oligonucleotide molecules in one or more regions on the substrate. In some embodiments, the oligonucleotide is prevented from hybridization to a nucleic acid such as a splint. In some embodiments, a photo-cleavable moiety disclosed herein is part of an oligonucleotide and inhibits or blocks ligation to either end of the oligonucleotide, while hybridization of a nucleic acid to the oligonucleotide may or may not be inhibited or blocked. For example, the photo-cleavable moiety may inhibit or block the 3' or 5' end of the oligonucleotide from chemical or enzymatic ligation, *e.g.,* even when a splint may hybridize to the oligonucleotide in order to bring a ligation partner in proximity to the 3' or 5' end of the oligonucleotide. In some embodiments, the photo-cleavable moiety may cap the 3' or 5' end of the oligonucleotide.

In some embodiments, the irradiation results in cleavage of the photo-cleavable moiety such that the inhibition or blocking of hybridization and/or ligation to the oligonucleotide molecules in the one or more regions is reduced or eliminated, whereas hybridization and/or ligation to the oligonucleotide nucleotide molecules in one or more other regions remain inhibited or blocked by a second photo-cleavable moiety.

In any of the preceding embodiments, physical masks, *e.g.,* a photolithography mask which is an opaque plate or film with transparent areas that allow light to shine through in a defined pattern, may be used. In any of the preceding embodiments, different protection groups and/or photolabile groups may be used. In any of the preceding embodiments, the light can have a wavelength between about 365 nm and about 440 nm, for example, about 366 nm, 405 nm, or 436 nm.

### B. Cleaning Feature Boundaries

In some examples, wherein the molecular array involves in-situ synthesized molecules (such as oligonucleotides), there can exist overlap of features, resulting in molecular impurities (such as oligonucleotide impurities) in the feature boundaries and/or openings between features (e.g. as shown in left panel of **FIG. 3**). In some embodiments, the oligonucleotide impurities comprise non-specific oligonucleotides. The overlap in features may result from technical reasons including but not limited to alignment error and/or stray light in the manufacturing of the molecular array. In some embodiments, the molecular impurities decrease sensitivity of spatial analysis using the molecular array. In some embodiments, the molecular impurities decrease resolution of spatial analysis using the molecular array.

In some aspects, disclosed herein are methods of improving the resolution and/or sensitivity of the molecular array. In some aspects, provided are methods for providing an array comprising first and second regions on a substrate, comprising: (a) attaching first oligonucleotide molecules to first nucleic acid molecules immobilized in the first region via hybridization and/or ligation to generate first extended nucleic acid molecules; (b) attaching second oligonucleotide molecules to second nucleic acid molecules immobilized in the second region via hybridization and/or ligation to generate second extended nucleic acid molecules; and (c) irradiating the substrate through a mask comprising an opening corresponding to a boundary region between the first and second regions, thereby removing, blocking, or inactivating nucleic acid molecules in the boundary region. In some embodiments, the first and second nucleic acid molecules are the same. In some embodiments, the first and second nucleic acid molecules are different. In some embodiments, irradiating the substrate through a mask comprising an opening corresponding to a boundary region between the first and second regions removes, blocks, or inactivates any suitable nucleic acid molecule in the boundary region, such as the first and second nucleic acid molecules, the first and second extended nucleic acid molecules, first and second further extended nucleic acid molecules, a first oligonucleotide molecule attached to a second nucleic acid molecule, and/or a second oligonucleotide molecule attached to a first nucleic acid molecule.

In some embodiments, the boundary region comprises part of the first region and/or part of the second region. In some embodiments, the boundary region comprises part of the first region. In some embodiments, the boundary region comprises part of the second region.

In some embodiments, the first and second regions are of about 1 µm to about 40 µm in average diameter. In some embodiments, the first and second regions are of about 2 µm to about 20 µm in average diameter. In some embodiments, the first and second regions are of about any one of: 1 µm, 2 µm, 3 µm, 4 µm, 5 µm, 6 µm, 7 µm, 8 µm, 9 µm, 10 µm, 11 µm, 12 µm, 13 µm, 14 µm, 15 µm, 16 µm, 17 µm, 18 µm, 19 µm, 20 µm, 25 µm, 30 µm or 40 µm in average diameter. In some embodiments, the first and second regions are of about 5 µm in diameter.

In some embodiments, the opening and/or the boundary region is between about 0.5 µm and about 40 µm in length and between about 0.05 µm and about 4 µm in width. In some embodiments, the opening and/or the boundary region is between about 1 µm and about 20 µm in length and between about 0.1 µm and about 2 µm in width. In some embodiments, the opening and/or the boundary region is about any one of: 1 µm, 2 µm, 3 µm, 4 µm, 5 µm, 6 µm, 7 µm, 8 µm, 9 µm, 10 µm, 11 µm, 12 µm, 13 µm, 14 µm, 15 µm, 16 µm, 17 µm, 18 µm, 19 µm, 20 µm, 25 µm, 30 µm or 40 µm in length. In some embodiments, the opening and/or the boundary region is about any one of: 0.05 µm, 0.1 µm, 0.2 µm, 0.3 µm, 0.4 µm, 0.5 µm, 0.6 µm, 0.7 µm, 0.8 µm, 0.9 µm, 1.0 µm, 1.1 µm, 1.2 µm, 1.3 µm, 1.4 µm, 1.5 µm, 1.6 µm, 1.7 µm, 1.8 µm, 1.9 µm, 2.0 µm, 2.5 µm, 3.0 µm or 4.0 µm in width.

In some embodiments, the removed or inactivated nucleic acid molecules in the boundary region comprise (i) a first oligonucleotide molecule attached to a second nucleic acid molecule and/or (ii) a second oligonucleotide molecule attached to a first nucleic acid molecule. In some embodiments, the removed or inactivated nucleic acid molecules in the boundary region comprise a first oligonucleotide molecule attached to a second nucleic acid molecule. In some embodiments, the removed or inactivated nucleic acid molecules in the boundary region comprise a second oligonucleotide molecule attached to a first nucleic acid molecule. In some embodiments, the removed or inactivated nucleic acid molecules in the boundary region comprise a first Round 2 oligonucleotide attached to a second Round 1 oligonucleotide molecule attached to a first nucleic acid molecule. In some embodiments, the removed or inactivated nucleic acid molecules in the boundary region comprise a second Round 2 oligonucleotide attached to a first Round 1 oligonucleotide molecule attached to a first nucleic acid molecule. In some embodiments, the removed or inactivated nucleic acid molecules in the boundary region comprise a second Round 2 oligonucleotide attached to a second Round 1 oligonucleotide molecule attached to a first nucleic acid molecule.

In some embodiments, the removed or inactivated nucleic acid molecules in the boundary region comprise a first Round 2 oligonucleotide attached to a second Round 1 oligonucleotide molecule attached to a second nucleic acid molecule. In some embodiments, the removed or inactivated nucleic acid molecules in the boundary region comprise a second Round 2 oligonucleotide attached to a second Round 1 oligonucleotide molecule attached to a second nucleic acid molecule. In some embodiments, the removed or inactivated nucleic acid molecules in the boundary region comprise a first Round 2 oligonucleotide attached to a first Round 1 oligonucleotide molecule attached to a second nucleic acid molecule.

In some embodiments according to any one of the methods described herein, the substrate is irradiated through the mask with irradiation (e.g., UV light) sufficient to cleave nucleic acid molecules in the boundary region off the substrate. In some embodiments, the method further comprises removing the cleaved nucleic acid molecules and/or cleavage products thereof.

In some embodiments, a negative photoresist is applied to the substrate and upon irradiation through the photomask, the negative photoresist is strengthened (e.g., hardened, polymerized or cross-linked) to block nucleic acid molecules in the boundary region. In some embodiments, the blocking comprises preventing access to the nucleic acid molecules. In some embodiments, the blocking comprises preventing access to the terminal groups of the nucleic acid molecules. In some embodiments, the blocking comprises rendering the nucleic acid molecules inactive for subsequent ligation reactions. In some embodiments, the negative photoresist in masked (not irradiated) regions can be removed to render the nucleic acid molecules therein available for hybridization and ligation.

In some embodiments, a positive photoresist is applied to the substrate and upon irradiation through the mask, the positive photoresist in the unmasked region(s) is degraded to expose nucleic acid molecules in the boundary region, while nucleic acid molecules in the masked region(s) remain covered by the positive photoresist. In some embodiments, the nucleic acid molecules in the boundary region can be removed (e.g., cleaved by UV light) and/or inactivated (e.g., by capping to block hybridization and/or ligation), whereas the nucleic acid molecules in masked (not irradiated) regions remain unaffected (e.g., by UV cleavage or capping) since they are covered by the positive photoresist in the masked regions. Subsequently the positive photoresist in the masked regions can be exposed to light to render nucleic acid molecules therein available for hybridization and/or ligation, while nucleic acid molecules in the boundary region have been removed and/or inactivated.

In some embodiments, the method further comprises removing the exposed nucleic acid molecules in the boundary region. In some embodiments, the exposed nucleic molecules are digested by a nuclease. In some embodiments, the nucleic acid molecules are digested by an endonuclease. In some embodiments, the nucleic acid molecules are digested by a 3'→5' exonuclease. In some embodiments, the nucleic acid molecules are digested by a 5'→3' exonuclease.

In some embodiments, the method further comprises inactivating the exposed nucleic acid molecules in the boundary region. In some embodiments, the exposed nucleic acid molecules are modified at the 3' or 5' end, rendering the exposed nucleic acid molecules it unavailable for ligation. In some embodiments, the exposed nucleic acid molecules comprise modified 3' ends selected from the group consisting of dideoxy C-3' (3'-ddC), 3' inverted dT, 3' C3 spacer, 3'Amino, and 3' phosphorylation. In some embodiments, the exposed nucleic acid molecules comprise modified 5' ends selected from the group consisting of dideoxy C-3' (5'-ddC), 5' inverted dT, 5' C3 spacer, 5'Amino, and 5' phosphorylation.

In some embodiments, provided are methods for providing an array comprising first and second regions on a substrate (e.g. corresponding to "Feature 1" and "Feature 2" in **FIG. 3**), comprising: (a) attaching first oligonucleotide molecules to first nucleic acid molecules immobilized in the first region via hybridization and/or ligation to generate first extended nucleic acid molecules; (b) attaching second oligonucleotide molecules to second nucleic acid molecules immobilized in the second region via hybridization and/or ligation to generate second extended nucleic acid molecules; and (c) irradiating the substrate through a mask (e.g., as shown in **FIG. 3**) comprising an opening corresponding to a boundary region between the first and second regions, thereby removing or inactivating the first and second nucleic acid molecules and/or the first and second extended nucleic acid molecules and/or the first and second further extended nucleic acid molecules in the boundary region.

In some embodiments, the attaching step of (a) and/or (b) comprise photolithography using a photoresist. In some embodiments, the attaching step of (a) and/or (b) comprise photolithography using a 3' or 5' photo-cleavable protective group. In some embodiments, the attaching step of (a) and/or (b) comprise a photo-cleavable polymer that blocks hybridization and/or ligation.

In some embodiments according to any one of the methods described herein, the step of (a) attaching the first oligonucleotide molecules to the first nucleic acid molecules immobilized in the first region comprises: (I) irradiating a substrate comprising an unmasked first region and a masked second region, whereby a photoresist in the first region is degraded to render the first nucleic acid molecules in the first region available for hybridization and/or ligation, whereas the second nucleic acid molecules in the second region are protected by a photoresist in the second region from hybridization and/or ligation; and (II) attaching the first oligonucleotide molecules optionally comprising a first barcode to the first nucleic acid molecules in the first region via hybridization and/or ligation to generate the first extended nucleic acid molecules. In some embodiments, the second nucleic acid molecules in the second region do not receive the first oligonucleotides. In some embodiments, the step of (b) attaching the second oligonucleotide molecules to the second nucleic acid molecules immobilized in the second region comprises: (III) irradiating a substrate comprising an unmasked second region and a masked first region, whereby a photoresist in the second region is degraded to render the second nucleic acid molecules in the second region available for hybridization and/or ligation, whereas the first nucleic acid molecules in the first region are protected by a photoresist in the first region from hybridization and/or ligation; and (IV) attaching the second oligonucleotide molecules optionally comprising a second barcode to the second nucleic acid molecules in the second region via hybridization and/or ligation to generate the second extended nucleic acid molecules. In some embodiments, the first nucleic acid molecules in the second region do not receive the second oligonucleotides.

In some embodiments, step of (a) attaching the first oligonucleotide molecules to first nucleic acid molecules immobilized in the first region comprises: (A) irradiating a substrate comprising an unmasked first region and a masked second region, whereby a photoresist in the first region is degraded to render the first nucleic acid molecules in the first region available for hybridization and/or ligation, whereas the second nucleic acids molecules in the second region are protected by the photoresist in the second region from hybridization and/or ligation; and (B) attaching the first oligonucleotide molecules optionally comprising a first barcode to the first nucleic acid molecules in the first region via hybridization and/or ligation to generate the first extended nucleic acid molecules. In some embodiments, the second nucleic acid molecules in the second region do not receive the first oligonucleotides. In some embodiments according to any one of the methods described above, wherein the photoresist is a first photoresist, and the first oligonucleotide is ligated to the first nucleic acid molecules in the first region to generate the first extended nucleic acid molecules, and the step of (b) attaching the second oligonucleotide molecules to the second nucleic acid molecules immobilized in the second region comprises: (C) applying a second photoresist to the substrate, optionally wherein the second photoresist is applied after the first photoresist is removed from the substrate; (D) irradiating the substrate while the first region is masked and the second region is unmasked, whereby the first or second photoresist in the second region is degraded to render the second nucleic acid molecules in the second region available for hybridization and/or ligation, whereas the first extended nucleic acid molecules in the first region are protected by the second photoresist in the first region from hybridization and/or ligation; and (E) attaching the second oligonucleotide molecules optionally comprising a second barcode to the second nucleic acid molecules in the second region via hybridization and/or ligation to generate the second extended nucleic acid molecules. In some embodiments, the first nucleic acid molecules in the second region do not receive the second oligonucleotides.

In some embodiments, the step of (a) attaching the first oligonucleotide molecules to first nucleic acid molecules immobilized in the first region comprises: (A) irradiating a substrate comprising an unmasked first region and a masked second region, whereby a photoresist in the first region is degraded to render the first nucleic acid molecules in the first region available for hybridization and/or ligation, whereas the second nucleic acids molecules in the second region are protected by the photoresist in the second region from hybridization and/or ligation; and (B) contacting the first nucleic acid molecules in the first region with a first splint and a first oligonucleotide comprising a first barcode sequence, wherein the first splint hybridizes to the first oligonucleotide and the first nucleic acid molecules in the first region to generate the first extended nucleic acid molecules, wherein the first oligonucleotide is ligated to the first nucleic acid molecules in the first region, and the first oligonucleotide is not ligated to second nucleic acid molecules in the second region. In some embodiments according to any one of the methods described above, wherein the photoresist is a first photoresist, and the first oligonucleotide is ligated to the first nucleic acid molecules in the first region to generate the first extended nucleic acid molecules, and the step of (b) attaching the second oligonucleotide molecules to the second nucleic acid molecules immobilized in the second region comprises: (C) applying a second photoresist to the substrate, optionally wherein the second photoresist is applied after the first photoresist is removed from the substrate; (D) irradiating the substrate while the first region is masked and the second region is unmasked, whereby the first or second photoresist in the second region is degraded to render the second nucleic acid molecules in the second region available for hybridization and/or ligation, whereas the first extended nucleic acid molecules in the first region are protected by the second photoresist in the first region from hybridization and/or ligation; and (E) contacting the second nucleic acid molecules in the second region with a second splint and a second oligonucleotide comprising a second barcode sequence, wherein the second splint hybridizes to the second oligonucleotide molecule and the second nucleic acid molecules in the second region, wherein the second oligonucleotide molecule is ligated to the second nucleic acid molecules in the second region to generate the second extended nucleic acid molecules, and the second oligonucleotide molecule is not ligated to the first extended nucleic acid molecules in the first region.

In some embodiments, the hybridization region between the first splint and the oligonucleotide first nucleic acid molecule is at least 3, 4, 5, 6, 7, 8, 9, 10 bp or more than 10 bp, and/or wherein the hybridization region between the second splint and the oligonucleotide second nucleic acid molecule is at least 3, 4, 5, 6, 7, 8, 9, 10 bp or more than 10 bp. In some embodiments, the hybridization region between the first splint and the first oligonucleotide molecule is at least 3, 4, 5, 6, 7, 8, 9, 10 bp or more than 10 bp, and/or wherein the hybridization region between the second splint and the second oligonucleotide molecule is at least 3, 4, 5, 6, 7, 8, 9, 10 bp or more than 10 bp. In some embodiments, hybridization to the first splint brings the terminal nucleotides of the first oligonucleotide molecules and the first nucleic acid molecules immediately next to each other, and the ligation does not require gap-filling, thereby generating the first extended nucleic acid molecules; and/or hybridization to the second splint brings the terminal nucleotides of the second oligonucleotide molecules and the second nucleic acid molecules immediately next to each other, and the ligation does not require gap-filling, thereby generating the second extended nucleic acid molecules. In some embodiments, hybridization to the first splint configures the terminal nucleotides of the first oligonucleotide molecules and the first nucleic acid molecules to be ligated without gap-filling prior to ligation, thereby generating the first extended nucleic acid molecules; and/or hybridization to the second splint configures the terminal nucleotides of the second oligonucleotide molecules and the second nucleic acid molecules to be ligated without gap-filling prior to ligation, thereby generating the second extended nucleic acid molecules. In some embodiments, hybridization to the first splint brings the terminal nucleotides of the first oligonucleotide molecules and the first nucleic acid molecules next to each other and separated by one or more nucleotides, and the ligation is preceded by gap-filling, thereby generating the first extended nucleic acid molecules; and/or hybridization to the second splint brings the terminal nucleotides of the second oligonucleotide molecules and the second nucleic acid molecules next to each other and separated by one or more nucleotides, and the ligation is preceded by gap-filling, thereby generating the second extended nucleic acid molecules. In some embodiments, following hybridization to the first splint, the terminal nucleotides of the first oligonucleotide molecules and the first nucleic acid molecules are hybridized to nucleotides on the first splint that are separated by one or more nucleotides, and the ligation is preceded by gap-filling, thereby generating the first extended nucleic acid molecules; and/or following hybridization to the second splint, the terminal nucleotides of the second oligonucleotide molecules and the second nucleic acid molecules are hybridized to nucleotides on the second splint that are separated by one or more nucleotides, and the ligation is preceded by gap-filling, thereby generating the second extended nucleic acid molecules.

In some embodiments according to any one of the methods described herein, steps (A)-(B) are part of a first cycle, steps (D)-(E) are part of a second cycle, and steps (A)-(E) are part of a first round, and wherein the method comprises one or more additional rounds, such as about any one of 1, 2, 3, 4, 5, 6, 7, 8, 9, 10, 12, 15, 20, 25, 30 or more additional rounds. In some embodiments, the method comprises the first round and one or more additional rounds, further wherein the method comprises the step (c) of irradiating the boundary region between the first and second regions at the end of the first round and/or one or more of the additional rounds. In some embodiments, the method comprises the first round and one or more additional rounds, and the method comprises the step (c) of irradiating the boundary region between the first and second regions at the end of the final round.

In some embodiments according to any one of the methods described above, wherein steps (A)-(E) are part of a first round, wherein the first and second oligonucleotides are Round 1 oligonucleotides, wherein the first and second barcode sequences are Round 1 barcode sequences, the method further comprises: (A') irradiating the substrate while the first region is unmasked and the second region is masked, whereby a photoresist in the first region is degraded to render the first extended nucleic acid molecules in the first region available for hybridization and/or ligation, whereas the second extended nucleic acid molecules in the second region are protected by the photoresist in the second region from hybridization and/or ligation; and (B') attaching a first Round 2 oligonucleotide comprising a first Round 2 barcode sequence to the first extended nucleic acid molecules in the first region via hybridization and/or ligation to generate first further extended nucleic acid molecules. In some embodiments, the second extended nucleic acid molecules in the second region do not receive the first Round 2 barcode sequence. In some embodiments, the photoresist is a first photoresist, the first Round 2 oligonucleotide is ligated to the first extended nucleic acid molecules in the first region to generate first further nucleic acid oligonucleotide molecules, and the method further comprises: (C') applying a second photoresist to the substrate, optionally wherein the second photoresist is applied after the first photoresist is removed from the substrate; (D') irradiating the substrate while the first region is masked and the second region is unmasked, whereby the first or second photoresist in the second region is degraded to render the second extended nucleic acid molecules in the second region available for hybridization and/or ligation, whereas the first further extended nucleic acid molecules in the first region are protected by the second photoresist in the first region from hybridization and/or ligation; and (E') attaching a second Round 2 oligonucleotide comprising a second Round 2 barcode sequence to the second extended nucleic acid molecules in the second region via hybridization and/or ligation to generate second further extended nucleic acid molecules. In some embodiments, the first further extended nucleic acid molecules in the first region do not receive the second Round 2 barcode sequence. In some embodiments, the Round 1 barcode sequences are different from each other. In some embodiments, the Round 1 barcode sequences are different from the Round 2 barcode sequences.

In some embodiments according to any one of the methods described herein, the step of (a) attaching the first oligonucleotide molecules to the first nucleic acid molecules immobilized in the first region comprises: (I) irradiating with light a substrate comprising an unmasked first region and a masked second region, whereby each of the first and second nucleic acid molecules comprise a photo-cleavable moiety that inhibits or blocks hybridization and/or ligation to the first and the second oligonucleotide molecules, respectively, thereby cleaving the photo-cleavable moiety to allow hybridization and/or ligation to the first nucleic acid molecules in the first region, whereas hybridization and/or ligation to second nucleic acid molecules remain inhibited or blocked by the photo-cleavable moiety; and (II) attaching the first oligonucleotide molecules optionally comprising a first barcode via hybridization and/or ligation to form the first extended nucleic acid molecules, wherein the first oligonucleotide molecules comprise a photo-cleavable moiety which inhibits or blocks hybridization and/or ligation to the first extended nucleic acid molecules, thereby generating the first extended nucleic acid molecules in the first region. In some embodiments, the step of (b) attaching the second oligonucleotide molecules to second nucleic acid molecules immobilized in the second region comprises: (III) irradiating with light a substrate comprising an unmasked second region and a masked first region, whereby each of the first and second nucleic acid molecules comprise a photo-cleavable moiety that inhibits or blocks hybridization and/or ligation to the first and the second oligonucleotides, respectively, thereby cleaving the photo-cleavable moiety to allow hybridization and/or ligation to the second nucleic acid molecules in the second region, whereas hybridization and/or ligation to first nucleic acid molecules remain inhibited or blocked by the photo-cleavable moiety; and (IV) attaching the second oligonucleotide molecules optionally comprising a second barcode to the second nucleic acid molecules via hybridization and/or ligation to form the second extended nucleic acid molecules, wherein the second oligonucleotide molecules comprise a photo-cleavable moiety which inhibits or blocks hybridization and/or ligation to the second extended nucleic acid molecules, thereby generating the second extended nucleic acid molecules in the second region.

In some embodiments, the step of (a) attaching the first oligonucleotide molecules to first nucleic acid molecules immobilized in the first region comprises: (A1) irradiating with light a substrate comprising an unmasked first region and a masked second region, whereby each of the first and second nucleic acid molecules comprise a photo-cleavable moiety that inhibits or blocks hybridization and/or ligation to the first and the second oligonucleotide molecules, respectively, thereby cleaving the photo-cleavable moiety to allow hybridization and/or ligation to the first nucleic acid molecules in the first region, whereas hybridization and/or ligation to second nucleic acid molecules remain inhibited or blocked by the photo-cleavable moiety; and (B 1) attaching the first oligonucleotide molecules optionally comprising a first barcode to the first nucleic acid molecules via hybridization and/or ligation to form the first extended nucleic acid molecules, wherein the first oligonucleotide molecules comprise a photo-cleavable moiety which inhibits or blocks hybridization and/or ligation to the first extended nucleic acid molecules, thereby providing on the substrate an array comprising the first extended nucleic acid molecules in the first region and the second nucleic acid molecules in the second region each comprising a photo-cleavable moiety that inhibits or blocks hybridization and/or ligation. In some embodiments, the method further comprises the step of (b) attaching the second oligonucleotide molecules to the second nucleic acid molecules immobilized in the second region comprising: (C1) irradiating with light the second nucleic acid molecules in the second region, while the first extended nucleic acid molecules in the first region are masked, thereby cleaving the photo-cleavable moiety to allow hybridization and/or ligation to the second nucleic acid molecules, whereas hybridization and/or ligation to the first extended nucleic acid molecules remain inhibited or blocked by the photo-cleavable moiety; and (D1) attaching the second oligonucleotide molecules optionally comprising a second barcode to the second nucleic acid molecules via hybridization and/or ligation to form the second extended nucleic acid molecules, wherein the second oligonucleotide molecules comprise a photo-cleavable moiety which inhibits or blocks hybridization and/or ligation to the second extended nucleic acid molecules, thereby providing on the substrate an array comprising the first extended nucleic acid molecules in the first region and the second extended nucleic acid molecules in the second region each comprising a photo-cleavable moiety that inhibits or blocks hybridization and/or ligation.

In some embodiments according to any one of the methods described herein, steps (A1)-(B1) are part of a first cycle, steps (C1)-(D1) are part of a second cycle, and steps (A1)-(D1) are part of a first round, and wherein the method comprises one or more additional rounds, such as about any one of 1, 2, 3, 4, 5, 6, 7, 8, 9, 10, 12, 15, 20, 25, 30 or more additional rounds. In some embodiments, the method comprises the first round and one or more additional rounds, and further comprises the step (c) of irradiating the boundary region between the first and second regions at the end of the first round and/or one or more of the additional rounds. In some embodiments, the method comprises the first round and one or more additional rounds, and further comprises the step (c) of irradiating the boundary region between the first and second regions at the end of the final round.

In some embodiments according to any one of the methods described herein, steps (A1)-(D1) are part of a first round, the first and second oligonucleotides are Round 1 oligonucleotides, the first and second barcode sequences are Round 1 barcode sequences, and the method further comprises: (A1') irradiating with light a substrate comprising an unmasked first region and a masked second region, whereby each of the first and second extended nucleic acid molecules comprise a photo-cleavable moiety that inhibits or blocks hybridization and/or ligation to the first and the second Round 2 oligonucleotide molecules, respectively, thereby cleaving the photo-cleavable moiety to allow hybridization and/or ligation to the first extended nucleic acid molecules in the first region, whereas hybridization and/or ligation to second nucleic acid molecules remain inhibited or blocked by the photo-cleavable moiety; and (B 1') attaching the first Round 2 oligonucleotide molecules optionally comprising a first Round 2 barcode to the first extended nucleic acid molecules via hybridization and/or ligation to form first further extended nucleic acid molecules, wherein the first Round 2 oligonucleotide molecules comprise a photo-cleavable moiety which inhibits or blocks hybridization and/or ligation to the first further extended nucleic acid molecules, thereby providing on the substrate an array comprising the first further extended nucleic acid molecules in the first region and the second extended nucleic acid molecules in the second region each comprising a photo-cleavable moiety that inhibits or blocks hybridization and/or ligation. In some embodiments, the method further comprises: (C 1') irradiating with light the second extended nucleic acid molecules in the second region, while the first further extended nucleic acid molecules in the first region are masked, thereby cleaving the photo-cleavable moiety to allow hybridization and/or ligation to the second extended nucleic acid molecules, whereas hybridization and/or ligation to the first further extended nucleic acid molecules remain inhibited or blocked by the photo-cleavable moiety; and (D1') attaching the second Round 2 oligonucleotide molecules optionally comprising a second Round 2 barcode to the second extended nucleic acid molecules via hybridization and/or ligation to form second further extended nucleic acid molecules, wherein the second Round 2 oligonucleotide molecules comprise a photo-cleavable moiety which inhibits or blocks hybridization and/or ligation to the second further extended nucleic acid molecules, thereby providing on the substrate an array comprising the first further extended nucleic acid molecules in the first region and the second further extended nucleic acid molecules in the second region each comprising a photo-cleavable moiety that inhibits or blocks hybridization and/or ligation. In some embodiments, the Round 1 barcode sequences are different from each other. In some embodiments, the Round 1 barcode sequences are different from the Round 2 barcode sequences.

In some embodiments according to any one of the methods described herein, the step of (a) attaching the first oligonucleotide molecules to the first nucleic acid molecules immobilized in the first region comprises: (I) irradiating with light a substrate comprising an unmasked first region and a masked second region, whereby each of the first and second nucleic acid molecules are bound to a photo-cleavable polymer that inhibits or blocks hybridization and/or ligation to the first and the second oligonucleotide molecules, respectively, thereby cleaving the photo-cleavable polymer to allow hybridization and/or ligation to the first nucleic acid molecules in the first region, whereas hybridization and/or ligation to second nucleic acid molecules remain inhibited or blocked by the photo-cleavable polymer; and (II) attaching the first oligonucleotide molecules optionally comprising a first barcode to the first nucleic acid molecules via hybridization and/or ligation to form the first extended nucleic acid molecules, wherein the first oligonucleotide molecules are bound to a photo-cleavable polymer which inhibits or blocks hybridization and/or ligation to the first extended nucleic acid molecules, thereby generating the first extended nucleic acid molecules in the first region. In some embodiments, the step of (b) attaching the second oligonucleotide molecules to second nucleic acid molecules immobilized in the first region comprises: (III) irradiating with light a substrate comprising an unmasked second region and a masked first region, whereby each of the first and second nucleic acid molecules are bound to a photo-cleavable polymer that inhibits or blocks hybridization and/or ligation to the first and the second oligonucleotides, respectively, thereby cleaving the photo-cleavable polymer to allow hybridization and/or ligation to the second nucleic acid molecules in the second region, whereas hybridization and/or ligation to first nucleic acid molecules remain inhibited or blocked by the photo-cleavable polymer; and (IV) attaching the second oligonucleotide molecules optionally comprising a second barcode to the second nucleic acid molecules via hybridization and/or ligation to form the second extended nucleic acid molecules, wherein the second oligonucleotide molecules are bound to a photo-cleavable polymer which inhibits or blocks hybridization and/or ligation to the second extended nucleic acid molecules, thereby generating the second extended nucleic acid molecules in the second region.

In some embodiments according to any one of the methods described herein, the step of (a) attaching the first oligonucleotide molecules to the first nucleic acid molecules immobilized in the first region comprises: (A2) irradiating with light a substrate comprising an unmasked first region and a masked second region, whereby each of the first and second nucleic acid molecules are bound to a photo-cleavable polymer that inhibits or blocks hybridization and/or ligation to the first and the second oligonucleotide molecules, respectively, thereby cleaving the photo-cleavable polymer to allow hybridization and/or ligation to the first nucleic acid molecules in the first region, whereas hybridization and/or ligation to the second nucleic acid molecules remain inhibited or blocked by the photo-cleavable polymer; and (B2) attaching the first oligonucleotide molecules optionally comprising a first barcode to the first nucleic acid molecules via hybridization and/or ligation to form the first extended nucleic acid molecules, wherein the first oligonucleotide molecules are bound to a photo-cleavable polymer which inhibits or blocks hybridization and/or ligation to the first extended nucleic acid molecules, thereby providing on the substrate an array comprising the first extended nucleic acid molecules in the first region and the second nucleic acid molecules in the second region each bound to a photo-cleavable polymer that inhibits or blocks hybridization and/or ligation. In some embodiments, the method further comprises the step of (b) attaching the second oligonucleotide molecules to second nucleic acid molecules immobilized in the first region comprising: (C2) irradiating with light the second nucleic acid molecules in the second region, while the first extended nucleic acid molecules in the first region are masked, thereby cleaving the photo-cleavable polymer to allow hybridization and/or ligation to the second nucleic acid molecules, whereas hybridization and/or ligation to the first extended nucleic acid molecules remain inhibited or blocked by the photo-cleavable polymer; and (D2) attaching the second oligonucleotide molecules optionally comprising a second barcode to the second nucleic acid molecules via hybridization and/or ligation to form the second extended nucleic acid molecules, wherein the second oligonucleotide molecules are bound to a photo-cleavable moiety polymer which inhibits or blocks hybridization and/or ligation to the second extended nucleic acid molecules, thereby providing on the substrate an array comprising the first extended nucleic acid molecules in the first region and the second extended nucleic acid molecules in the second region each bound to a photo-cleavable polymer that inhibits or blocks hybridization and/or ligation.

In some embodiments according to any one of the methods described herein, steps (A2)-(B2) are part of a first cycle, steps (C2)-(D2) are part of a second cycle, and steps (A2)-(D2) are part of a first round, and wherein the method comprises one or more additional rounds, such as about any one of 1, 2, 3, 4, 5, 6, 7, 8, 9, 10, 12, 15, 20, 25, 30 or more additional rounds. In some embodiments, the method comprises the first round and one or more additional rounds, and further comprises the step (c) of irradiating the boundary region between the first and second regions at the end of the first round and/or one or more of the additional rounds. In some embodiments, the method comprises the first round and one or more additional rounds, and further comprises the step (c) of irradiating the boundary region between the first and second regions at the end of the final round.

In some embodiments according to any one of the methods described herein, wherein steps (A2)-(D2) are part of a first round, the first and second oligonucleotides are Round 1 oligonucleotides, the first and second barcode sequences are Round 1 barcode sequences, and the method further comprises: (A2') irradiating with light a substrate comprising an unmasked first region and a masked second region, whereby each of the first and second extended nucleic acid molecules is bound to a photo-cleavable polymer that inhibits or blocks hybridization and/or ligation to the first and the second Round 2 oligonucleotide molecules, respectively, thereby cleaving the photo-cleavable polymer to allow hybridization and/or ligation to the first extended nucleic acid molecules in the first region, whereas hybridization and/or ligation to second nucleic acid molecules remain inhibited or blocked by the photo-cleavable polymer; and (B2') attaching the first Round 2 oligonucleotide molecules optionally comprising a first Round 2 barcode to the first extended nucleic acid molecules via hybridization and/or ligation to form first further extended nucleic acid molecules, wherein the first Round 2 oligonucleotide molecules are bound to a photo-cleavable polymer which inhibits or blocks hybridization and/or ligation to the first further extended nucleic acid molecules, thereby providing on the substrate an array comprising the first further extended nucleic acid molecules in the first region and the second extended nucleic acid molecules in the second region each bound to a photo-cleavable polymer that inhibits or blocks hybridization and/or ligation. In some embodiments, the method further comprises: (C2') irradiating with light the second extended nucleic acid molecules in the second region, while the first further extended nucleic acid molecules in the first region are masked, thereby cleaving the photo-cleavable polymer to allow hybridization and/or ligation to the second extended nucleic acid molecules, whereas hybridization and/or ligation to the first further extended nucleic acid molecules remain inhibited or blocked by the photo-cleavable polymer; and (D2') attaching the second Round 2 oligonucleotide molecules optionally comprising a second Round 2 barcode to the second extended nucleic acid molecules via hybridization and/or ligation to form second further extended nucleic acid molecules, wherein the second oligonucleotide molecules are bound to a photo-cleavable polymer which inhibits or blocks hybridization and/or ligation to the second further extended nucleic acid molecules, thereby providing on the substrate an array comprising the first further extended nucleic acid molecules in the first region and the second further extended nucleic acid molecules in the second region each bound to a photo-cleavable polymer that inhibits or blocks hybridization and/or ligation. In some embodiments, the Round 1 barcode sequences are different from each other. In some embodiments, the Round 1 barcode sequences are different from the Round 2 barcode sequences.

In any of the preceding embodiments, the photo-cleavable polymer can be a first photo-cleavable polymer or a second photo-cleavable polymer. In any of the preceding embodiments, the first photo-cleavable polymer and the second photo-cleavable polymer can be the same or different. In any of the preceding embodiments, the first photo-cleavable polymer and/or the second photo-cleavable polymer can bind to the first and second polynucleotides, respectively, in a non-sequence-specific manner. In any of the preceding embodiments, the first photo-cleavable polymer and/or the second photo-cleavable polymer can inhibit hybridization and/or ligation to the first and second polynucleotides, respectively, in a non-sequence-specific manner. In any of the preceding embodiments, the first photo-cleavable polymer and/or the second photo-cleavable polymer can be UV degradable. In any of the preceding embodiments, the first photo-cleavable polymer and/or the second photo-cleavable polymer can be synthetic, semi-synthetic, or naturally derived.

In any of the preceding embodiments, the first photo-cleavable polymer and/or the second photo-cleavable polymer can comprises a material selected from the group consisting of a PEG (polyethylene glycol), a PDMS (polydimethylsiloxane), a polyethylenimine (PEI), a polyacrylate, a lipid, a nanoparticle, a DNA, an RNA, a synthetic oligodeoxynucleotide (ODN), a xeno nucleic acid (XNA), a peptide nucleic acid (PNA), a locked nucleic acid (LNA), a 1,5-anhydrohexitol nucleic acid (HNA), a cyclohexene nucleic acid (CeNA), a threose nucleic acid (TNA), a glycol nucleic acid (GNA), a fluoro arabino nucleic acid (FANA), and a polypeptide. In some embodiments, the polyacrylate and/or the lipid is cationic, optionally wherein the cationic lipid is Lipofectamine.

In any of the embodiments herein, pre-patterning the substrate may be used prior to the light-controlled surface patterning in situ. For instance, when an initial layer of oligos on a surface is pre-patterned, the number of cycles and/or rounds of photo cleavage, hybridization, and ligation may be reduced. In some embodiments, positive photoresist exposure and developing are used to create a patterned surface to allow immobilization of oligos only at specified surface locations, for examples, in rows and/or columns. In some embodiments, the pattern comprises regular and/or irregular shapes (e.g., polygons). In some embodiments, the patterned surface may comprise wells, and each well receives a unique oligo, e.g., one having a sequencing adapter (e.g., partial or complete Read1)-Unique Molecular Identifier (UMI)-barcode1-bridge sequence (splint).

### IV. COMPOSITIONS, HITS, AND ARTICLES OF MANUFACTURE

Also provided are compositions produced according to the methods described herein. These compositions include nucleic acid molecules and complexes, such as hybridization complexes, and kits and articles of manufacture (such as arrays) comprising such molecules and complexes. In other aspect, provided herein is an array of oligonucleotides produced by the method of any of the embodiments herein.

In some embodiments, the arrays are arrays of nucleic acids, including oligonucleotides, polynucleotides, cDNAs, mRNAs, synthetic mimetics thereof, and the like. Where the arrays are arrays of nucleic acids, the nucleic acids may be covalently attached to the arrays at any point along the nucleic acid chain, but are generally attached at one of their termini, e.g. the 3' or 5' terminus.

Arrays can be used to measure large numbers of analytes simultaneously. In some embodiments, oligonucleotides are used, at least in part, to create an array. For example, one or more copies of a single species of oligonucleotide (e.g., capture probe) can correspond to or be directly or indirectly attached to a given feature in the array. In some embodiments, a given feature in the array includes two or more species of oligonucleotides (e.g., capture probes). In some embodiments, the two or more species of oligonucleotides (e.g., capture probes) attached directly or indirectly to a given feature on the array include a common (e.g., identical) spatial barcode.

In some embodiments, an array can include a capture probe attached directly or indirectly to the substrate. The capture probe can include a capture domain (e.g., a nucleotide sequence) that can specifically bind (e.g., hybridize) to a target analyte (e.g., mRNA, DNA, or protein) within a sample. In some embodiments, the binding of the capture probe to the target (e.g., hybridization) can be detected and quantified by detection of a visual signal, e.g., a fluorophore, a heavy metal (e.g., silver ion), or chemiluminescent label, which has been incorporated into the target. In some embodiments, the intensity of the visual signal correlates with the relative abundance of each analyte in the biological sample. Since an array can contain thousands or millions of capture probes (or more), an array can interrogate many analytes in parallel. In some embodiments, the binding (e.g., hybridization) of the capture probe to the target can be detected and quantified by creation of a molecule (e.g., cDNA from captured mRNA generated using reverse transcription) that is removed from the array, and sequenced.

Kits for use in analyte detection assays are provided. In some embodiments, the kit at least includes an array disclosed herein. The kits may further include one or more additional components necessary for carrying out an analyte detection assay, such as sample preparation reagents, buffers, labels, and the like. As such, the kits may include one or more containers such as vials or bottles, with each container containing a separate component for the assay, and reagents for carrying out an array assay such as a nucleic acid hybridization assay or the like. The kits may also include a denaturation reagent for denaturing the analyte, buffers such as hybridization buffers, wash mediums, enzyme substrates, reagents for generating a labeled target sample such as a labeled target nucleic acid sample, negative and positive controls and written instructions for using the subject array assay devices for carrying out an array based assay. The instructions may be printed on a substrate, such as paper or plastic, etc. As such, the instructions may be present in the kits as a package insert, in the labeling of the container of the kit or components thereof (*e.g.*, associated with the packaging or sub-packaging) *etc.*

In particular embodiments, provided herein are kits and compositions for spatial array-based analysis of biological samples. Array-based spatial analysis methods involve the transfer of one or more analytes from a biological sample to an array of features on a substrate, where each feature is associated with a unique spatial location on the array. Subsequent analysis of the transferred analytes includes determining the identity of the analytes and the spatial location of each analyte within the biological sample. The spatial location of each analyte within the biological sample is determined based on the feature to which each analyte is bound on the array, and the feature's relative spatial location within the array. In some embodiments, the array of features on a substrate comprise a spatial barcode that corresponds to the feature's relative spatial location within the array. Each spatial barcode of a feature may further comprise a fluorophore, to create a fluorescent hybridization array. A feature may comprise UMIs that are generally unique per nucleic acid molecule in the feature - this is so the number of unique molecules can be estimated, as opposed to an artifact in experiments or PCR amplification bias that drives amplification of smaller, specific nucleic acid sequences.

In particular embodiments, the kits and compositions for spatial array-based analysis provide for the detection of differences in an analyte level (*e.g.,* gene and/or protein expression) within different cells in a tissue of a mammal or within a single cell from a mammal. For example, the kits and compositions can be used to detect the differences in analyte levels (*e.g.,* gene and/or protein expression) within different cells in histological slide samples (e.g., intact tissue section), the data from which can be reassembled to generate a three-dimensional map of analyte levels (*e.g.,* gene and/or protein expression) of a tissue sample obtained from a mammal, *e.g.,* with a degree of spatial resolution (*e.g.*, single-cell scale resolution).

Also provided herein are arrays comprising any one or more of the molecules, complexes, and/or compositions disclosed herein. In some embodiments, provided herein is an array produced by any of the methods described herein, a composition comprising the array, or a kit comprising the array. Typically, an array includes at least two distinct nucleic acids that differ by monomeric sequence immobilized on, *e.g.,* covalently to, different and known locations on the substrate surface. In certain embodiments, each distinct nucleic acid sequence of the array is typically present as a composition of multiple copies of the polymer on the substrate surface, *e.g.* as a spot on the surface of the substrate. The number of distinct nucleic acid sequences, and hence spots or similar structures, present on the array may vary, but is generally at least, usually at least 5 and more usually at least 10, where the number of different spots on the array may be as a high as 50, 100, 500, 1000, 10,000 1,000,000, 10,000,000 or higher, depending on the intended use of the array. The spots of distinct polymers present on the array surface are generally present as a pattern, where the pattern may be in the form of organized rows and columns of spots, *e.g.* a grid of spots, across the substrate surface, a series of curvilinear rows across the substrate surface, *e.g.* a series of concentric circles or semi-circles of spots, and the like. The density of spots present on the array surface may vary, but is generally at least about 10 and usually at least about 100 spots/cm ², where the density may be as high as 10⁶ or higher, or about 10⁵ spots/cm². In other embodiments, the polymeric sequences are not arranged in the form of distinct spots, but may be positioned on the surface such that there is substantially no space separating one polymer sequence/feature from another. The density of nucleic acids within an individual feature on the array may be as high as 1,000, 10,000, 25,000, 50,000, 100,000, 500,000, 1,000,000, or higher per square micron depending on the intended use of the array.

In some embodiments, the arrays are arrays of nucleic acids, including oligonucleotides, polynucleotides, cDNAs, mRNAs, synthetic mimetics thereof, and the like. Where the arrays are arrays of nucleic acids, the nucleic acids may be covalently attached to the arrays at any point along the nucleic acid chain, but are generally attached at one of their termini, e.g. the 3' or 5' terminus.

Arrays can be used to measure large numbers of analytes simultaneously. In some embodiments, oligonucleotides are used, at least in part, to create an array. For example, one or more copies of a single species of oligonucleotide (e.g., capture probe) can correspond to or be directly or indirectly attached to a given feature in the array. In some embodiments, a given feature in the array includes two or more species of oligonucleotides (e.g., capture probes). In some embodiments, the two or more species of oligonucleotides (e.g., capture probes) are attached directly or indirectly to a given feature on the array include a common (e.g., identical) spatial barcode.

In some embodiments, an array can include a capture probe attached directly or indirectly to the substrate. The capture probe can include a capture domain (e.g., a nucleotide or amino acid sequence) that can specifically bind (e.g., hybridize) to a target analyte (e.g., mRNA, DNA, or protein) within a sample. In some embodiments, the binding of the capture probe to the target (e.g., hybridization) can be detected and quantified by detection of a visual signal, e.g., a fluorophore, a heavy metal (e.g., silver ion), or chemiluminescent label, which has been incorporated into the target. In some embodiments, the intensity of the visual signal correlates with the relative abundance of each analyte in the biological sample. Since an array can contain thousands or millions of capture probes (or more), an array can interrogate many analytes in parallel. In some embodiments, the binding (e.g., hybridization) of the capture probe to the target can be detected and quantified by creation of a molecule (e.g., cDNA from captured mRNA generated using reverse transcription) that is removed from the array, and sequenced.

Kits for use in analyte detection assays are provided. In some embodiments, the kit at least includes an array disclosed herein. The kits may further include one or more additional components necessary for carrying out an analyte detection assay, such as sample preparation reagents, buffers, labels, and the like. As such, the kits may include one or more containers such as tubes, vials or bottles, with each container containing a separate component for the assay, and reagents for carrying out an array assay such as a nucleic acid hybridization assay or the like. The kits may also include a denaturation reagent for denaturing the analyte, buffers such as hybridization buffers, wash mediums, enzyme substrates, reagents for generating a labeled target sample such as a labeled target nucleic acid sample, negative and positive controls and written instructions for using the subject array assay devices for carrying out an array based assay. The instructions may be printed on a substrate, such as paper or plastic, etc. As such, the instructions may be present in the kits as a package insert, in the labeling of the container of the kit or components thereof (*e.g.,* associated with the packaging or sub-packaging) *etc.*

The subject arrays find use in a variety of different applications, where such applications are generally analyte detection applications in which the presence of a particular analyte in a given sample is detected at least qualitatively, if not quantitatively. Protocols for carrying out such assays are described extensively elsewhere and need not be described in great detail here. Generally, the sample suspected of comprising the analyte of interest is contacted with an array produced according to the subject methods under conditions sufficient for the analyte to bind to its respective binding pair member that is present on the array. Thus, if the analyte of interest is present in the sample, it binds to the array at the site of its complementary binding member and a complex is formed on the array surface. The presence of this binding complex on the array surface is then detected, e.g. through use of a signal production system, e.g. an isotopic or fluorescent label present on the analyte, e.g., through sequencing the analyte or product thereof, etc. The presence of the analyte in the sample is then deduced from the detection of binding complexes on the substrate surface, or sequence detection and/or analysis (e.g., by sequencing) on molecules indicative of the formation of the binding complex. In some embodiments, RNA molecules (e.g., mRNA) from a sample are captured by oligonucleotides (e.g., probes comprising a barcode and a poly(dT) sequence) on an array prepared by a method disclosed herein, cDNA molecules are generated via reverse transcription of the captured RNA molecules, and the cDNA molecules (e.g., a first strand cDNA) or portions or products (e.g., a second strand cDNA synthesized using a template switching oligonucleotide) thereof can be separated from the array and sequenced. Sequencing data obtained from molecules prepared on the array can be used to deduce the presence/absence or an amount of the RNA molecules in the sample.

Specific analyte detection applications of interest include hybridization assays in which the nucleic acid arrays of the present disclosure are employed. In these assays, a sample of target nucleic acids or a sample comprising intact cells or a tissue section is first prepared, where preparation may include labeling of the target nucleic acids with a label, e.g. a member of signal producing system. Following sample preparation, the sample is contacted with the array under hybridization conditions, whereby complexes are formed between target nucleic acids that are complementary to probe sequences attached to the array surface. The formation and/or presence of hybridized complexes is then detected, e.g., by analyzing molecules that are generated following the formation of the hybridized complexes, such as cDNA or a second strand generated from an RNA captured on the array. Specific hybridization assays of interest which may be practiced using the subject arrays include: gene discovery assays, differential gene expression analysis assays; nucleic acid sequencing assays, single nucleotide polymorphism assays, copy number variation assays, and the like.

### SPATIAL ANALYSIS

In some aspects, provided herein is a method for construction of a hybridization complex or an array comprising nucleic acid molecules and complexes. Oligonucleotide probe for capturing analytes or proxies thereof which may be generated using a method disclosed herein, for example, using two, three, four, or more rounds of hybridization and ligation, e.g. as shown in **FIGS. 2A-2B****.**

In some embodiments, the oligonucleotide probe for capturing analytes or proxies thereof may be generated from an existing array with a ligation strategy. In some embodiments, an array containing a plurality of oligonucleotides (*e.g., in situ* synthesized oligonucleotides) can be modified to generate a variety of oligonucleotide probes. The oligonucleotides can include various domains such as, spatial barcodes, UMIs, functional domains (*e.g.,* sequencing handle), cleavage domains, and/or ligation handles.

In some embodiments, an oligonucleotide probe can directly capture an analyte, such as mRNAs based on a poly(dT) capture domain on the oligonucleotide probe immobilized on an array. In some embodiments, the oligonucleotide probe is used for indirect analyte capture. For example, in fixed samples, such as FFPE, a probe pair can be used, and probes pairs can be target specific for each gene of the transcriptome. The probe pairs are delivered to a tissue section (which is itself on a spatial array) with a decrosslinking agent and a ligase, and the probe pairs are left to hybridize and ligate, thereby forming ligation products. The ligation products contain sequences in one or more overhangs of the probes, and the overhangs are not target specific and are complementary to capture domains on oligonucleotides immobilized on a spatial array, thus allowing the ligation product (which is a proxy for the analyte) to be captured on the array, processed, and subsequently analyzed (e.g., using a sequencing method).

A "spatial barcode" may comprise a contiguous nucleic acid segment or two or more non-contiguous nucleic acid segments that function as a label or identifier that conveys or is capable of conveying spatial information. In some embodiments, a capture probe includes a spatial barcode that possesses a spatial aspect, where the barcode is associated with a particular location within an array or a particular location on a substrate. A spatial barcode can be part of a capture probe on an array generated herein. A spatial barcode can also be a tag attached to an analyte (e.g., a nucleic acid molecule) or a combination of a tag in addition to an endogenous characteristic of the analyte (e.g., size of the analyte or end sequence(s)). A spatial barcode can be unique. In some embodiments where the spatial barcode is unique, the spatial barcode functions both as a spatial barcode and as a unique molecular identifier (UMI), associated with one particular capture probe. Spatial barcodes can have a variety of different formats. For example, spatial barcodes can include polynucleotide spatial barcodes; random nucleic acid and/or amino acid sequences; and synthetic nucleic acid and/or amino acid sequences. In some embodiments, a spatial barcode is attached to an analyte in a reversible or irreversible manner. In some embodiments, a spatial barcode is added to, for example, a fragment of a DNA or RNA sample before sequencing of the sample. In some embodiments, a spatial barcode allows for identification and/or quantification of individual sequencing-reads. In some embodiments, a spatial barcode is a used as a fluorescent barcode for which fluorescently labeled oligonucleotide probes hybridize to the spatial barcode.

In some embodiments, a spatial array is generated after ligating capture domains (*e.g.,* poly(T) or gene specific capture domains) to the oligonucleotide molecule (*e.g.,* generating capture oligonucleotides). The spatial array can be used with any of the spatial analysis methods described herein. For example, a biological sample (e.g., a tissue section) can be provided to the generated spatial array. In some embodiments, the biological sample is permeabilized. In some embodiments, the biological sample is permeabilized under conditions sufficient to allow one or more analytes present in the biological sample to interact with the capture probes of the spatial array. After capture of analytes from the biological sample, the analytes can be analyzed (*e.g.,* reverse transcribed, amplified, and/or sequenced) by any of the variety of methods described herein.

Sequential hybridization/ligation of various domains can be used to generate an oligonucleotide probe for capturing analytes or proxies thereof, by a photo-hybridization/ligation method described herein. The photo-hybridization ligation can be performed using exemplary methods and reagents described in US 2022/0228201 A1, US 2022/0228210 A1, and US 2022/0314187 A1. In some embodiments, an oligonucleotide is immobilized on a substrate (*e.g.,* an array) and may comprise a functional sequence such as a primer sequence. In some embodiments, the primer sequence is a sequencing handle that comprises a primer binding site for subsequent processing. The primer sequence can generally be selected for compatibility with any of a variety of different sequencing systems, e.g., 454 Sequencing, Ion Torrent Proton or PGM, Illumina X10, PacBio, Nanopore, etc., and the requirements thereof. In some embodiments, functional sequences can be selected for compatibility with non-commercialized sequencing systems. Examples of such sequencing systems and techniques, for which suitable functional sequences can be used, include (but are not limited to) Roche 454 sequencing, Ion Torrent Proton or PGM sequencing, Illumina X10 sequencing, PacBio SMRT sequencing, and Oxford Nanopore sequencing. Further, in some embodiments, functional sequences can be selected for compatibility with other sequencing systems, including non-commercialized sequencing systems.

In some embodiments, in a first round hybridization/ligation, an oligonucleotide comprising a part of a barcode (*e.g.,* part A of the barcode) is attached to the oligonucleotide molecule comprising the primer (e.g., R1 primer). In some embodiments, the barcode part can be common to all of the oligonucleotide molecules in a given feature. In some embodiments, the barcode part can be common to all of the oligonucleotide molecules in multiple substrate regions (e.g., features) in the same cycle. In some embodiments, the barcode part can be different for oligonucleotide molecules in different substrate regions (e.g., features) in different cycle. In some embodiments, a splint with a sequence complementary to a portion of the primer of the immobilized oligonucleotide and an additional sequence complementary to a portion of the oligonucleotide comprising the part of the barcode (e.g., part A of the barcode) facilitates the ligation of the immobilized oligonucleotide and the oligonucleotide comprising the barcode part. In some embodiments, the splint for attaching the part of the barcode of various sequences to different substrate regions (e.g., features) is common among the cycles of the same round. In some embodiments, the splint for attaching the part of the barcode of various sequences to different substrate regions (e.g., features) can be different among the cycles of the same round. In some embodiments, the splint for attaching the part of the barcode may comprise a sequence complementary to the part or a portion thereof.

A second round hybridization/ligation can involve the addition of another oligonucleotide comprising another part of a barcode (*e.g.,* part B of the barcode) to the immobilized oligonucleotide molecule comprising the primer and part A of the barcode. As shown in the figure, in some embodiments, a splint with a sequence complementary to a portion of the immobilized oligonucleotide comprising part A of the barcode and an additional sequence complementary to a portion of the oligonucleotide comprising part B of the barcode facilitates the ligation of the oligonucleotide comprising part B and the immobilized oligonucleotide comprising part A. In some embodiments, the splint for attaching part B of various sequences to different substrate regions (e.g., features) is common among the cycles of the same round. In some embodiments, the splint for attaching part B to different substrate regions (e.g., features) can be different among the cycles of the same round. In some embodiments, the splint for attaching part B may comprise a sequence complementary to part B or a portion thereof and/or a sequence complementary to part A or a portion thereof.

A third round hybridization/ligation can involve the addition of another oligonucleotide comprising another part of a barcode (*e.g.,* part C of the barcode), added to the immobilized oligonucleotide molecule comprising the primer, part A, and part B. In some embodiments, a splint with a sequence complementary to a portion of the immobilized oligonucleotide molecule comprising part B and an additional sequence complementary to a portion of the oligonucleotide comprising part C facilitates the ligation of the immobilized oligonucleotide molecule comprising part B and the oligonucleotide comprising part C. In some embodiments, the splint for attaching part C of various sequences to different substrate regions (e.g., features) is common among the cycles of the same round. In some embodiments, the splint for attaching part C to different substrate regions (e.g., features) can be different among the cycles of the same round. In some embodiments, the splint for attaching part C may comprise a sequence complementary to part C or a portion thereof and/or a sequence complementary to part B or a portion thereof.

A fourth round hybridization/ligation may be performed, which involves the addition of another oligonucleotide comprising another part of a barcode (*e.g.,* part D of the barcode), added to the immobilized oligonucleotide molecule comprising the primer, part A, part B, and part C. In some embodiments, a splint with a sequence complementary to a portion of the immobilized oligonucleotide molecule comprising part C and an additional sequence complementary to a portion of the oligonucleotide comprising part D facilitates the ligation. In some embodiments, the splint for attaching part D of various sequences to different substrate regions (e.g., features) is common among the cycles of the same round. In some embodiments, the splint for attaching part D to different substrate regions (e.g., features) can be different among the cycles of the same round. In some embodiments, the splint for attaching part D may comprise a sequence complementary to part D or a portion thereof and/or a sequence complementary to part C or a portion thereof. In some embodiments, an oligonucleotide comprising part D further comprises a UMI and/or a capture domain.

In particular embodiments, provided herein are kits and compositions for spatial array-based analysis of biological samples. Array-based spatial analysis methods involve the transfer of one or more analytes from a biological sample to an array of features on a substrate, where each feature is associated with a unique spatial location on the array. Subsequent analysis of the transferred analytes includes determining the identity of the analytes and the spatial location of each analyte within the biological sample. The spatial location of each analyte within the biological sample is determined based on the feature to which each analyte is bound on the array, and the feature's relative spatial location within the array. In some embodiments, the array of features on a substrate comprises a spatial barcode that corresponds to the feature's relative spatial location within the array. Each spatial barcode of a feature may further comprise a fluorophore, to create a fluorescent hybridization array. A feature may comprise UMIs that are generally unique per nucleic acid molecule in the feature so the number of unique molecules can be estimated, as opposed to an artifact in experiments or PCR amplification bias that drives amplification of smaller, specific nucleic acid sequences.

In particular embodiments, the kits and compositions for spatial array-based analysis provide for the detection of differences in an analyte level (*e.g.,* gene and/or protein expression) within different cells in a tissue of a mammal or within a single cell from a mammal. For example, the kits and compositions can be used to detect the differences in analyte levels (*e.g.,* gene and/or protein expression) within different cells in histological slide samples (e.g., tissue section), the data from which can be reassembled to generate a three-dimensional map of analyte levels (*e.g.,* gene and/or protein expression) of a tissue sample obtained from a mammal, *e.g.,* with a degree of spatial resolution (*e.g.,* single-cell resolution).

In some embodiments, an array generated using a method disclosed herein can be used in array-based spatial analysis methods which involve the transfer of one or more analytes from a biological sample to an array of features on a substrate, each of which is associated with a unique spatial location on the array. Subsequent analysis of the transferred analytes includes determining the identity of the analytes and the spatial location of each analyte within the sample. The spatial location of each analyte within the sample is determined based on the feature to which each analyte is bound in the array, and the feature's relative spatial location within the array.

There are at least two general methods to associate a spatial barcode with one or more neighboring cells, such that the spatial barcode identifies the one or more cells, and/or contents of the one or more cells, as associated with a particular spatial location. One general method is to drive target analytes out of a cell and towards the spatially-barcoded array. In some embodiments, the spatially-barcoded array populated with capture probes is contacted with a sample (e.g., a tissue section or a population of single cells), and the sample is permeabilized, allowing the target analyte to migrate away from the sample and toward the array. The target analyte interacts with a capture probe on the spatially-barcoded array. Once the target analyte hybridizes/is bound to the capture probe, the sample is optionally removed from the array and the capture probes are analyzed in order to obtain spatially-resolved analyte information. Methods for performing such spatial analysis of tissue sections include but are not limited to those methods disclosed in US Patent 10,030,261, US Patent 11,332,790 and US Patent Pub No. 20220127672 and US Patent Pub No. 20220106632.

Another general method is to cleave the spatially-barcoded capture probes from an array, and drive the spatially-barcoded capture probes towards and/or into or onto the sample. In some embodiments, the spatially-barcoded array populated with capture probes is contacted with a sample. The spatially-barcoded capture probes are cleaved and then interact with cells within the provided sample (See, for example, US Patent 11,352,659). The interaction can be a covalent or non-covalent cell-surface interaction. The interaction can be an intracellular interaction facilitated by a delivery system or a cell penetration peptide. Once the spatially-barcoded capture probe is associated with a particular cell, the sample can be optionally removed for analysis. The sample can be optionally dissociated before analysis. Once the tagged cell is associated with the spatially-barcoded capture probe, the capture probes can be analyzed (e.g., by sequencing) to obtain spatially-resolved information about the tagged cell.

Sample preparation may include placing the sample on a slide, fixing the sample, and/or staining the sample for imaging. The stained sample may be imaged on the array using both brightfield (to image the sample hematoxylin and eosin stain) and/or fluorescence (to image features) modalities. In some embodiments, target analytes are then released from the sample and capture probes forming the spatially-barcoded array hybridize or bind the released target analytes. The sample is then removed from the array and the capture probes cleaved from the array. The sample and array are then optionally imaged a second time in one or both modalities (brightfield and fluorescence) while the analytes are reverse transcribed into cDNA, and an amplicon library is prepared and sequenced. In some embodiments, the two sets of images can then be spatially-overlaid in order to correlate spatially-identified sample information. When the sample and array are not imaged a second time, a spot coordinate file may be supplied. The spot coordinate file can replace the second imaging step. Further, amplicon library preparation can be performed with a unique PCR adapter and sequenced.

In some embodiments, a spatially-labelled array on a substrate is used, where capture probes labelled with spatial barcodes are clustered at areas called features. The spatially-labelled capture probes can include a cleavage domain, one or more functional sequences, a spatial barcode, a unique molecular identifier, and a capture domain. The spatially-labelled capture probes can also include a 5' end modification for reversible attachment to the substrate. The spatially-barcoded array is contacted with a sample, and the sample is permeabilized through application of permeabilization reagents. Permeabilization reagents may be administered by placing the array/sample assembly within a bulk solution. Alternatively, permeabilization reagents may be administered to the sample via a diffusion-resistant medium and/or a physical barrier such as a lid, wherein the sample is sandwiched between the diffusion-resistant medium and/or barrier and the array-containing substrate. The analytes are migrated toward the spatially-barcoded capture array using any number of techniques disclosed herein. For example, analyte migration can occur using a diffusion-resistant medium lid and passive migration. As another example, analyte migration can be active migration, using an electrophoretic transfer system, for example. Once the analytes are in close proximity to the spatially-barcoded capture probes, the capture probes can hybridize or otherwise bind a target analyte. The sample can be optionally removed from the array.

Adapters and assay primers can be used to allow the capture probe or the analyte capture agent to be attached to any suitable assay primers and used in any suitable assays. A capture probe that includes a spatial barcode can be attached to a bead that includes a poly(dT) sequence. A capture probe including a spatial barcode and a poly(T) sequence can be used to assay multiple biological analytes as generally described herein (e.g., the biological analyte includes a poly(A) sequence or is coupled to or otherwise is associated with an analyte capture agent comprising a poly(A) sequence as the analyte capture sequence).

The capture probes can be optionally cleaved from the array, and the captured analytes or proxies thereof can be spatially-tagged, e.g., by performing a reverse transcriptase first strand cDNA reaction. A first strand cDNA reaction can be optionally performed using template switching oligonucleotides. For example, a template switching oligonucleotide can hybridize to a poly(C) tail added to a 3'end of the cDNA by a reverse transcriptase enzyme. The original mRNA template and template switching oligonucleotide can then be denatured from the cDNA and the barcoded capture probe can then hybridize with the cDNA and a complement of the cDNA can be generated. The first strand cDNA can then be purified and collected for downstream amplification steps. The first strand cDNA can be amplified using PCR, wherein forward and reverse primers flank the spatial barcode and target analyte regions of interest, generating a library associated with a particular spatial barcode. In some embodiments, the cDNA comprises a sequencing by synthesis (SBS) primer sequence. The library amplicons are sequenced and analyzed to decode spatial information.

In some embodiments, the sample is removed from the spatially-barcoded array and the spatially-barcoded capture probes are removed from the array for barcoded analyte amplification and library preparation. In some embodiments, the sample is removed from the spatially-barcoded array prior to removal of the spatially-barcoded capture probes from the array. Another embodiment includes performing first strand synthesis using template switching oligonucleotides on the spatially-barcoded array without cleaving the capture probes. Once the capture probes capture the target analyte(s), first strand cDNA created by template switching and reverse transcriptase is then denatured and the second strand is then extended. The second strand cDNA is then denatured from the first strand cDNA, neutralized, and transferred to a tube. cDNA quantification and amplification can be performed using standard techniques discussed herein. The cDNA can then be subjected to library preparation and indexing, including fragmentation, end-repair, A-tailing, and indexing PCR steps, and then sequenced.

### V. APPLICATIONS OF SPATIAL ARRAYS

The arrays generated using a method disclosed herein find use in a variety of different applications, where such applications are generally analyte detection applications in which the presence of a particular analyte in a given sample is detected at least qualitatively, if not quantitatively. Protocols for carrying out such assays are described elsewhere and need not be described in great detail here. Generally, the sample suspected of comprising the analyte of interest is contacted with an array produced according to the subject methods under conditions sufficient for the analyte to bind to its respective binding pair member that is present on the array. Thus, if the analyte of interest is present in the sample, it binds to the array at the site of its complementary binding member and a complex is formed on the array surface. The presence of this binding complex on the array surface is then detected, e.g. through use of a signal production system, e.g. an isotopic or fluorescent label present on the analyte, etc., and/or through sequencing of one or more components of the binding complex or a product thereof. The presence of the analyte in the sample is then deduced from the detection of binding complexes on the substrate surface, or sequence detection and/or analysis (e.g., by sequencing) on molecules indicative of the formation of the binding complex. In some embodiments, RNA molecules (e.g., mRNA) from a sample are captured by oligonucleotides (e.g., probes comprising a barcode and a poly(dT) sequence) on an array prepared by a method disclosed herein, cDNA molecules are generated via reverse transcription of the captured RNA molecules, and the cDNA molecules (e.g., a first strand cDNA) or portions or products (e.g., a second strand cDNA synthesized using a template switching oligonucleotide) thereof can be separated from the array and sequenced. Sequencing data obtained from molecules prepared on the array can be used to deduce the presence/absence or an amount of the RNA molecules in the sample.

Specific analyte detection applications of interest include hybridization assays in which the nucleic acid arrays of the subject disclosure are employed. In these assays, a sample of target nucleic acids or a tissue section is first prepared, where preparation may include labeling of the target nucleic acids with a label, e.g. a member of signal producing system. Following sample preparation, the sample is contacted with the array under hybridization conditions, whereby complexes are formed between target nucleic acids that are complementary to probe sequences attached to the array surface. The formation and/or presence of hybridized complexes is then detected, e.g., by analyzing molecules that are generated following the formation of the hybridized complexes, such as cDNA or a second strand generated from an RNA captured on the array. Specific hybridization assays of interest which may be practiced using the subject arrays include: gene discovery assays, differential gene expression analysis assays; nucleic acid sequencing assays, and the like.

In some embodiments, an array generated using a method disclosed herein can be used in array-based spatial analysis methods which involve the transfer of one or more analytes from a biological sample to an array of features on a substrate, each of which is associated with a unique spatial location on the array. Subsequent analysis of the transferred analytes includes determining the identity of the analytes and the spatial location of each analyte within the sample. The spatial location of each analyte within the sample is determined based on the feature to which each analyte is bound in the array, and the feature's relative spatial location within the array.

### VI. TERMINOLOGY

Unless defined otherwise, all terms of art, notations and other technical and scientific terms or terminology used herein are intended to have the same meaning as is commonly understood by one of ordinary skill in the art to which the claimed subject matter pertains. In some cases, terms with commonly understood meanings are defined herein for clarity and/or for ready reference, and the inclusion of such definitions herein should not necessarily be construed to represent a substantial difference over what is generally understood in the art.

Throughout this disclosure, various aspects of the claimed subject matter are presented in a range format. It should be understood that the description in range format is merely for convenience and brevity and should not be construed as an inflexible limitation on the scope of the claimed subject matter. Accordingly, the description of a range should be considered to have specifically disclosed all the possible sub-ranges as well as individual numerical values within that range. For example, where a range of values is provided, it is understood that each intervening value, between the upper and lower limit of that range and any other stated or intervening value in that stated range is encompassed within the claimed subject matter. The upper and lower limits of these smaller ranges may independently be included in the smaller ranges, and are also encompassed within the claimed subject matter, subject to any specifically excluded limit in the stated range. Where the stated range includes one or both of the limits, ranges excluding either or both of those included limits are also included in the claimed subject matter. This applies regardless of the breadth of the range.

Use of ordinal terms such as "first", "second", "third", etc., in the claims to modify a claim element does not by itself connote any priority, precedence, or order of one claim element over another or the temporal order in which acts of a method are performed, but are used merely as labels to distinguish one claim element having a certain name from another element having a same name (but for use of the ordinal term) to distinguish the claim elements. Similarly, use of a), b), etc., or i), ii), etc. does not by itself connote any priority, precedence, or order of steps in the claims. Similarly, the use of these terms in the specification does not by itself connote any required priority, precedence, or order.

As used in this specification and the appended claims, the singular forms "a," "an" and "the" include plural referents unless the context clearly dictates otherwise. Thus, for example, reference to "a molecule" includes a plurality of such molecules, and the like.

The term "about" as used herein refers to the usual error range for the respective value readily known to the skilled person in this technical field. Reference to "about" a value or parameter herein comprises (and describes) embodiments that are directed to that value or parameter *per se.*

Throughout this disclosure, various aspects of the claimed subject matter are presented in a range format. It should be understood that the description in range format is merely for convenience and brevity and should not be construed as an inflexible limitation on the scope of the claimed subject matter. Accordingly, the description of a range should be considered to have specifically disclosed all the possible sub-ranges as well as individual numerical values within that range. For example, where a range of values is provided, it is understood that each intervening value, between the upper and lower limit of that range and any other stated or intervening value in that stated range is encompassed within the claimed subject matter. The upper and lower limits of these smaller ranges may independently be comprised in the smaller ranges, and are also encompassed within the claimed subject matter, subject to any specifically excluded limit in the stated range. Where the stated range comprises one or both of the limits, ranges excluding either or both of those comprised limits are also comprised in the claimed subject matter. This applies regardless of the breadth of the range.

A sample such as a biological sample can include any number of macromolecules, for example, cellular macromolecules and organelles (e.g., mitochondria and nuclei). The biological sample can be obtained as a tissue sample, such as a tissue section, biopsy, a core biopsy, needle aspirate, or fine needle aspirate. The sample can be a fluid sample, such as a blood sample, urine sample, or saliva sample. The sample can be a skin sample, a colon sample, a cheek swab, a histology sample, a histopathology sample, a plasma or serum sample, a tumor sample, living cells, cultured cells, a clinical sample such as, for example, whole blood or blood-derived products, blood cells, or cultured tissues or cells, including cell suspensions. In some embodiments, the biological sample may comprise cells which are deposited on a surface.

The term "barcode," comprises a label, or identifier, that conveys or is capable of conveying information (*e.g.,* information about an analyte in a sample, a bead, and/or a capture probe). A barcode can be part of an analyte, or independent of an analyte. A barcode can be attached to an analyte. A particular barcode can be unique relative to other barcodes. Barcodes can have a variety of different formats. For example, barcodes can include polynucleotide barcodes, random nucleic acid and/or amino acid sequences, and synthetic nucleic acid and/or amino acid sequences. A barcode can be attached to an analyte or to another moiety or structure in a reversible or irreversible manner. A barcode can be added to, for example, a fragment of a deoxyribonucleic acid (DNA) or ribonucleic acid (RNA) sample before or during sequencing of the sample. Barcodes can allow for identification and/or quantification of individual sequencing-reads (*e.g.,* a barcode can be or can include a unique molecular identifier or "UMI").

Barcodes can spatially-resolve molecular components found in biological samples, for example, at single-cell scale resolution (*e.g.,* a barcode can be or can include a "spatial barcode"). In some embodiments, a barcode includes both a UMI and a spatial barcode. In some embodiments, a barcode includes two or more sub-barcodes that together function as a single barcode. For example, a polynucleotide barcode can include two or more polynucleotide sequences (*e.g.,* sub-barcodes) that are separated by one or more non-barcode sequences.

As used herein, the term "substrate" generally refers to a substance, structure, surface, material, means, or composition, which comprises a nonbiological, synthetic, nonliving, planar, spherical or flat surface. The substrate may include, for example and without limitation, semiconductors, synthetic metals, synthetic semiconductors, insulators and dopants; metals, alloys, elements, compounds and minerals; synthetic, cleaved, etched, lithographed, printed, machined and microfabricated slides, wafers, devices, structures and surfaces; industrial polymers, plastics, membranes; silicon, silicates, glass, metals and ceramics; wood, paper, cardboard, cotton, wool, cloth, woven and nonwoven fibers, materials and fabrics; nanostructures and microstructures. The substrate may comprise an immobilization matrix such as but not limited to, insolubilized substance, solid phase, surface, layer, coating, woven or nonwoven fiber, matrix, crystal, membrane, insoluble polymer, plastic, glass, biological or biocompatible or bioerodible or biodegradable polymer or matrix, microparticle or nanoparticle. Other examples may include, for example and without limitation, monolayers, bilayers, commercial membranes, resins, matrices, fibers, separation media, chromatography supports, polymers, plastics, glass, mica, gold, beads, microspheres, nanospheres, silicon, gallium arsenide, organic and inorganic metals, semiconductors, insulators, microstructures and nanostructures. Microstructures and nanostructures may include, without limitation, microminiaturized, nanometer-scale and supramolecular probes, tips, bars, pegs, plugs, rods, sleeves, wires, filaments, and tubes.

As used herein, the term "nucleic acid" generally refers to a polymer comprising one or more nucleic acid subunits or nucleotides. A nucleic acid may include one or more subunits selected from adenosine (A), cytosine (C), guanine (G), thymine (T) and uracil (U), or variants thereof. A nucleotide can include A, C, G, T or U, or variants thereof. A nucleotide can include any subunit that can be incorporated into a growing nucleic acid strand. Such subunit can be an A, C, G, T, or U, or any other subunit that is specific to one or more complementary A, C, G, T or U, or complementary to a purine (e.g., A or G, or variant thereof) or a pyrimidine (e.g., C, T or U, or variant thereof). A subunit can enable individual nucleic acid bases or groups of bases (e.g., AA, TA, AT, GC, CG, CT, TC, GT, TG, AC, CA, or uracil-counterparts thereof) to be resolved. In some examples, a nucleic acid is deoxyribonucleic acid (DNA) or ribonucleic acid (RNA), or derivatives thereof. A nucleic acid may be single-stranded or double-stranded.

The term "nucleic acid sequence" or "nucleotide sequence" as used herein generally refers to nucleic acid molecules with a given sequence of nucleotides, of which it may be desired to know the presence or amount. The nucleotide sequence can comprise ribonucleic acid (RNA) or DNA, or a sequence derived from RNA or DNA. Examples of nucleotide sequences are sequences corresponding to natural or synthetic RNA or DNA including genomic DNA and messenger RNA. The length of the sequence can be any length that can be amplified into nucleic acid amplification products, or amplicons, for example, up to about 20, 50, 100, 200, 300, 400, 500, 600, 700, 800, 1000, 1200, 1500, 2000, 5000, 10000 or more than 10000 nucleotides in length, or at least about 20, 50, 100, 200, 300, 400, 500, 600, 700, 800, 1000, 1200, 1500, 2000, 5000, 10000 nucleotides in length.

The terms "oligonucleotide" and "polynucleotide" are used interchangeably to refer to a single-stranded multimer of nucleotides from about 2 to about 500 nucleotides in length. Oligonucleotides can be synthetic, made enzymatically (e.g., via polymerization), or using a "split-pool" method. Oligonucleotides can include ribonucleotide monomers (e.g., can be oligoribonucleotides) and/or deoxyribonucleotide monomers (e.g., oligodeoxyribonucleotides). In some examples, oligonucleotides can include a combination of both deoxyribonucleotide monomers and ribonucleotide monomers in the oligonucleotide (e.g., random or ordered combination of deoxyribonucleotide monomers and ribonucleotide monomers). An oligonucleotide can be 4 to 10, 10 to 20, 21 to 30, 31 to 40, 41 to 50, 51 to 60, 61 to 70, 71 to 80, 80 to 100, 100 to 150, 150 to 200, 200 to 250, 250 to 300, 300 to 350, 350 to 400, or 400-500 nucleotides in length, for example. Oligonucleotides can include one or more functional moieties that are attached (e.g., covalently or non-covalently) to the multimer structure. For example, an oligonucleotide can include one or more detectable labels (e.g., a radioisotope or fluorophore).

As used herein, the term "adjacent" or "adjacent to," includes "next to," "adjoining," and "abutting." In one example, a first location is adjacent to a second location when the first location is in direct contact and shares a common border with the second location and there is no space between the two locations. In some cases, the adjacent is not diagonally adjacent.

An "adaptor," an "adapter," and a "tag" are terms that are used interchangeably in this disclosure, and refer to species that can be coupled to a polynucleotide sequence (in a process referred to as "tagging") using any one of many different techniques including (but not limited to) ligation, hybridization, and tagmentation. Adaptors can also be nucleic acid sequences that add a function, e.g., spacer sequences, primer sequences/sites, barcode sequences, unique molecular identifier sequences.

The terms "hybridizing," "hybridize," "annealing," and "anneal" are used interchangeably in this disclosure, and refer to the pairing of substantially complementary or complementary nucleic acid sequences within two different molecules. Pairing can be achieved by any process in which a nucleic acid sequence joins with a substantially or fully complementary sequence through base pairing to form a hybridization complex. For purposes of hybridization, two nucleic acid sequences are "substantially complementary" if at least 60% (e.g., at least 70%, at least 80%, or at least 90%) of their individual bases are complementary to one another.

A "proximity ligation" is a method of ligating two (or more) nucleic acid sequences that are in proximity with each other through enzymatic means (e.g., a ligase). In some embodiments, proximity ligation can include a "gap-filling" step that involves incorporation of one or more nucleic acids by a polymerase, based on the nucleic acid sequence of a template nucleic acid molecule, spanning a distance between the two nucleic acid molecules of interest (see, e.g., U.S. Patent No. 7,264,929).

A wide variety of different methods can be used for proximity ligating nucleic acid molecules, including (but not limited to) "sticky-end" and "blunt-end" ligations. Additionally, single-stranded ligation can be used to perform proximity ligation on a single-stranded nucleic acid molecule. Sticky-end proximity ligations involve the hybridization of complementary single-stranded sequences between the two nucleic acid molecules to be joined, prior to the ligation event itself. Blunt-end proximity ligations generally do not include hybridization of complementary regions from each nucleic acid molecule because both nucleic acid molecules lack a single-stranded overhang at the site of ligation

As used herein, the term "splint" is an oligonucleotide that, when hybridized to other polynucleotides, acts as a "splint" to position the polynucleotides next to one another so that they can be ligated together. In some embodiments, the splint is DNA or RNA. The splint can include a nucleotide sequence that is partially complimentary to nucleotide sequences from two or more different oligonucleotides. In some embodiments, the splint assists in ligating a "donor" oligonucleotide and an "acceptor" oligonucleotide. In general, an RNA ligase, a DNA ligase, or another other variety of ligase is used to ligate two nucleotide sequences together.

In some embodiments, the splint is between 6 and 50 nucleotides in length, e.g., between 6 and 45, 6 and 40, 6 and 35, 6 and 30, 6 and 25, or 6 and 20 nucleotides in length. In some embodiments, the splint is between 10 and 50 nucleotides in length, e.g., between 10 and 45, 10 and 40, 10 and 35, 10 and 30, 10 and 25, or 10 and 20 nucleotides in length. In some embodiments, the splint is between 15 and 50, 15 and 45, 15 and 40, 15 and 35, 15 and 30, or 15 and 25 nucleotides in length.

A "feature" is an entity that acts as a support or repository for various molecular entities used in sample analysis. In some embodiments, some or all of the features in an array are functionalized for analyte capture. In some embodiments, functionalized features include one or more capture probe(s). Examples of features include, but are not limited to, a bead, a spot of any two- or three-dimensional geometry (e.g., an inkjet spot, a masked spot, a square on a grid), a well, and a hydrogel pad. In some embodiments, features are directly or indirectly attached or fixed to a substrate. In some embodiments, the features are not directly or indirectly attached or fixed to a substrate, but instead, for example, are disposed within an enclosed or partially enclosed three dimensional space (e.g., wells or divots).

The term "sequencing," as used herein, generally refers to methods and technologies for determining the sequence of nucleotide bases in one or more polynucleotides. The polynucleotides can be, for example, nucleic acid molecules such as deoxyribonucleic acid (DNA) or ribonucleic acid (RNA), including variants or derivatives thereof (e.g., single stranded DNA). Sequencing can be performed by various systems currently available, such as, without limitation, a sequencing system by Illumina^{®}, Pacific Biosciences (PacBio^{®}), Oxford Nanopore^{®}, or Life Technologies (Ion Torrent^{®}). Alternatively or in addition, sequencing may be performed using nucleic acid amplification, polymerase chain reaction (PCR) (e.g., digital PCR, quantitative PCR, or real time PCR), or isothermal amplification. Such systems may provide a plurality of raw genetic data corresponding to the genetic information of a subject (e.g., human), as generated by the systems from a sample provided by the subject. In some examples, such systems provide sequencing reads (also "reads" herein). A read may include a string of nucleic acid bases corresponding to a sequence of a nucleic acid molecule that has been sequenced. In some situations, systems and methods provided herein may be used with proteomic information.

The term "template" as used herein generally refers to individual polynucleotide molecules from which another nucleic acid, including a complementary nucleic acid strand, can be synthesized by a nucleic acid polymerase. In addition, the template can be one or both strands of the polynucleotides that are capable of acting as templates for template-dependent nucleic acid polymerization catalyzed by the nucleic acid polymerase. Use of this term should not be taken as limiting the scope of the present disclosure to polynucleotides which are actually used as templates in a subsequent enzyme-catalyzed polymerization reaction. The template can be an RNA or DNA. The template can be cDNA corresponding to an RNA sequence. The template can be DNA.

As used herein, "amplification" of a template nucleic acid generally refers to a process of creating (e.g., in vitro) nucleic acid strands that are identical or complementary to at least a portion of a template nucleic acid sequence, or a universal or tag sequence that serves as a surrogate for the template nucleic acid sequence, all of which are only made if the template nucleic acid is present in a sample. Typically, nucleic acid amplification uses one or more nucleic acid polymerase and/or transcriptase enzymes to produce multiple copies of a template nucleic acid or fragments thereof, or of a sequence complementary to the template nucleic acid or fragments thereof. In vitro nucleic acid amplification techniques are may include transcription-associated amplification methods, such as Transcription-Mediated Amplification (TMA) or Nucleic Acid Sequence-Based Amplification (NASBA), and other methods such as Polymerase Chain Reaction (PCR), Reverse Transcriptase-PCR (RT-PCR), Replicase Mediated Amplification, and Ligase Chain Reaction (LCR).

The terms "polynucleotide," "polynucleotide," and "nucleic acid molecule", used interchangeably herein, refer to polymeric forms of nucleotides of any length, either ribonucleotides or deoxyribonucleotides. Thus, this term comprises, but is not limited to, single-, double-, or multi-stranded DNA or RNA, genomic DNA, cDNA, DNA-RNA hybrids, or a polymer comprising purine and pyrimidine bases or other natural, chemically or biochemically modified, non-natural, or derivatized nucleotide bases. The backbone of the polynucleotide can comprise sugars and phosphate groups (as may typically be found in RNA or DNA), or modified or substituted sugar or phosphate groups.

"Hybridization" as used herein may refer to the process in which two single-stranded polynucleotides bind non-covalently to form a stable double-stranded polynucleotide. In one aspect, the resulting double-stranded polynucleotide can be a "hybrid" or "duplex." "Hybridization conditions" typically include salt concentrations of approximately less than 1 M, often less than about 500 mM and may be less than about 200 mM. A "hybridization buffer" includes a buffered salt solution such as 5% SSPE, or other such buffers known in the art. Hybridization temperatures can be as low as 5°C, but are typically greater than 22°C, and more typically greater than about 30°C, and typically in excess of 37°C. Hybridizations are often performed under stringent conditions, *e.g.,* conditions under which a sequence will hybridize to its target sequence but will not hybridize to other, non-complementary sequences. Stringent conditions are sequence-dependent and are different in different circumstances. For example, longer fragments may require higher hybridization temperatures for specific hybridization than short fragments. As other factors may affect the stringency of hybridization, including base composition and length of the complementary strands, presence of organic solvents, and the extent of base mismatching, the combination of parameters is more important than the absolute measure of any one parameter alone. Generally stringent conditions are selected to be about 5°C lower than the T*ₘ* for the specific sequence at a defined ionic strength and pH. The melting temperature T*ₘ* can be the temperature at which a population of double-stranded nucleic acid molecules becomes half dissociated into single strands. Several equations for calculating the T*ₘ* of nucleic acids are well known in the art. As indicated by standard references, a simple estimate of the T*ₘ* value may be calculated by the equation, T*ₘ* =81.5 + 0.41 (% G + C), when a nucleic acid is in aqueous solution at 1 M NaCl (see *e.g.,* Anderson and Young, Quantitative Filter Hybridization, in Nucleic Acid Hybridization (1985)). Other references (*e.g.,* Allawi and SantaLucia, Jr., Biochemistry, 36:10581-94 (1997)) include alternative methods of computation which take structural and environmental, as well as sequence characteristics into account for the calculation of T*ₘ*.

In general, the stability of a hybrid is a function of the ion concentration and temperature. Typically, a hybridization reaction is performed under conditions of lower stringency, followed by washes of varying, but higher, stringency. Exemplary stringent conditions include a salt concentration of at least 0.01 M to no more than 1 M sodium ion concentration (or other salt) at a pH of about 7.0 to about 8.3 and a temperature of at least 25°C. For example, conditions of 5 × SSPE (750 mM NaCl, 50 mM sodium phosphate, 5 mM EDTA at pH 7.4) and a temperature of approximately 30°C are suitable for allele-specific hybridizations, though a suitable temperature depends on the length and/or GC content of the region hybridized. In one aspect, "stringency of hybridization" in determining percentage mismatch can be as follows: 1) high stringency: 0.1 × SSPE, 0.1% SDS, 65°C; 2) medium stringency: 0.2 × SSPE, 0.1% SDS, 50°C (also referred to as moderate stringency); and 3) low stringency: 1.0 × SSPE, 0.1% SDS, 50°C. It is understood that equivalent stringencies may be achieved using alternative buffers, salts and temperatures. For example, moderately stringent hybridization can refer to conditions that permit a nucleic acid molecule such as a probe to bind a complementary nucleic acid molecule. The hybridized nucleic acid molecules generally have at least 60% identity, including for example at least any of 70%, 75%, 80%, 85%, 90%, or 95% identity. Moderately stringent conditions can be conditions equivalent to hybridization in 50% formamide, 5 × Denhardt's solution, 5x SSPE, 0.2% SDS at 42°C, followed by washing in 0.2 × SSPE, 0.2% SDS, at 42°C. High stringency conditions can be provided, for example, by hybridization in 50% formamide, 5 × Denhardt's solution, 5 × SSPE, 0.2% SDS at 42°C, followed by washing in 0.1 × SSPE, and 0.1% SDS at 65°C. Low stringency hybridization can refer to conditions equivalent to hybridization in 10% formamide, 5 × Denhardt's solution, 6 × SSPE, 0.2% SDS at 22°C, followed by washing in 1x SSPE, 0.2% SDS, at 37°C. Denhardt's solution contains 1% Ficoll, 1% polyvinylpyrolidone, and 1% bovine serum albumin (BSA). 20 × SSPE (sodium chloride, sodium phosphate, ethylene diamide tetraacetic acid (EDTA)) contains 3M sodium chloride, 0.2M sodium phosphate, and 0.025 M EDTA. Other suitable moderate stringency and high stringency hybridization buffers and conditions are well known to those of skill in the art and are described, for example, in Sambrook et al., Molecular Cloning: A Laboratory Manual, 2nd ed., Cold Spring Harbor Press, Plainview, N.Y. (1989); and Ausubel et al., Short Protocols in Molecular Biology, 4th ed., John Wiley & Sons (1999).

Alternatively, substantial complementarity exists when an RNA or DNA strand will hybridize under selective hybridization conditions to its complement. Typically, selective hybridization will occur when there is at least about 65% complementary over a stretch of at least 14 to 25 nucleotides, preferably at least about 75%, more preferably at least about 90% complementary. *See* M. Kanehisa, Nucleic Acids Res. 12:203 (1984).

A "primer" used herein can be an oligonucleotide, either natural or synthetic, that is capable, upon forming a duplex with a polynucleotide template, of acting as a point of initiation of nucleic acid synthesis and being extended from its 3' end along the template so that an extended duplex is formed. The sequence of nucleotides added during the extension process is determined by the sequence of the template polynucleotide. Primers usually are extended by a DNA polymerase.

"Ligation" may refer to the formation of a covalent bond or linkage between the termini of two or more nucleic acids, *e.g.,* oligonucleotides and/or polynucleotides, in a template-driven reaction. The nature of the bond or linkage may vary widely and the ligation may be carried out enzymatically or chemically. As used herein, ligations are usually carried out enzymatically to form a phosphodiester linkage between a 5' carbon terminal nucleotide of one oligonucleotide with a 3' carbon of another nucleotide.

"Sequencing," "sequence determination" and the like means determination of information relating to the nucleotide base sequence of a nucleic acid. Such information may include the identification or determination of partial as well as full sequence information of the nucleic acid. Sequence information may be determined with varying degrees of statistical reliability or confidence. In one aspect, the term includes the determination of the identity and ordering of a plurality of contiguous nucleotides in a nucleic acid. "High throughput digital sequencing" or "next generation sequencing" means sequence determination using methods that determine many (typically thousands to billions) of nucleic acid sequences in an intrinsically parallel manner, *e.g.* where DNA templates are prepared for sequencing not one at a time, but in a bulk process, and where many sequences are read out preferably in parallel, or alternatively using an ultra-high throughput serial process that itself may be parallelized. Such methods include but are not limited to pyrosequencing (for example, as commercialized by 454 Life Sciences, Inc., Branford, Conn.); sequencing by ligation (for example, as commercialized in the SOLiD^{™} technology, Life Technologies, Inc., Carlsbad, Calif.); sequencing by synthesis using modified nucleotides (such as commercialized in TruSeq^{™} and HiSeq^{™} technology by Illumina, Inc., San Diego, Calif.; HeliScope^{™} by Helicos Biosciences Corporation, Cambridge, Ma.; and PacBio RS by Pacific Biosciences of California, Inc., Menlo Park, Calif.), sequencing by ion detection technologies (such as Ion Torrent^{™} technology, Life Technologies, Carlsbad, Calif.); sequencing of DNA nanoballs (Complete Genomics, Inc., Mountain View, Calif.); nanopore-based sequencing technologies (for example, as developed by Oxford Nanopore Technologies, LTD, Oxford, UK), and like highly parallelized sequencing methods.

"Multiplexing" or "multiplex assay" herein may refer to an assay or other analytical method in which the presence and/or amount of multiple targets, *e.g.,* multiple nucleic acid target sequences, can be assayed simultaneously by using more than one capture probe conjugate, each of which has at least one different detection characteristic, *e.g.,* fluorescence characteristic (for example excitation wavelength, emission wavelength, emission intensity, FWHM (full width at half maximum peak height), or fluorescence lifetime) or a unique nucleic acid or protein sequence characteristic.

### EXAMPLES

The following examples are included for illustrative purposes only and are not intended to limit the scope of the disclosure.

### EXAMPLE 1: GENERATION OF AN OLIGONUCLEOTIDE ARRAY USING PHOTO-HYBRIDIZATION/LIGATION

This example demonstrates a successful barcode assembly following four cycles of photoresist, on a lawn of immobilized oligonucleotides (*e.g.,* an R1 primer lawn). In particular, this example describes the generation of a barcoded oligonucleotide array using photo-hybridization/ligation.

**FIG. 1** shows an array of barcoded immobilized oligonucleotides. A lawn of primer oligonucleotides (e.g. shown as "Oligo Lawn") is immobilized on a substrate. The substrate may be, for example, a glass slide. The barcode sequences are assembled on immobilized oligonucleotides, *e.g.*, based on hybridization and/or ligation, on a substrate surface. In some embodiments, a method of generating the array comprises irradiating a substrate covered with a photoresist as shown in **FIG. 1****.** A four-part photo-hybridization/ligation method comprises four cycles each comprising attaching a barcode to immobilized oligonucleotides in one or more features. Each attaching step can comprise hybridizing a splint to immobilized oligonucleotides in the features and oligonucleotides comprising barcode sequences to be added, and then ligating the oligonucleotides. The splints can be designed to prevent undesirable insertions or deletions during the hybridization/ligation. The photo-hybridization ligation can be performed using exemplary methods and reagents described in US 2022/0228201 A1, US 2022/0228210 A1, and US 2022/0314187 A1.

For example, a 1^{st} cycle of hybridization/ligation can be achieved by subjecting the primer lawn to hybridization/ligation upon degradation of a photoresist, by irradiating a substrate through a photomask. A 2^{nd}, 3^{rd}, and 4^{th} cycle of hybridization/ligation may comprise the use of the same photomask (e.g., by translating the photomask to expose different regions exposed in a previous cycle) or a different photomask, allowing for the assembly of additional barcode sequences upon molecules in the features from the previous cycle of hybridization/ligation. In some aspects, the features can have a mean diameter of about 3.01 µm and a mean pitch of about 5.62 µm.

As illustrated in **FIGS. 2A-2B****,** a 4^{th} cycle of hybridization/ligation further adds a UMI and a poly(T) domain. Poly(T) sequences are configured to interact with messenger RNA (mRNA) molecules via the poly(A) tail of an mRNA transcript.

### EXAMPLE 2: REMOVING NUCLEIC ACIDS AT FEATURE BOUNDARIES IN A SPATIAL ARRAY.

This example demonstrates an application of improving an array of synthesized oligos by removing, blocking and/or inactivating oligonucleotide impurities in boundaries between features (such as gaps between features) due to overlap.

**FIG. 3** shows an exemplary spatial array generated using a combinatorial barcoding approach, e.g. as illustrated in **FIG. 1** and **FIGS. 2A****-2B.** The exemplary spatial array demonstrates a grid of nine features shown in different shades, wherein the boundary between labeled Feature 1 and Feature 2 (striped area) comprises oligo impurities resulting from overlap of features which can be caused by alignment error and/or stray light in generation of the array.

**FIG. 3** shows an exemplary design of mask (such as a photomask) to protect synthesized features while allowing selective targeting and removal of nucleic acids in boundary regions. In the mask, black-shaded area do not allow passage of irradiation (such as light), and correspond to the regions occupied by the 9 exemplary features. The white gaps between black-shaded area of the mask permit passage of irradiation (such as light), and correspond to the regions of boundaries between the 9 exemplary features.

**FIG. 3** additionally shows an exemplary scheme of cleaning (e.g. removing and/or inactivating of nucleic acids) in the feature boundaries (oligonucleotide impurities). For example, a positive photoresist can be coated on the exemplary spatial array, and subsequently covered by the mask before light exposure, which allows the positive photoresist to be degraded to expose nucleic acid molecules in the boundary region which are cleaved by irradiation (e.g., UV) or digestion (e.g., by a nuclease). **FIG. 3** shows removal of nucleic acid molecules in the boundary region, shown as the white gap between features, e.g. between Feature 1 and Feature 2 (labeled as "cleaned boundary region").

In a different exemplification for **FIG. 3****,** the nucleic acids in the boundary region can be selectively blocked or inactivated by selectively polymerizing (e.g. by irradiation through the patterned mask) a pre-coated negative photoresist to render the nucleic acids in the boundary regions inactive and/or inaccessible for hybridization and/or ligation.

The present disclosure is not intended to be limited in scope to the particular disclosed embodiments, which are provided, for example, to illustrate various aspects of the present disclosure. Various modifications to the compositions and methods described will become apparent from the description and teachings herein.

## Claims

1. A method for providing an array comprising first and second regions on a substrate, comprising:
(a) attaching first oligonucleotide molecules to first nucleic acid molecules immobilized in the first region via hybridization and/or ligation to generate first extended nucleic acid molecules;
(b) attaching second oligonucleotide molecules to second nucleic acid molecules immobilized in the second region via hybridization and/or ligation to generate second extended nucleic acid molecules; and
(c) irradiating the substrate through a mask comprising an opening corresponding to a boundary region between the first and second regions, thereby removing, blocking, or inactivating nucleic acid molecules immobilized in the boundary region,
optionally wherein the boundary region comprises part of the first region and/or part of the second region,
optionally wherein the attaching step of (a) and/or (b) comprises photolithography using a photoresist, a 3' or 5' photo-cleavable protective group, and/or a photo-cleavable polymer that blocks hybridization and/or ligation.

2. The method of claim 1, wherein the first and second regions are of about 2 µm to about 20 µm in average diameter, optionally wherein the first and second regions are of about 5 µm in average diameter.

3. The method of claim 1 or claim 2, wherein the opening corresponding to the boundary region is between about 1 µm and about 20 µm in length, optionally wherein the opening is about 5 µm in length and between about 0.1 µm and about 2 µm in width, optionally wherein the opening is about 0.5 µm in width.

4. The method of any of claims 1-3, wherein the removed, blocked, or inactivated nucleic acid molecules in the boundary region comprise (i) a first oligonucleotide molecule attached to a second nucleic acid molecule and/or (ii) a second oligonucleotide molecule attached to a first nucleic acid molecule.

5. The method of any of claims 1-4, wherein the substrate is irradiated through the mask with irradiation sufficient to cleave the nucleic acid molecules in the boundary region off the substrate, optionally wherein the substrate is irradiated through the mask with UV light, optionally wherein the method further comprises removing the cleaved nucleic acid molecules and/or cleavage products thereof.

6. The method of claim 5, wherein a positive photoresist is applied to the substrate and upon irradiation through the mask, the positive photoresist is degraded to expose nucleic acid molecules in the boundary region which are cleaved by the irradiation.

7. The method of any of claims 1-4, wherein a negative photoresist is applied to the substrate and upon irradiation through the mask, the negative photoresist is strengthened to render nucleic acid molecules in the boundary region inactive or inaccessible for ligation, optionally wherein the negative photoresist is strengthened by polymerization or cross-linking.

8. The method of any of claims 1-4, wherein a positive photoresist is applied to the substrate and upon irradiation through the mask, the positive photoresist is degraded to expose nucleic acid molecules in the boundary region, while nucleic acid molecules in the masked region(s) remain covered by the positive photoresist,
optionally further comprising removing or inactivating the exposed nucleic acid molecules.

9. The method of claim 8, wherein the exposed nucleic acid molecules are modified at the 3' or 5' end, rendering the exposed nucleic acid molecules unavailable for ligation, optionally
wherein the modified exposed nucleic acid molecules comprise modified 3' ends selected from the group consisting of dideoxy C-3' (3'-ddC), 3' inverted dT, 3' C3 spacer, 3'Amino, and 3' phosphorylation.

10. The method of any of claims 1-9, wherein the step of (a) attaching the first oligonucleotide molecules to the first nucleic acid molecules immobilized in the first region comprises:
(I) irradiating a substrate comprising an unmasked first region and a masked second region, whereby a photoresist in the first region is degraded to render the first nucleic acid molecules in the first region available for hybridization and/or ligation, whereas the second nucleic acid molecules in the second region are protected by a photoresist in the second region from hybridization and/or ligation; and
(II) attaching the first oligonucleotide molecules to the first nucleic acid molecules in the first region via hybridization and/or ligation to generate the first extended nucleic acid molecules.

11. The method of any of claims 1-10, wherein the step of (b) attaching the second oligonucleotide molecules to the second nucleic acid molecules immobilized in the second region comprises:
(III) irradiating a substrate comprising an unmasked second region and a masked first region, whereby a photoresist in the second region is degraded to render the second nucleic acid molecules in the second region available for hybridization and/or ligation, whereas the first nucleic acid molecules in the first region are protected by a photoresist in the first region from hybridization and/or ligation; and
(IV) attaching the second oligonucleotide molecules to the second nucleic acid molecules in the second region via hybridization and/or ligation to generate the second extended nucleic acid molecules.

12. The method of any of claims 1-9, wherein the step of (a) attaching the first oligonucleotide molecules to first nucleic acid molecules immobilized in the first region comprises:
(A) irradiating a substrate comprising an unmasked first region and a masked second region, whereby a photoresist in the first region is degraded to render the first nucleic acid molecules in the first region available for hybridization and/or ligation, whereas the second nucleic acid molecules in the second region are protected by the photoresist in the second region from hybridization and/or ligation; and
(B) contacting the first nucleic acid molecules in the first region with a first splint and a first oligonucleotide comprising a first barcode sequence, wherein the first splint hybridizes to the first oligonucleotide and the first nucleic acid molecules in the first region to generate the first extended nucleic acid molecules,
wherein the first oligonucleotide is ligated to the first nucleic acid molecules in the first region, and the first oligonucleotide is not ligated to second nucleic acid molecules in the second region.

13. The method of any of claims 1-9 or claim 12, wherein the photoresist is a first photoresist, and the first oligonucleotide is ligated to the first nucleic acid molecules in the first region to generate the first extended nucleic acid molecules, and the step of (b) attaching the second oligonucleotide molecules to the second nucleic acid molecules immobilized in the second region comprises:
(C) applying a second photoresist to the substrate, optionally wherein the second photoresist is applied after the first photoresist is removed from the substrate;
(D) irradiating the substrate while the first region is masked and the second region is unmasked, whereby the first or second photoresist in the second region is degraded to render the second nucleic acid molecules in the second region available for hybridization and/or ligation, whereas the first extended nucleic acid molecules in the first region are protected by the second photoresist in the first region from hybridization and/or ligation; and
(E) contacting the second nucleic acid molecules in the second region with a second splint and a second oligonucleotide comprising a second barcode sequence, wherein the second splint hybridizes to the second oligonucleotide molecule and the second nucleic acid molecules in the second region,
wherein the second oligonucleotide molecule is ligated to the second nucleic acid molecules in the second region to generate the second extended nucleic acid molecules, and the second oligonucleotide molecule is not ligated to the first extended nucleic acid molecules in the first region.

14. The method of claim 13, wherein steps (A)-(E) are part of a first round, wherein the first and second oligonucleotides are Round 1 oligonucleotides, wherein the first and second barcode sequences are Round 1 barcode sequences, and the method further comprises:
(A') irradiating the substrate while the first region is unmasked and the second region is masked, whereby a photoresist in the first region is degraded to render the first extended nucleic acid molecules in the first region available for hybridization and/or ligation, whereas the second extended nucleic acid molecules in the second region are protected by the photoresist in the second region from hybridization and/or ligation; and
(B') attaching a first Round 2 oligonucleotide comprising a first Round 2 barcode sequence to the first extended nucleic acid molecules in the first region via hybridization and/or ligation to generate first further extended nucleic acid molecules.

15. The method of claim 14, wherein the photoresist is a first photoresist, wherein the first Round 2 oligonucleotide is ligated to the first extended nucleic acid molecules in the first region to generate first further nucleic acid oligonucleotide molecules, and the method further comprises:
(C') applying a second photoresist to the substrate, optionally wherein the second photoresist is applied after the first photoresist is removed from the substrate;
(D') irradiating the substrate while the first region is masked and the second region is unmasked, whereby the first or second photoresist in the second region is degraded to render the second extended nucleic acid molecules in the second region available for hybridization and/or ligation, whereas the first further extended nucleic acid molecules in the first region are protected by the second photoresist in the first region from hybridization and/or ligation; and
(E') attaching a second Round 2 oligonucleotide comprising a second Round 2 barcode sequence to the second extended nucleic acid molecules in the second region via hybridization and/or ligation to generate second further extended nucleic acid molecules.

## Patentansprüche

1. Verfahren zum Bereitstellen einer Anordnung mit einer ersten und einer zweiten Region auf einem Substrat, umfassend:
(a) Anlagern an erste Nukleinsäuremoleküle, die in der ersten Region durch Hybridisierung und/oder Ligation immobilisiert sind, zum Erzeugen erster verlängerter Nukleinsäuremoleküle;
(b) Anlagern zweiter Oligonukleotidmolekülen an zweite Nukleinsäuremoleküle, die in der zweiten Region durch Hybridisierung und/oder Ligation immobilisiert sind, zum Erzeugen zweiter verlängerter Nukleinsäuremoleküle; und
(c) Bestrahlen des Substrats durch eine eine Öffnung umfassende Maske, die einer Grenzregion zwischen der ersten und der zweiten Region entspricht, wodurch in der Grenzregion immobilisierte Nukleinsäuremoleküle entfernt, blockiert oder inaktiviert werden,
optional wobei die Grenzregion einen Teil der ersten Region und/oder einen Teil der zweiten Region umfasst,
optional, wobei der Schritt des Anlagerns von (a) und/oder (b) die Fotolithografie unter Verwendung eines Fotolacks, einer 3' oder 5' fotospaltbaren Schutzgruppe und/oder eines fotospaltbaren Polymers umfasst, das die Hybridisierung und/oder Ligation blockiert.

2. Verfahren nach Anspruch 1, wobei die erste und zweite Region einen durchschnittlichen Durchmesser von etwa 2 µm bis etwa 20 µm aufweisen, optional wobei die erste und zweite Region einen durchschnittlichen Durchmesser von etwa 5 µm aufweisen.

3. Verfahren nach Anspruch 1 oder Anspruch 2, wobei die der Grenzregion entsprechende Öffnung eine Länge zwischen etwa 1 µm und etwa 20 µm aufweist, optional, wobei die Öffnung eine Länge von etwa 5 µm und eine Breite zwischen etwa 0,1 pm und etwa 2 µm aufweist, optional, wobei die Öffnung eine Breite von etwa 0,5 µm aufweist.

4. Verfahren nach einem der Ansprüche 1 bis 3, wobei die entfernten, blockierten oder inaktivierten Nukleinsäuremoleküle in der Grenzregion (i) ein erstes Oligonukleotidmolekül, das an ein zweites Nukleinsäuremolekül angelagert ist, und/oder (ii) ein zweites Oligonukleotidmolekül, das an ein erstes Nukleinsäuremolekül angelagert ist, umfassen.

5. Verfahren nach einem der Ansprüche 1 bis 4, wobei das Substrat durch die Maske mit einer Strahlung bestrahlt wird, die zum Abspalten der Nukleinsäuremoleküle in der Grenzregion von dem Substrat ausreichend ist, optional, wobei das Substrat durch die Maske mit UV-Licht bestrahlt wird, optional, wobei das Verfahren ferner das Entfernen der abgespaltenen Nukleinsäuremoleküle und/oder deren Spaltprodukte umfasst.

6. Verfahren nach Anspruch 5, wobei ein positiver Fotolack auf das Substrat aufgebracht wird und bei Bestrahlung durch die Maske der positive Fotolack abgebaut wird, um Nukleinsäuremoleküle in der Grenzregion zu exponieren, die durch die Bestrahlung gespalten werden.

7. Verfahren nach einem der Ansprüche 1 bis 4, wobei ein negativer Fotolack auf das Substrat aufgebracht wird und bei Bestrahlung durch die Maske der negative Fotolack verstärkt wird, um Nukleinsäuremoleküle in der Grenzregion inaktiv oder für die Ligation unzugänglich zu machen, optional wobei der negative Fotolack durch Polymerisation oder Vernetzung verstärkt wird.

8. Verfahren nach einem der Ansprüche 1 bis 4, wobei ein positiver Fotolack auf das Substrat aufgebracht wird und bei Bestrahlung durch die Maske der positive Fotolack abgebaut wird, um Nukleinsäuremoleküle in der Grenzregion zu exponieren, während Nukleinsäuremoleküle in der/den maskierten Region(en) von dem positiven Fotolack bedeckt bleiben,
optional ferner umfassend das Entfernen oder Inaktivieren der exponierten Nukleinsäuremoleküle.

9. Verfahren nach Anspruch 8, wobei die exponierten Nukleinsäuremoleküle an dem 3'- oder 5'-Ende modifiziert sind, wodurch die exponierten Nukleinsäuremoleküle für die Ligation nicht mehr verfügbar sind, optional wobei die modifizierten exponierten Nukleinsäuremoleküle modifizierte 3'-Enden umfassen, ausgewählt aus der Gruppe bestehend aus Dideoxy C-3' (3'-ddC), 3'-invertiertem dT, 3'-C3-Spacer, 3'-Amino und 3'-Phosphorylierung.

10. Verfahren nach einem der Ansprüche 1 bis 9, wobei der Schritt (a) des Anlagerns der ersten Oligonukleotidmoleküle an die ersten in der ersten Region immobilisierten Nukleinsäuremoleküle umfasst:
(I) Bestrahlen eines Substrats, das eine unmaskierte erste Region und eine maskierte zweite Region umfasst, wobei ein Fotolack in der ersten Region abgebaut wird, um die ersten Nukleinsäuremoleküle in der ersten Region für die Hybridisierung und/oder Ligation verfügbar zu machen, während die zweiten Nukleinsäuremoleküle in der zweiten Region durch einen Fotolack in der zweiten Region vor Hybridisierung und/oder Ligation geschützt sind; und
(II) Anlagern der ersten Oligonukleotidmoleküle an die ersten Nukleinsäuremoleküle in der ersten Region durch Hybridisierung und/oder Ligation zum Erzeugen der ersten verlängerten Nukleinsäuremoleküle.

11. Verfahren nach einem der Ansprüche 1 bis 10, wobei der Schritt (b) des Anlagerns der zweiten Oligonukleotidmoleküle an die zweiten in der zweiten Region immobilisierten Nukleinsäuremoleküle umfasst:
(III) Bestrahlen eines Substrats, das eine unmaskierte zweite Region und eine maskierte erste Region umfasst, wobei ein Fotolack in der zweiten Region abgebaut wird, um die zweite Nukleinsäuremoleküle in der zweiten Region für die Hybridisierung und/oder Ligation verfügbar zu machen, während die ersten Nukleinsäuremoleküle in der ersten Region durch einen Fotolack in der ersten Region vor Hybridisierung und/oder Ligation geschützt sind; und
(IV) Anlagern der zweiten Oligonukleotidmoleküle an die zweiten Nukleinsäuremoleküle in der zweiten Region durch Hybridisierung und/oder Ligation zum Erzeugen der zweiten verlängerten Nukleinsäuremoleküle.

12. Verfahren nach einem der Ansprüche 1 bis 9, wobei der Schritt (a) des Anlagerns der ersten Oligonukleotidmoleküle an erste in der ersten Region immobilisierte Nukleinsäuremoleküle umfasst:
(A) Bestrahlen eines Substrats, das eine unmaskierte erste Region und eine maskierte zweite Region umfasst, wobei ein Fotolack in der ersten Region abgebaut wird, um die ersten Nukleinsäuremoleküle in der ersten Region für die Hybridisierung und/oder Ligation verfügbar zu machen, während die zweiten Nukleinsäuremoleküle in der zweiten Region durch einen Fotolack in der zweiten Region vor Hybridisierung und/oder Ligation geschützt sind; und
(B) Inkontaktbringen der ersten Nukleinsäuremoleküle in der ersten Region mit einem ersten Splint und einem ersten Oligonukleotid, das eine erste Barcode-Sequenz umfasst, wobei der erste Splint an das erste Oligonukleotid und die ersten Nukleinsäuremoleküle in der ersten Region zum Erzeugen der ersten verlängerten Nukleinsäuremoleküle hybridisiert,
wobei das erste Oligonukleotid an die ersten Nukleinsäuremoleküle in der ersten Region ligiert ist, und das erste Oligonukleotid nicht an zweite Nukleinsäuremoleküle in der zweiten Region ligiert ist.

13. Verfahren nach einem der Ansprüche 1 bis 9 oder Anspruch 12, wobei der Fotolack ein erster Fotolack ist und das erste Oligonukleotid an die ersten Nukleinsäuremoleküle in der ersten Region ligiert wird, um die ersten verlängerten Nukleinsäuremoleküle zu erzeugen, und der Schritt (b) des Anlagerns der zweiten Oligonukleotidmoleküle an die in der zweiten Region immobilisierten zweiten Nukleinsäuremoleküle umfasst:
(C) Aufbringen eines zweiten Fotolacks auf das Substrat, optional, wobei der zweite Fotolack aufgebracht wird, nachdem der erste Fotolack von dem Substrat entfernt wurde;
(D) Bestrahlen des Substrats, während die erste Region maskiert und die zweite Region unmaskiert ist, wodurch der erste oder zweite Fotolack in der zweiten Region abgebaut wird, um die zweiten Nukleinsäuremoleküle in der zweiten Region für die Hybridisierung und/oder Ligation verfügbar zu machen, während die ersten verlängerten Nukleinsäuremoleküle in der ersten Region durch den zweiten Fotolack in der ersten Region vor Hybridisierung und/oder Ligation geschützt sind; und
(E) Inkontaktbringen der zweiten Nukleinsäuremoleküle in der zweiten Region mit einem zweiten Splint und einem zweiten Oligonukleotid, das eine zweite Barcode-Sequenz umfasst, wobei der zweite Splint an das zweite Oligonukleotidmolekül und die zweiten Nukleinsäuremoleküle in der zweiten Region hybridisiert,
wobei das zweite Oligonukleotidmolekül an die zweiten Nukleinsäuremoleküle in der zweiten Region ligiert ist, um die zweiten verlängerten Nukleinsäuremoleküle zu erzeugen, und das zweite Oligonukleotidmolekül nicht an die ersten verlängerten Nukleinsäuremoleküle in der ersten Region ligiert ist.

14. Verfahren nach Anspruch 13, wobei die Schritte (A)-(E) Teil eines ersten Durchgangs sind, wobei die ersten und zweiten Oligonukleotide Oligonukleotide des Durchgangs 1 sind, wobei die ersten und zweiten Barcode-Sequenzen Barcode-Sequenzen des Durchgangs 1 sind, und das Verfahren ferner umfasst:
(A') Bestrahlen des Substrats, während die erste Region unmaskiert und die zweite Region maskiert ist, wodurch ein Fotolack in der ersten Region abgebaut wird, um die ersten verlängerten Nukleinsäuremoleküle in der ersten Region für die Hybridisierung und/oder Ligation verfügbar zu machen, während die zweiten verlängerten Nukleinsäuremoleküle in der zweiten Region durch den Fotolack in der zweiten Region vor Hybridisierung und/oder Ligation geschützt sind; und
(B') Anlagern eines ersten Oligonukleotids des Durchgangs 2, das eine erste Barcode-Sequenz des Durchgangs 2 umfasst, an die ersten verlängerten Nukleinsäuremoleküle in der ersten Region durch Hybridisierung und/oder Ligation, um erste weiter verlängerte Nukleinsäuremoleküle zu erzeugen.

15. Verfahren nach Anspruch 14, wobei der Fotolack ein erster Fotolack ist, wobei das erste Oligonukleotid des Durchgangs 2 an die ersten verlängerten Nukleinsäuremoleküle in der ersten Region ligiert wird, um erste weitere Nukleinsäure-Oligonukleotidmoleküle zu erzeugen, und das Verfahren ferner umfasst:
(C') Aufbringen eines zweiten Fotolacks auf das Substrat, optional, wobei der zweite Fotolack aufgebracht wird, nachdem der erste Fotolack von dem Substrat entfernt wurde;
(D') Bestrahlen des Substrats, während die erste Region maskiert und die zweite Region unmaskiert ist, wodurch der erste oder zweite Fotolack in der zweiten Region abgebaut wird, um die zweiten verlängerten Nukleinsäuremoleküle in der zweiten Region für die Hybridisierung und/oder Ligation verfügbar zu machen, während die ersten weiter verlängerten Nukleinsäuremoleküle in der ersten Region durch den zweiten Fotolack in der ersten Region vor Hybridisierung und/oder Ligation geschützt sind; und
(E') Anlagern eines zweiten Oligonukleotids des Durchgangs 2, das eine zweite Barcode-Sequenz des Durchgangs 2 umfasst, an die zweiten verlängerten Nukleinsäuremoleküle in der zweiten Region durch Hybridisierung und/oder Ligation, um zweite weiter verlängerte Nukleinsäuremoleküle zu erzeugen.

## Revendications

1. Procédé pour fournir un réseau comprenant des première et deuxième régions sur un substrat, comprenant :
(a) la fixation de premières molécules d'oligonucléotide à des premières molécules d'acide nucléique immobilisées dans la première région par hybridation et/ou ligature pour générer des premières molécules d'acide nucléique étendues ;
(b) la fixation de deuxièmes molécules d'oligonucléotide à des deuxièmes molécules d'acide nucléique immobilisées dans la deuxième région par hybridation et/ou ligature pour générer des deuxièmes molécules d'acide nucléique étendues ; et
(c) l'irradiation du substrat à travers un masque comprenant une ouverture correspondant à une région limite entre les première et deuxième régions, éliminant, bloquant ou inactivant ainsi les molécules d'acide nucléique immobilisées dans la région limite,
facultativement, dans lequel la région limite comprend une partie de la première région et/ou une partie de la deuxième région,
facultativement, dans lequel l'étape de fixation (a) et/ou (b) comprend la photolithographie en utilisant une photorésine, un groupe protecteur photoclivable en 3' ou 5', et/ou un polymère photoclivable qui bloque l'hybridation et/ou la ligature.

2. Procédé selon la revendication 1, dans lequel les première et deuxième régions ont un diamètre moyen d'environ 2 µm à environ 20 µm, facultativement dans lequel les première et deuxième régions ont un diamètre moyen d'environ 5 µm.

3. Procédé selon la revendication 1 ou la revendication 2, dans lequel l'ouverture correspondant à la région limite a une longueur entre environ 1 µm et environ 20 µm, facultativement dans lequel l'ouverture a une longueur d'environ 5 µm et une largeur entre environ 0,1 µm et environ 2 µm, facultativement dans lequel l'ouverture a une largeur d'environ 0,5 µm.

4. Procédé selon l'une quelconque des revendications 1 à 3, dans lequel les molécules d'acide nucléique éliminées, bloquées ou inactivées dans la région limite comprennent (i) une première molécule d'oligonucléotide fixée à une deuxième molécule d'acide nucléique et/ou (ii) une deuxième molécule d'oligonucléotide fixée à une première molécule d'acide nucléique.

5. Procédé selon l'une quelconque des revendications 1 à 4, dans lequel le substrat est irradié à travers le masque avec une irradiation suffisante pour cliver les molécules d'acide nucléique dans la région limite du substrat, facultativement dans lequel le substrat est irradié à travers le masque avec une lumière UV, facultativement dans lequel le procédé comprend en outre l'élimination des molécules d'acide nucléique clivées et/ou des produits de clivage de celles-ci .

6. Procédé selon la revendication 5, dans lequel une photorésine positive est appliquée sur le substrat et, lors d'une irradiation à travers le masque, la photorésine positive est dégradée pour exposer des molécules d'acide nucléique dans la région limite qui sont clivées par l'irradiation.

7. Procédé selon l'une quelconque des revendications 1 à 4, dans lequel une photorésine négative est appliquée sur le substrat et, lors d'une irradiation à travers le masque, la photorésine négative est renforcée pour rendre les molécules d'acide nucléique dans la région limite inactives ou inaccessibles pour la ligature, facultativement, dans lequel la photorésine négative est renforcée par polymérisation ou réticulation.

8. Procédé selon l'une quelconque des revendications 1 à 4, dans lequel une photorésine positive est appliquée sur le substrat et, lors d'une irradiation à travers le masque, la photorésine positive est dégradée pour exposer des molécules d'acide nucléique dans la région limite, tandis que des molécules d'acide nucléique dans la ou les région(s) masquées restent recouvertes par la photorésine positive, facultativement, comprenant en outre l'élimination ou l'inactivation des molécules d'acide nucléique exposées.

9. Procédé selon la revendication 8, dans lequel les molécules d'acide nucléique exposées sont modifiées à l'extrémité 3' ou 5', rendant les molécules d'acide nucléique exposées indisponibles pour la ligature, facultativement dans lequel les molécules d'acide nucléique exposées modifiées comprennent des extrémités 3' modifiées choisies dans le groupe constitué de didésoxy C-3' (3'-ddC), 3' dT inversé, 3' espaceur C3, 3' amino et phosphorylation en 3'.

10. Procédé selon l'une quelconque des revendications 1 à 9, dans lequel l'étape (a) de fixation des premières molécules d'oligonucléotide aux premières molécules d'acide nucléique immobilisées dans la première région comprend :
(I) l'irradiation d'un substrat comprenant une première région non masquée et une deuxième région masquée, moyennant quoi une photorésine dans la première région est dégradée pour rendre les premières molécules d'acide nucléique dans la première région disponibles pour l'hybridation et/ou la ligature, alors que les deuxièmes molécules d'acide nucléique dans la deuxième région sont protégées par une photorésine dans la deuxième région contre l'hybridation et/ou la ligature ; et
(II) la fixation des premières molécules d'oligonucléotides aux premières molécules d'acide nucléique dans la première région par hybridation et/ou ligature pour générer les premières molécules d'acide nucléique étendues.

11. Procédé selon l'une quelconque des revendications 1 à 10, dans lequel l'étape (b) de fixation des deuxièmes molécules d'oligonucléotide aux deuxièmes molécules d'acide nucléique immobilisées dans la deuxième région comprend :
(III) l'irradiation d'un substrat comprenant une deuxième région non masquée et une première région masquée, moyennant quoi une photorésine dans la deuxième région est dégradée pour rendre les deuxièmes molécules d'acide nucléique dans la deuxième région disponibles pour l'hybridation et/ou la ligature, alors que les premières molécules d'acide nucléique dans la première région sont protégées par une photorésine dans la première région contre l'hybridation et/ou la ligature ; et
(IV) la fixation des deuxièmes molécules d'oligonucléotide aux deuxièmes molécules d'acide nucléique dans la deuxième région par hybridation et/ou ligature pour générer les deuxièmes molécules d'acide nucléique étendues.

12. Procédé selon l'une quelconque des revendications 1 à 9, dans lequel l'étape (a) de fixation des premières molécules d'oligonucléotide à des premières molécules d'acide nucléique immobilisées dans la première région comprend :
(A) l'irradiation d'un substrat comprenant une première région non masquée et une deuxième région masquée, moyennant quoi une photorésine dans la première région est dégradée pour rendre les premières molécules d'acide nucléique dans la première région disponibles pour l'hybridation et/ou la ligature, alors que les deuxièmes molécules d'acide nucléique dans la deuxième région sont protégées par une photorésine dans la deuxième région contre l'hybridation et/ou la ligature ; et
(B) la mise en contact des premières molécules d'acide nucléique dans la première région avec une première attelle et un premier oligonucléotide comprenant une première séquence de code à barres, dans lequel la première attelle s'hybride au premier oligonucléotide et aux premières molécules d'acide nucléique dans la première région pour générer les premières molécules d'acide nucléique étendues, dans lequel le premier oligonucléotide est ligaturé aux premières molécules d'acide nucléique dans la première région, et le premier oligonucléotide n'est pas ligaturé aux deuxièmes molécules d'acide nucléique dans la deuxième région.

13. Procédé selon l'une quelconque des revendications 1 à 9 ou la revendication 12, dans lequel la photorésine est une première photorésine, et le premier oligonucléotide est ligaturé aux premières molécules d'acide nucléique dans la première région pour générer les premières molécules d'acide nucléique étendues, et l'étape (b) de fixation des deuxièmes molécules d'oligonucléotide aux deuxièmes molécules d'acide nucléique immobilisées dans la deuxième région comprend :
(C) l'application d'une deuxième photorésine sur le substrat, facultativement dans lequel la deuxième photorésine est appliquée après que la première photorésine a été éliminée du substrat ;
(D) l'irradiation du substrat tandis que la première région est masquée et la deuxième région est non masquée, moyennant quoi la première ou deuxième photorésine dans la deuxième région est dégradée pour rendre les deuxièmes molécules d'acide nucléique dans la deuxième région disponibles pour l'hybridation et/ou la ligature, alors que les premières molécules d'acide nucléique étendues dans la première région sont protégées par la deuxième photorésine dans la première région contre l'hybridation et/ou la ligature ; et
(E) la mise en contact des deuxièmes molécules d'acide nucléique dans la deuxième région avec une deuxième attelle et un deuxième oligonucléotide comprenant une deuxième séquence de code à barres, dans lequel la deuxième attelle s'hybride à la deuxième molécule d'oligonucléotide et aux deuxièmes molécules d'acide nucléique dans la deuxième région,
dans lequel la deuxième molécule d'oligonucléotide est ligaturée aux deuxièmes molécules d'acide nucléique dans la deuxième région pour générer les deuxièmes molécules d'acide nucléique étendues, et la deuxième molécule d'oligonucléotide n'est pas ligaturée aux premières molécules d'acide nucléique étendues dans la première région.

14. Procédé selon la revendication 13, dans lequel les étapes (A) à (E) font partie d'un premier tour (round), dans lequel les premier et deuxième oligonucléotides sont des oligonucléotides Round 1, dans lequel les première et deuxième séquences de code à barres sont des séquences de code à barres Round 1, et le procédé comprend en outre :
(A') l'irradiation du substrat tandis que la première région est non masquée et la deuxième région est masquée, moyennant quoi une photorésine dans la première région est dégradée pour rendre les premières molécules d'acide nucléique dans la première région disponibles pour l'hybridation et/ou la ligature, alors que les deuxièmes molécules d'acide nucléique étendues dans la deuxième région sont protégées par la photorésine dans la deuxième région contre l'hybridation et/ou la ligature ; et
(B') la fixation d'un premier oligonucléotide Round 2 comprenant une première séquence de code à barres Round 2 aux premières molécules d'acide nucléique étendues dans la première région par hybridation et/ou ligature pour générer des premières molécules d'acide nucléique étendues supplémentaires.

15. Procédé selon la revendication 14, dans lequel la photorésine est une première photorésine, dans lequel le premier oligonucléotide Round 2 est ligaturé aux premières molécules d'acide nucléique étendues dans la première région pour générer des premières molécules d'oligonucléotide d'acide nucléique supplémentaires, et le procédé comprend en outre :
(C') l'application d'une deuxième photorésine sur le substrat, facultativement dans lequel la deuxième photorésine est appliquée après que la première photorésine a été éliminée du substrat ;
(D') l'irradiation du substrat tandis que la première région est masquée et la deuxième région est non masquée, moyennant quoi la première ou deuxième photorésine dans la deuxième région est dégradée pour rendre les deuxièmes molécules d'acide nucléique étendues dans la deuxième région disponibles pour l'hybridation et/ou la ligature, alors que les premières molécules d'acide nucléique étendues supplémentaires dans la première région sont protégées par la deuxième photorésine dans la première région contre l'hybridation et/ou la ligature ; et
(E') la fixation d'un deuxième oligonucléotide Round 2 comprenant une deuxième séquence de code à barres Round 2 aux deuxièmes molécules d'acide nucléique étendues dans la deuxième région par hybridation et/ou ligature pour générer des deuxièmes molécules d'acide nucléique étendues supplémentaires.
